# EUROPÄISCHE PATENTSCHRIFT

(11) **EP 0 751 942 B1**
(45) Veröffentlichungstag und Bekanntmachung des Hinweises auf die Patenterteilung: **31.05.2000**
(21) Anmeldenummer: 95913114.5
(22) Anmeldetag: 14.03.1995
(51) Int. Cl.: C07D 333/50, C07D 333/32, C07C 323/22, C07C 323/61

(54) **3-ARYL-4-HYDROXY-DELTA 3 -DIHYDROTHIOPHENON-DERIVATE**
3-ARYL-4-HYDROXY-DELTA 3 -DIHYDROTHIOPHENONE DERIVATIVES
DERIVES DE 3-ARYL-4-HYDROXY-DELTA 3 -DIHYDROTHIOPHENONE

(30) Priorität: 25.03.1994 DE 4410420
(43) Veröffentlichungstag der Anmeldung: 08.01.1997
(73) Patentinhaber: BAYER AG, 51368 Leverkusen (DE)
(72) Erfinder: FISCHER, Reiner, D-40789 Monheim (DE); DUMAS, Jacques, D-51061 Köln (DE); BRETSCHNEIDER, Thomas, D-53797 Lohmar (DE); ERDELEN, Christoph, D-42799 Leichlingen (DE); WACHENDORFF-NEUMANN, Ulrike, D-40789 Monheim (DE)
(86) Internationale Anmeldenummer: EP9500953
(87) Internationale Veröffentlichungsnummer: WO9526345

(56) Entgegenhaltungen:
- EP-A- 0 528 156
- JOURNAL OF THE CHEMICAL SOCIETY, CHEMICAL COMMUNICATIONS., Nr.16, 1987, LETCHWORTH GB Seiten 1228 - 1230 M. S. CHAMBERS ET AL. 'An Asymmetric Synthesis of Thiotetronic Acids using Chirality Transfer via an Allyl Xanthate-to-Dithiocarbonate Rearrangement. X-Ray Crystal Structure of (5R)-2,5-Dihydr o-4-hydroxy-5-methyl-3-phenyl-5-prop-1'-en yl-2-oxothiophene'
- JOURNAL OF ANTIBIOTICS, Bd.36, Nr.11, 1983, TOKYO JP Seiten 1589 - 1591 K. TSUZUKI ET AL. 'Synthesis and Biological Activities of Thiotetromycin Analogs'

## Beschreibung

Die vorliegende Erfindung betrifft neue 3-Aryl-4-hydroxy-Δ³-dihydrothiophenon-Derivate, Verfahren zu ihrer Herstellung und ihre Verwendung als Schädlingsbekämpfungsmittel.

Es ist bekannt, daß bestimmte substituierte Δ³-Dihydrofuran-2-on-Derivate herbizide Eigenschaften besitzen (vgl. DE-A 4 014 420). Die Synthese der als Ausgangsverbindungen verwendeten Tetronsäurederivate (wie z.B. 3-(3-Methyl-phenyl)-4-hydroxy-5-(4-fluorphenyl)-Δ³-dihydrofuranon-(2) ist ebenfalls in DE-A 4 014 420 beschrieben. Ähnlich strukturierte Verbindungen ohne Angabe einer insektiziden und/oder akariziden Wirksamkeit sind aus der Publikation Campbell et al. J. Chem. Soc., Perkin Trans. 1 1985, (8) 1567-8 bekannt. Weiterhin sind 3-Aryl-Δ³-dihydrofuranon-Derivate mit herbiziden, akariziden und insektiziden Eigenschaften aus EP 528 156 bekannt, jedoch ist die dort beschriebene Wirkung nicht immer ausreichend.

Es wurden nun neue 3-Aryl-4-hydroxy-Δ³-dihydrothiophenon-Derivate der Formel (I) 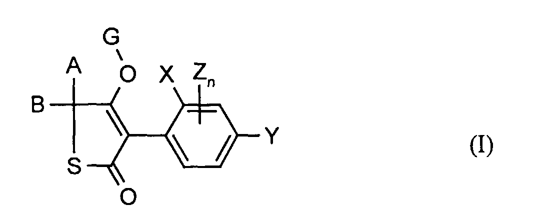 gefunden,
in welcher
- X: für C₁-C₆-Alkyl, Halogen, C₁-C₆-Alkoxy oder C₁-C₃-Halogenalkyl steht,
- Y: für Wasserstoff, C₁-C₆-Alkyl, Halogen, C₁-C₆-Alkoxy oder C₁-C₃-Halogenalkyl steht,
- Z: für C₁-C₆-Alkyl, Halogen oder C₁-C₆-Alkoxy steht,
- n: für eine Zahl von 0 bis 3 steht
oder die Reste X und Z gemeinsam mit dem Phenylrest an den sie gebunden sind, für den Naphthalinrest der Formel stehen, 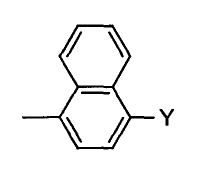 in welchem
- Y: die oben angegebene Bedeutung hat,
- A und B: gemeinsam mit dem Kohlenstoffatom, an das sie gebunden sind, für einen gesättigten oder ungesättigten, gegebenenfalls durch Sauerstoff und/oder Schwefel unterbrochenen und gegebenenfalls einfach oder mehrfach gleich oder verschieden durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls einfach oder mehrfach, gleich oder verschieden durch Halogen, C₁-C₆-Alkyl oder C₁-C₆-Alkoxy substituiertes Phenyl substituierten 3- bis 8-gliedrigen Ring stehen oder
- A und B: gemeinsam mit dem Kohlenstoffatom, an das sie gebunden sind, für einen C₃-C₈-gliedrigen Ring stehen, bei dem zwei Substituenten gemeinsam mit den Kohlenstoffatomen, an die sie gebunden sind, für einen gegebenenfalls einfach oder mehrfach gleich oder verschieden durch C₁-C₆-Alkyl, C₁-C₆-Alkoxy oder Halogen substituierten gesättigten oder einfach ungesättigten C₅-C₇-Ring stehen, der durch Sauerstoff oder Schwefel unterbrochen sein kann,
- G: für Wasserstoff (a) oder für eine der Gruppen 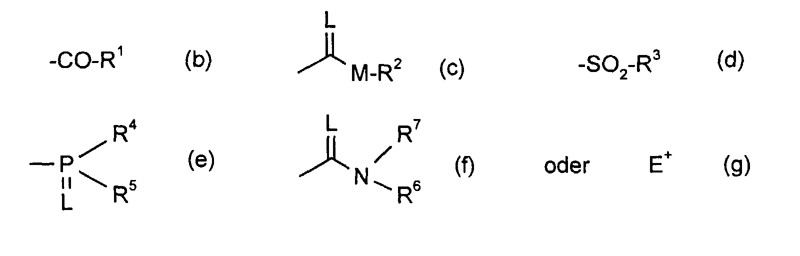 steht, in welchen
- E⁺: für ein Metallionäquivalent oder ein Ammoniumion steht,
- L: für Sauerstoff oder Schwefel steht und
- M: für Sauerstoff oder Schwefel steht,
- R¹: für jeweils gegebenenfalls einfach oder mehrfach, gleich oder verschieden durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, C₁-C₈-Polyalkoxy-C₂-C₈-alkyl oder gegebenenfalls durch Halogen oder C₁-C₆-Alkyl substituiertes Cycloalkyl mit 3 bis 8 Ringatomen, das durch mindestens ein Sauerstoff- und/oder Schwefelatom unterbrochen sein kann,
für gegebenenfalls einfach oder mehrfach, gleich oder verschieden durch Halogen, Nitro, C₁-C₆-Alkyl, C₁-C₆-Alkoxy, C₁-C₆-Halogenalkyl oder C₁-C₆-Halogenalkoxy substituiertes Phenyl,
für gegebenenfalls einfach oder mehrfach, gleich oder verschieden durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls einfach oder mehrfach, gleich oder verschieden durch Halogen oder C₁-C₆-Alkyl substituiertes 5- oder 6-gliedriges Hetaryl,
für gegebenenfalls einfach oder mehrfach, gleich oder verschieden durch Halogen oder C₁-C₆-Alkyl substituiertes Phenoxy-C₁-C₆-alkyl oder
für gegebenenfalls einfach oder mehrfach, gleich oder verschieden durch Halogen, Amino oder C₁-C₆-Alkyl substituiertes 5- oder 6-gliedriges Hetaryloxy-C₁-C₆-alkyl steht,
- R²: für jeweils gegebenenfalls einfach oder mehrfach, gleich oder verschieden durch Halogen substituiertes C₁-C₂₀-Alkyl, C₂-C₂₀-Alkenyl, C₁-C₈-Alkoxy-C₂-C₈-alkyl oder C₁-C₈-Polyalkoxy-C₂-C₈-alkyl oder für gegebenenfalls durch Halogen, C₁-C₄-Alkyl oder C₁-C₄-Alkoxy substituiertes C₃-C₈-Cycloalkyl oder
für jeweils gegebenenfalls einfach oder mehrfach, gleich oder verschieden durch Halogen, Nitro, C₁-C₆-Alkyl, C₁-C₆-Alkoxy oder C₁-C₆-Halogenalkyl substituiertes Phenyl oder Benzyl steht,
- R³, R⁴ und R⁵: unabhängig voneinander für jeweils gegebenenfalls einfach oder mehrfach, gleich oder verschieden durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alkinylthio, C₃-C₇-Cycloalkylthio oder für jeweils gegebenenfalls einfach oder mehrfach, gleich oder verschieden durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Benzyl, Phenoxy oder Phenylthio stehen und
- R⁶ und R⁷: unabhängig voneinander für Wasserstoff, jeweils gegebenenfalls einfach oder mehrfach, gleich oder verschieden durch Halogen substituiertes C₁-C₂₀-Alkyl, C₁-C₂₀-Alkoxy, C₂-C₈-Alkenyl oder C₁-C₂₀-Alkoxy-C₁-C₂₀-alkyl, für jeweils gegebenenfalls einfach oder mehrfach, gleich oder verschieden durch Halogen, C₁-C₂₀-Halogenalkyl, C₁-C₂₀-Alkyl oder C₁-C₂₀-Alkoxy substituiertes Phenyl oder Benzyl oder zusammen für einen gegebenenfalls durch Sauerstoff unterbrochenen C₂-C₆-Alkylenrest stehen.

Aufgrund eines oder mehrerer Chiralitätszentren fallen die Verbindungen der Formeln (Ia) - (Ig) im allgemeinen als Stereoisomerengemisch an, die gegebenenfalls in üblicher Art und Weise getrennt werden könnnen. Sie können sowohl in Form ihrer Diastereomerengemische als auch als reine Diastereomere oder Enantiomere verwendet werden. Im folgenden wird der Einfachheit halber stets von Verbindungen der Formeln (Ia) bis (Ig) gesprochen, obwohl sowohl die reinen Verbindungen, als auch die Gemische mit unterschiedlichen Anteilen an isomeren, enantiomeren und stereomeren Verbindungen gemeint sind.

Weiterhin wurde gefunden,
(A)
   daß man 3-Aryl-4-hydroxy-Δ³-dihydrothiophenon-Derivate der Formel (Ia) 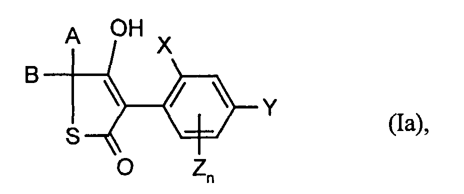 in welcher
   A, B, X, Y, Z und n die oben angegebene Bedeutung haben,
   erhält, wenn man
   β-Ketocarbonsäureester der Formel (II) 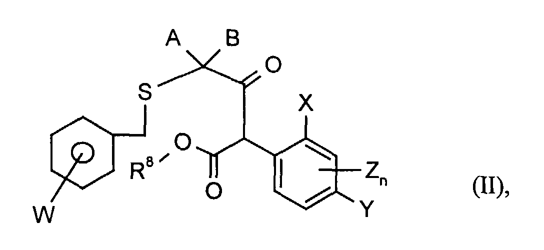 in welcher
   A, B, X, Y, Z und n die oben angegebene Bedeutung haben,

   - W: für Wasserstoff, Halogen, C₁-C₈-Alkyl oder C₁-C₈-Alkoxy,
   und
   - R⁸: für C₁-C₈-Alkyl steht,
   gegebenenfalls in Gegenwart eines Verdünnungsmittels und in Gegenwart einer Säure intramolekular cyclisiert und
(B)
   daß man Verbindungen der Formel (Ib) 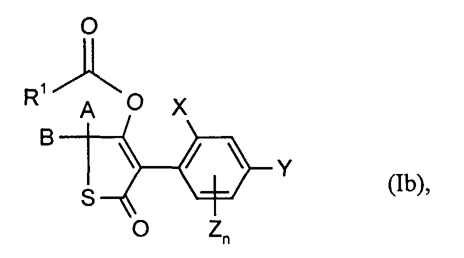 in welcher
   A, B, X, Y, Z, R¹ und n die oben angegebene Bedeutung haben,
   erhält, wenn man Verbindungen der Formel (Ia) 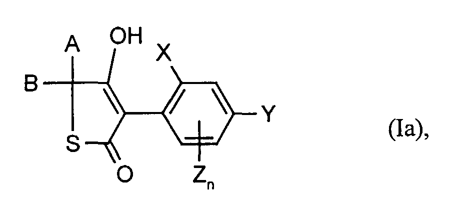 in welcher
   A, B, X, Y, Z und n die oben angegebene Bedeutung haben,

   α) mit Säurehalogeniden der Formel (III) 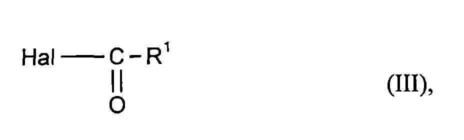 in welcher
      - R¹: die oben angegebene Bedeutung hat
      und
      - Hal: für Halogen, insbesondere Chlor oder Brom steht,
      gegebenenfalls in Gegenwart eines Verdünnungsmittels und gegebenenfalls in Gegenwart eines Säurebindemittels umsetzt
      oder
   β) mit Carbonsäureanhydriden der Formel (IV)

      R¹-CO-O-CO-R¹ (IV),

      in welcher
      - R¹: die oben angegebene Bedeutung hat,
      gegebenenfalls in Gegenwart eines Verdünnungsmittels und gegebenenfalls in Gegenwart eines Säurebindemittels,
   umsetzt und
(C)
   daß man Verbindungen der Formel (Ic) 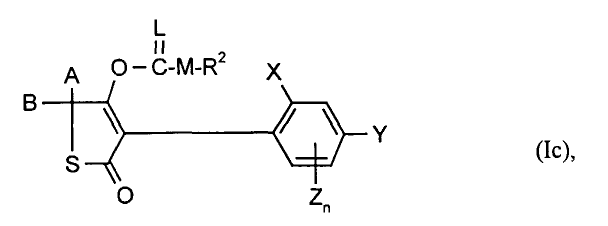 in welcher
   A, B, X, Y, Z, R² und n die oben angegebene Bedeutung haben,

   - L: für Sauerstoff und
   - M: für Sauerstoff oder Schwefel steht,
   erhält, wenn man Verbindungen der Formel (Ia) 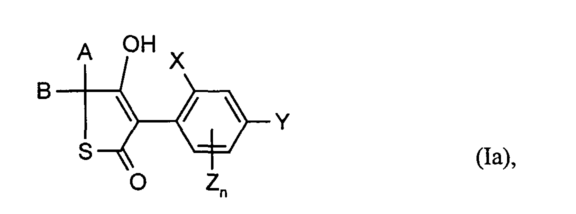 in welcher
   A, B, X, Y, Z und n die oben angegebene Bedeutung haben,
   mit Chlorameisensäureester oder Chlorameisensäurethiolester der Formel (V)

   R²-M-CO-Cl (V),

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt, und
(D)
   daß man Verbindungen der Formel (Ic) 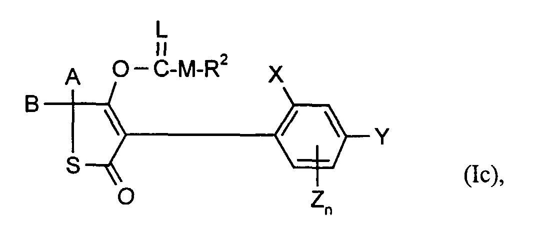 in welcher
   A, B, R², X, Y, Z und n die oben angegebene Bedeutung haben,

   - L: für Schwefel und
   - M: für Sauerstoff oder Schwefel steht,
   erhält, wenn man Verbindungen der Formel (Ia) 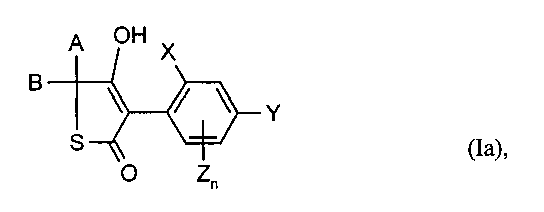 in welcher
   A, B, X, Y, Z und n die oben angegebene Bedeutung haben,

   α) mit Chlormonothioameisensäureestern oder Chlordithioameisensäureestern der Formel (VI) 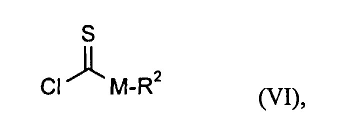 in welcher
      - M und R²: die oben angegebene Bedeutung haben
      gegebenenfalls in Gegenwart eines Verdünnungsmittels und gegebenenfalls in Gegenwart eines Säurebindemittels umsetzt, oder
   β) mit Schwefelkohlenstoff und anschließend mit Alkylhalogeniden der Formel (VII)

      R²-Hal (VII),

      in welcher
      - R²: die oben angegebene Bedeutung hat
      und
      - Hal: für Chlor, Brom oder Iod steht, umsetzt und
(E)
   daß man Verbindungen der Formel (Id) 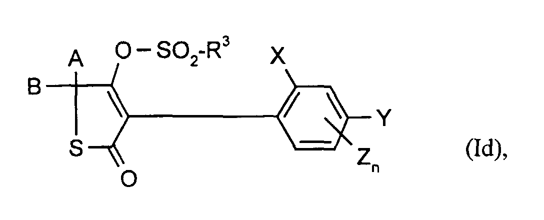 in welcher
   A, B, X, Y, Z, R³ und n die oben angegebene Bedeutung haben,
   erhält, wenn man Verbindungen der Formel (Ia) 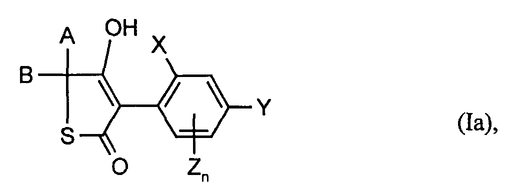 in welcher
   A, B, X, Y, Z und n die oben angegebene Bedeutung haben,
   mit Sulfonsäurechloriden der Formel (VIII)

   R³-SO₂-Cl (VIII),

   in welcher
   - R³: die oben angegebene Bedeutung hat
   gegebenenfalls in Gegenwart eines Verdünnungsmittels und gegebenenfalls in Gegenwart eines Säurebindemittels,
   umsetzt, und
(F)
   daß man Verbindungen der Formel (Ie) 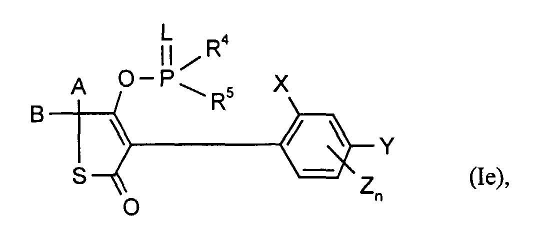 in welcher
   A, B, L, X, Y, Z, R⁴, R⁵ und n die oben angegebene Bedeutung haben,
   erhält, wenn man
   Verbindungen der Formel (Ia) 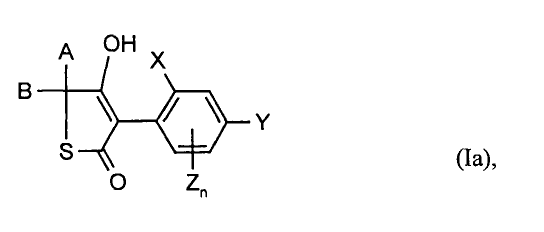 in welcher
   A, B, X, Y, Z und n die oben angegebene Bedeutung haben
   mit Phosphorverbindungen der Formel (IX) 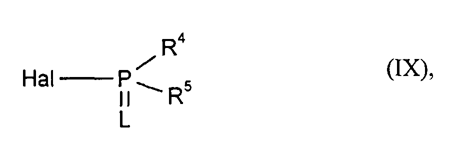 in welcher
   - L, R⁴ und R⁵: die oben angegebene Bedeutung haben und
   - Hal: für Halogen, insbesondere Chlor oder Brom steht,
   gegebenenfalls in Gegenwart eines Verdünnungsmittels und gegebenenfalls in Gegenwart eines Säurebindemittels umsetzt, und
(G) daß man Verbindungen der Formel (If) 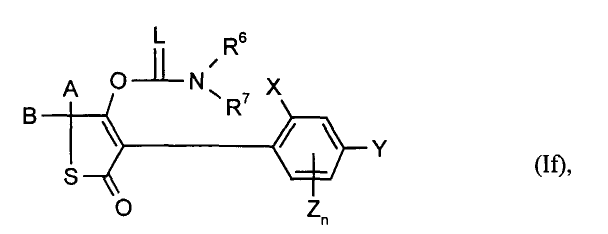 in welcher
   A, B, L, X, Y, Z, R⁶, R⁷ und n die oben angegebene Bedeutung haben,
   erhält, wenn man Verbindungen der Formel (Ia) 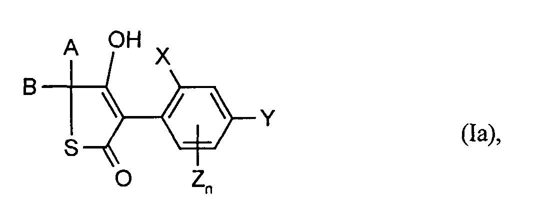 in welcher
   A, B, X, Y, Z und n die oben angegebene Bedeutung haben

   α) mit Isocyanaten oder Isothiocyanaten der Formel (X)

      R⁶-N=C=L (X),

      in welcher
      - R⁶ und L: die oben angegebene Bedeutung hat
      gegebenenfalls in Gegenwart eines Verdünnungsmittels und gegebenenfalls in Gegenwart eines Katalysators umsetzt,
      oder
   β) mit Carbamidsäurechloriden oder Thiocarbamidsäurechloriden der Formel (XI) 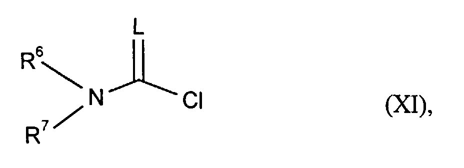 in welcher
      - L, R⁶ und R⁷: die oben angegebene Bedeutung haben,
      gegebenenfalls in Gegenwart eines Verdünnungsmittels und gegebenenfalls in Gegenwart eines Säurebindemittels,
   umsetzt, und
(H) daß man Verbindungen der Formel (Ig) 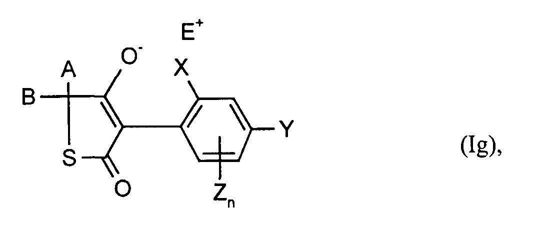 in welcher
   X, Y, Z, A, B und n die oben angegebene Bedeutung haben,
   und E⁺ für ein Metallionäquivalent oder für ein Ammoniumion steht,
   erhält, wenn man Verbindungen der Formel (Ia) 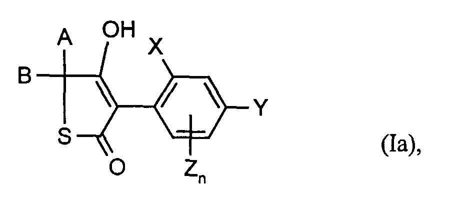 in welcher
   X, Y, Z, A, B und n die oben angegebene Bedeutung haben,
   mit Metallhydroxiden, Metallalkoxiden oder Aminen der Formeln (XII) und (XIII)

   Me(OR⁹)ₜ (XII)

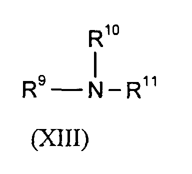

   in welchen
   - Me: für ein- oder zweiwertige Metallionen, beispielsweise der Metalle Natrium, Kalium, Magnesium oder Calcium und
   - t: für die Zahl 1 oder 2 steht und
   - R⁹, R¹⁰ und R¹¹: abhängig voneinander für Wasserstoff oder Alkyl stehen,
   gegebenenfalls in Gegenwart eines Verdünnungsmittels, umsetzt.

Weiterhin wurde gefunden, daß sich die neuen 3-Aryl-4-hydroxy-Δ³-dihydrothiophenon-Derivate der Formel (I) durch hervorragende akarizide und insektizide Wirkungen auszeichnen.

Die erfindungsgemäßen Verbindungen sind durch die Formel (I) allgemein definiert.
- X: steht besonders bevorzugt für C₁-C₆-Alkyl, Halogen, C₁-C₆-Alkoxy oder C₁-C₂-Halogenalkyl.
- Y: steht besonders bevorzugt für Wasserstoff, C₁-C₆-Alkyl, Halogen, C₁-C₆-Alkoxy oder C₁-C₂-Halogenalkyl.
- Z: steht besonders bevorzugt für C₁-C₄-Alkyl, Halogen oder C₁-C₄-Alkoxy.
- n: steht besonders bevorzugt für eine Zahl von 0 bis 2.
oder die Reste X und Z stehen besonders bevorzugt gemeinsam mit dem Phenylrest an den sie gebunden sind, für den Naphthalinrest der Formel 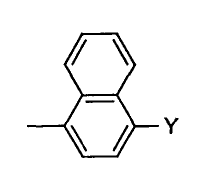 in welchem
Y die oben angegebene Bedeutung hat.
- A und B: stehen besonders bevorzugt gemeinsam mit dem Kohlenstoffatom, an das sie gebunden sind, für einen gesättigten oder ungesättigten, gegebenenfalls durch Sauerstoff und/oder Schwefel unterbrochen und gegebenenfalls einfach oder mehrfach, gleich oder verschieden durch Fluor, Chlor, C₁-C₅-Alkyl, C₁-C₅-Alkoxy, C₁-C₃-Halogenalkyl, C₁-C₄-Halogenalkoxy, C₁-C₃-Alkylthio oder gegebenenfalls einfach oder mehrfach, gleich oder verschieden durch Fluor, Chlor, Brom, C₁-C₄-Alkyl oder C₁-C₄-Alkoxy substituiertes Phenyl substituierten 3- bis 8-gliedrigen Ring, oder
- A und B: stehen besonders bevorzugt gemeinsam mit dem Kohlenstoffatom, an das sie gebunden sind, für einen C₄-C₇-gliedrigen Ring, bei dem zwei Substituenten gemeinsam mit den Kohlenstoffatomen, an die sie gebunden sind, für einen gegebenenfalls einfach oder mehrfach, gleich oder verschieden durch C₁-C₂-Alkyl, C₁-C₃-Alkoxy, Fluor oder Chlor substituierten gesättigten oder ungesättigten C₅-C₆-Ring stehen, der durch Sauerstoff oder Schwefel unterbrochen sein kann.
- G: steht besonders bevorzugt für Wasserstoff (a) oder für eine der Gruppen 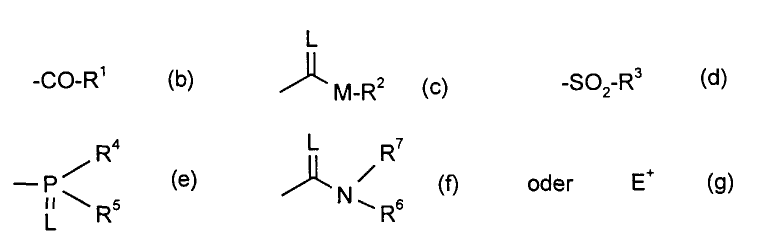 in welchen
- E⁺: für ein Metallionäquivalent oder ein Ammoniumion steht und
- L: für Sauerstoff oder Schwefel steht und
- M: für Sauerstoff oder Schwefel steht.
- R¹: steht besonders bevorzugt für jeweils gegebenenfalls einfach oder mehrfach, gleich oder verschieden durch Halogen substituiertes C₁-C₁₆-Alkyl, C₁-C₁₆-Alkenyl, C₁-C₆-Alkoxy-C₁-C₆-alkyl, C₁-C₆-Alkylthio-C₁-C₆-alkyl, C₁-C₆-Polyalkoxy-C₂-C₆-alkyl oder gegebenenfalls durch Fluor, Chlor oder C₁-C₄-Alkyl substituiertes Cycloalkyl mit 3 bis 7 Ringatomen, das durch 1 bis 2 Sauerstoff- und/oder Schwefelatome unterbrochen sein kann,
für gegebenenfalls einfach bis fünffach gleich oder verschieden durch Halogen, Nitro, C₁-C₄-Alkyl, C₁-C₄-Alkoxy, C₁-C₃-Halogenalkyl oder C₁-C₃-Halogenalkoxy substituiertes Phenyl,
für gegebenenfalls einfach bis fünffach, gleich oder verschieden durch Halogen, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für gegebenenfalls einfach bis vierfach, gleich oder verschieden durch Halogen oder C₁-C₆-Alkyl substituiertes Pyridyl, Thienyl, Furanyl, Pyrimidyl, Thiazolyl oder Pyrazolyl,
für gegebenenfalls einfach bis fünffach, gleich oder verschieden durch Halogen oder C₁-C₄-Alkyl substituiertes Phenoxy-C₁-C₅-alkyl,
für gegebenenfalls einfach bis vierfach, gleich oder verschieden durch Halogen, Amino oder C₁-C₄-Alkyl substituiertes Pyridyloxy-, Pyrimidyloxy- oder Thiazolyloxy-C₁-C₅-alkyl.
- R²: steht besonders bevorzugt für jeweils gegebenenfalls einfach oder mehrfach, gleich oder verschieden durch Halogen substituiertes C₁-C₁₆-Alkyl, C₂-C₁₆-Alkenyl, C₁-C₆-Alkoxy-C₂-C₆-alkyl oder C₁-C₆-Polyalkoxy-C₂-C₆-alkyl, für gegebenenfalls durch Fluor, Chlor, C₁-C₃-Alkyl oder C₁-C₃-Alkoxy substituiertes C₃-C₇-Cycloalkyl oder
für jeweils gegebenenfalls einfach bis fünffach, gleich oder verschieden durch Halogen, Nitro, C₁-C₄-Alkyl, C₁-C₃-Alkoxy oder C₁-C₃-Halogenalkyl substituiertes Phenyl oder Benzyl.
- R³, R⁴ und R⁵: stehen unabhängig voneinander besonders bevorzugt für jeweils gegebenenfalls einfach oder mehrfach, gleich oder verschieden durch Halogen substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄Alkenylthio, C₂-C₄-Alkinylthio, C₃-C₆-Cycloalkylthio oder für jeweils gegebenenfalls einfach bis fünffach, gleich oder verschieden durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Benzyl, Phenoxy oder Phenylthio.
- R⁶ und R⁷: stehen unabhängig voneinander besonders bevorzugt für Wasserstoff, jeweils gegebenenfalls einfach oder mehrfach, gleich oder verschieden durch Halogen substituiertes C₁-C₂₀-Alkyl, C₁-C₂₀-Alkoxy, C₂-C₈-Alkenyl oder C₁-C₂₀-Alkoxy-C₁-C₂₀-alkyl, für gegebenenfalls einfach bis fünffach gleich oder verschieden durch Halogen, C₁-C₅-Halogenalkyl, C₁-C₅-Alkyl oder C₁-C₅-Alkoxy substituiertes Phenyl oder Benzyl oder zusammen für einen gegebenenfalls durch Sauerstoff oder Schwefel unterbrochenen C₄-C₆-Alkylenrest.
- X: steht ganz besonders bevorzugt für Methyl, Ethyl, Propyl, i-Propyl, Fluor, Chlor, Brom, Methoxy, Ethoxy oder Trifluormethyl.
- Y: steht ganz besonders bevorzugt für Wasserstoff, Methyl, Ethyl, Propyl, i-Propyl, Butyl, i-Butyl, tert.-Butyl, Fluor, Chlor, Brom, Methoxy, Ethoxy oder Trifluormethyl.
- Z: steht ganz besonders bevorzugt für Methyl, Ethyl, i-Propyl, Butyl, i-Butyl, tert.-Butyl, Fluor, Chlor, Brom, Methoxy oder Ethoxy.
- n: steht ganz besonders bevorzugt für eine Zahl 0 oder 1
oder die Reste X und Z stehen ganz besonders bevorzugt gemeinsam mit dem Phenylrest an den sie gebunden sind für den Rest der Formel 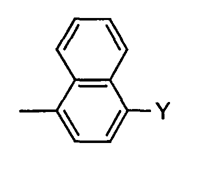 in welchem Y die oben angegebene Bedeutung hat.
- A und B: stehen ganz besonders bevorzugt gemeinsam mit dem Kohlenstoffatom, an das sie gebunden sind für einen gesättigten oder ungesättigten, gegebenenfalls durch Sauerstoff und/oder Schwefel unterbrochenen und gegebenenfalls einfach bis fünffach, gleich oder verschieden durch Fluor, Chlor, C₁-C₄-Alkyl, C₁-C₄-Alkoxy, Trifluormethyl, C₁-C₂-Alkylthio oder gegebenenfalls einfach bis dreifach, gleich oder verschieden durch Fluor, Chlor, Methyl oder Methoxy substituiertes Phenyl substituierten 3- bis 8-gliedrigen Ring oder
- A und B: stehen ganz besonders bevorzugt gemeinsam mit dem Kohlenstoffatom, an das sie gebunden sind, für einen C₃-C₆-gliedrigen Ring, bei dem zwei Substituenten gemeinsam mit den Kohlenstoffatomen, an die sie gebunden sind, für einen gegebenenfalls einfach bis fünffach, gleich oder verschieden durch Methyl, Ethyl, Methoxy, Ethoxy, Fluor oder Chlor substituierten gesättigten oder ungesättigten C₅-C₆-Ring stehen, der durch Sauerstoff oder Schwefel unterbrochen sein kann.
- G: steht ganz besonders bevorzugt für Wasserstoff (a) oder für eine der Gruppen 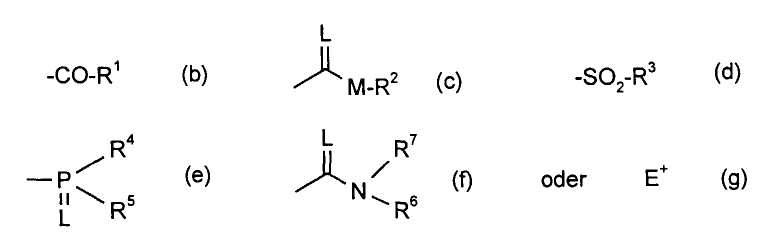 in welchen
E⁺ für ein Metallionäquivalent oder ein Ammoniumion steht,
L für Sauerstoff oder Schwefel steht und
M für Sauerstoff oder Schwefel steht.
- R¹: steht ganz besonders bevorzugt für jeweils gegebenenfalls einfach bis neunfach, gleich oder verschieden durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₂-C₄-alkyl oder gegebenenfalls durch Fluor, Chlor, Methyl oder Ethyl substituiertes Cycloalkyl mit 3 bis 6 Ringatomen, das durch 1-2 Sauerstoff- und/oder Schwefelatome unterbrochen sein kann,
für gegebenenfalls einfach bis dreifach, gleich oder verschieden durch Fluor, Chlor, Brom, Methyl, Ethyl, Propyl, i-Propyl, Methoxy, Ethoxy, Trifluormethyl, Trifluormethoxy oder Nitro substituiertes Phenyl,
für gegebenenfalls einfach bis dreifach, gleich oder verschieden durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl,
für jeweils gegebenenfalls einfach bis dreifach, gleich oder verschieden durch Fluor, Chlor, Brom, Methyl oder Ethyl substituiertes Pyridyl, Thienyl oder Furanyl,
für gegebenenfalls einfach bis dreifach, gleich oder verschieden durch Fluor, Chlor, Methyl, Ethyl substituiertes Phenoxy-C₁-C₄-alkyl oder
für jeweils gegebenenfalls einfach bis dreifach, gleich oder verschieden durch Fluor, Chlor, Amino, Methyl oder Ethyl substituiertes Pyridyloxy-C₁-C₄-alkyl, Pyrimidyloxy-C₁-C₄-alkyl und Thiazolyloxy-C₁-C₄-alkyl.
- R²: steht ganz besonders bevorzugt für jeweils gegebenenfalls einfach bis neunfach, gleich oder verschieden durch Fluor oder Chlor substituiertes C₁-C₁₄-Alkyl, C₂-C₁₄-Alkenyl, C₁-C₄-Alkoxy-C₂-C₆-alkyl oder C₁-C₄-Polyalkoxy-C₂-C₆-alkyl, für gegebenenfalls durch Fluor, Chlor, Methyl, Ethyl, Methoxy oder Ethoxy substituiertes C₃-C₆-Cycloalkyl,
oder für jeweils gegebenenfalls einfach bis dreifach, gleich oder verschieden durch Fluor, Chlor, Nitro, Methyl, Ethyl, Propyl, i-Propyl, Methoxy, Ethoxy oder Trifluormethyl substituiertes Phenyl oder Benzyl.
- R³, R⁴ und R⁵: stehen unabhängig voneinander ganz besonders bevorzugt für jeweils gegebenenfalls einfach bis fünffach, gleich oder verschieden durch Fluor oder Chlor substituiertes C₁-C₄-Alkyl, C₁-C₄-Alkoxy, C₁-C₄-Alkylamino, Di-(C₁-C₄-Alkyl)-amino, C₁-C₄-Alkylthio, für jeweils gegebenenfalls einfach bis dreifach, gleich oder verschieden durch Fluor, Chlor, Brom, Nitro, Cyano, C₁-C₂-Alkoxy, C₁-C₂-Fluoralkoxy, C₁-C₂-Chloralkoxy, C₁-C₂-Alkylthio, C₁-C₂-Fluoralkylthio, C₁-C₂-Chloralkylthio oder C₁-C₃-Alkyl substituiertes Phenyl, Benzyl, Phenoxy oder Phenylthio.
- R⁶ und R⁷: stehen unabhängig voneinander ganz besonders bevorzugt für Wasserstoff, jeweils gegebenenfalls einfach bis neunfach, gleich oder verschieden durch Fluor, Chlor oder Brom substituiertes C₁-C₁₀-Alkyl, C₁-C₁₀-Alkoxy oder C₁-C₁₀-Alkoxy-(C₁-C₁₀)alkyl, für gegebenenfalls einfach bis dreifach, gleich oder verschieden durch Fluor, Chlor, Brom, C₁-C₂-Halogenalkyl, C₁-C₂-Alkyl oder C₁-C₄-Alkoxy substituiertes Phenyl, für gegebenenfalls einfach bis dreifach, gleich oder verschieden durch Fluor, Chlor, Brom, C₁-C₄-Alkyl, C₁-C₄-Halogenalkyl oder C₁-C₄-Alkoxy substituiertes Benzyl oder zusammen für einen gegebenenfalls durch Sauerstoff oder Schwefel unterbrochenen C₄-C₆-Alkylenrest.

Dabei sind die stereoisomerenreinen Formen (Diastereomere, Enantiomere) sowie Gemische der Stereoisomeren von Verbindungen der Formel (I) jeweils eingeschlossen.

Kohlenwasserstoffteste können, auch in Verbindung mit Heteroatomen, wie z.B. in Alkoxy oder Alkenylthio, jeweils soweit möglich, geradkettig oder verzweigt sein.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß besonders bevorzugt werden die Verbindungen der allgemeinen Formel (I), in welchen eine Kombination der vorstehend als besonders bevorzugt aufgeführten Bedeutungen vorliegt.

Erfindungsgemäß ganz besonders bevorzugt werden die Verbindungen der allgemeinen Formel (I), in welchen eine Kombination der vorstehend als ganz besonders bevorzugt aufgeführten Bedeutungen vorliegt.

Im einzelnen seien außer den bei den Herstellungsbeispielen genannten Verbindungen die folgenden 3-Aryl-4-hydroxy-Δ³-dihydrothiophen-2-on-Derivate der Formel (Ia) genannt:

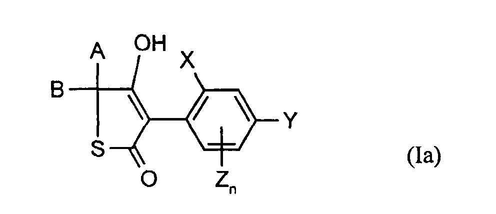

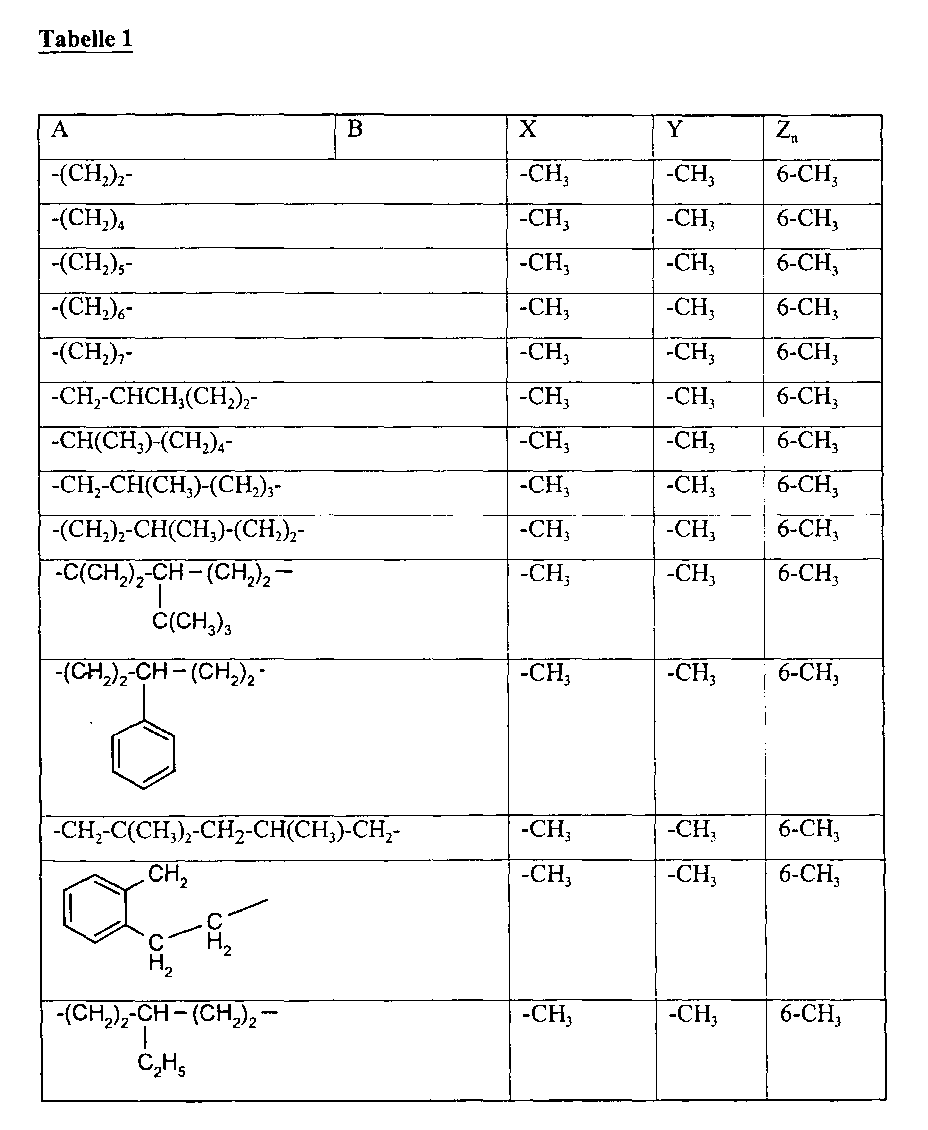

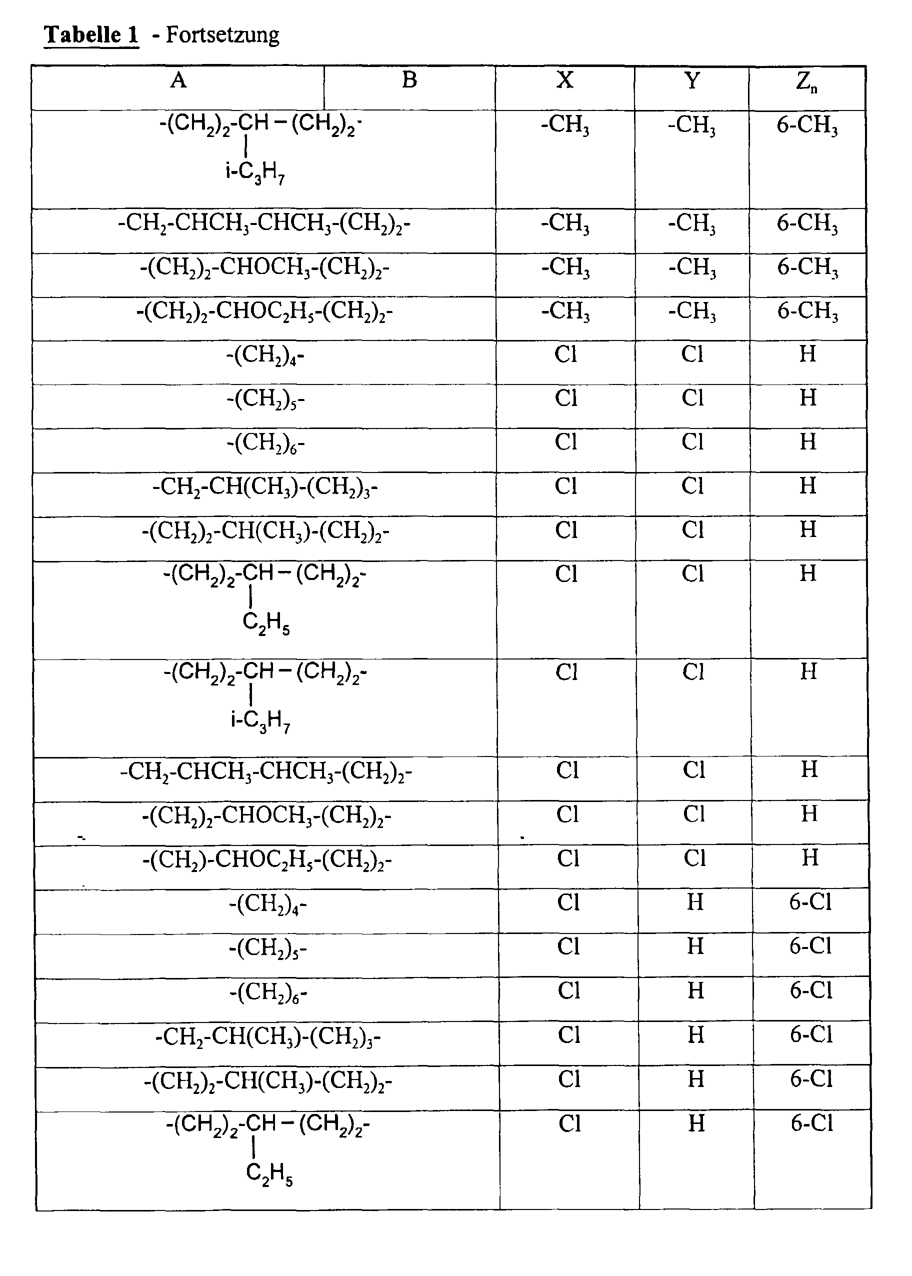

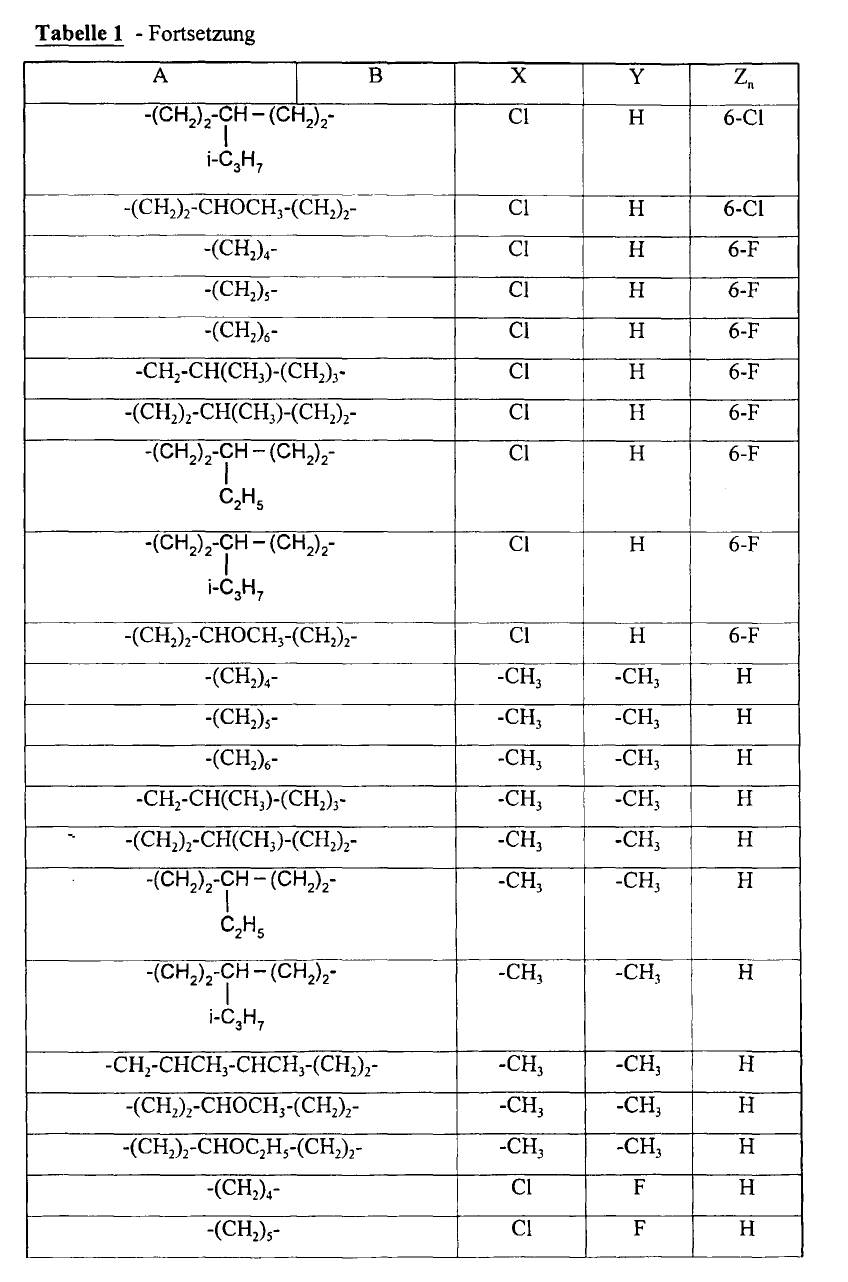

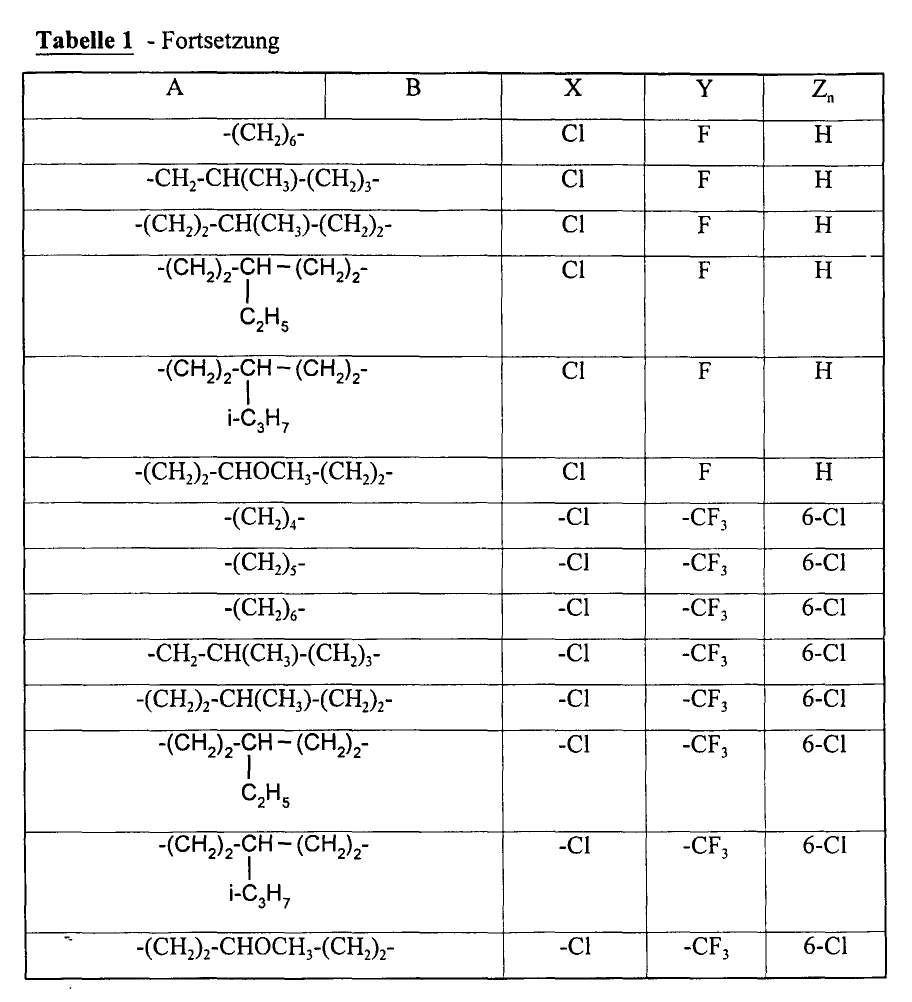

Im einzelnen seien außer den bei den Herstellungsbeispielen genannten Verbindungen die folgenden 3-Aryl-4-hydroxy-Δ³-dihydrothiophen-2-on-Derivate der Formel (Ib) genannt (Tabelle 2):

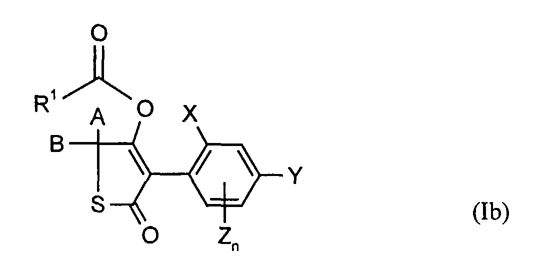

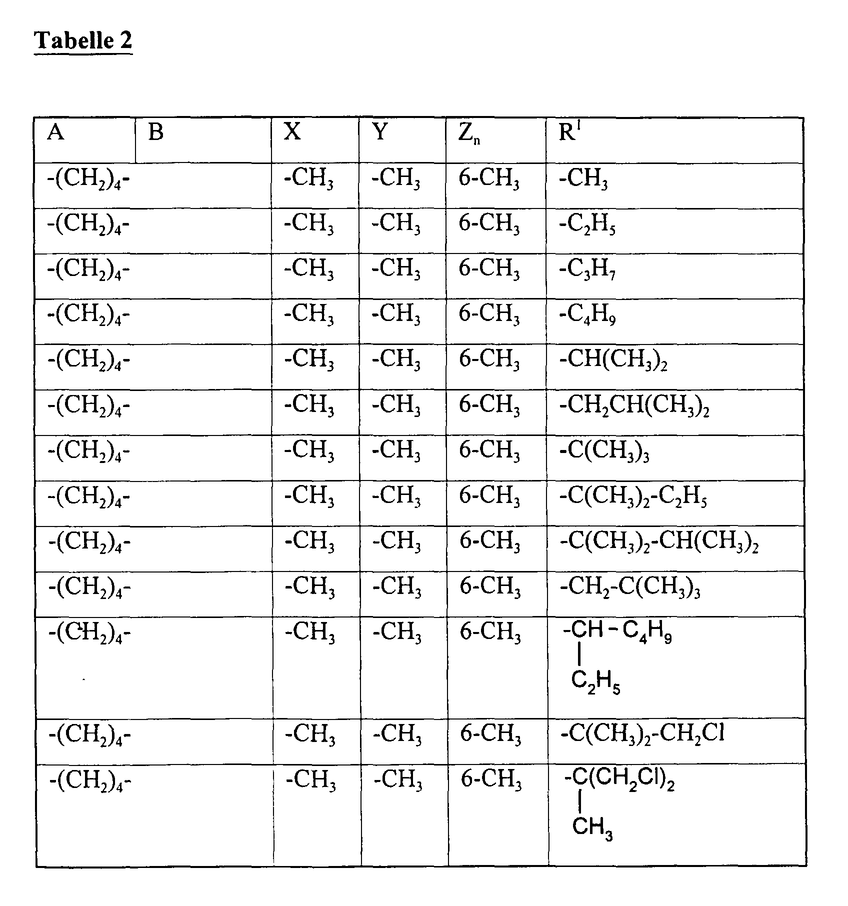

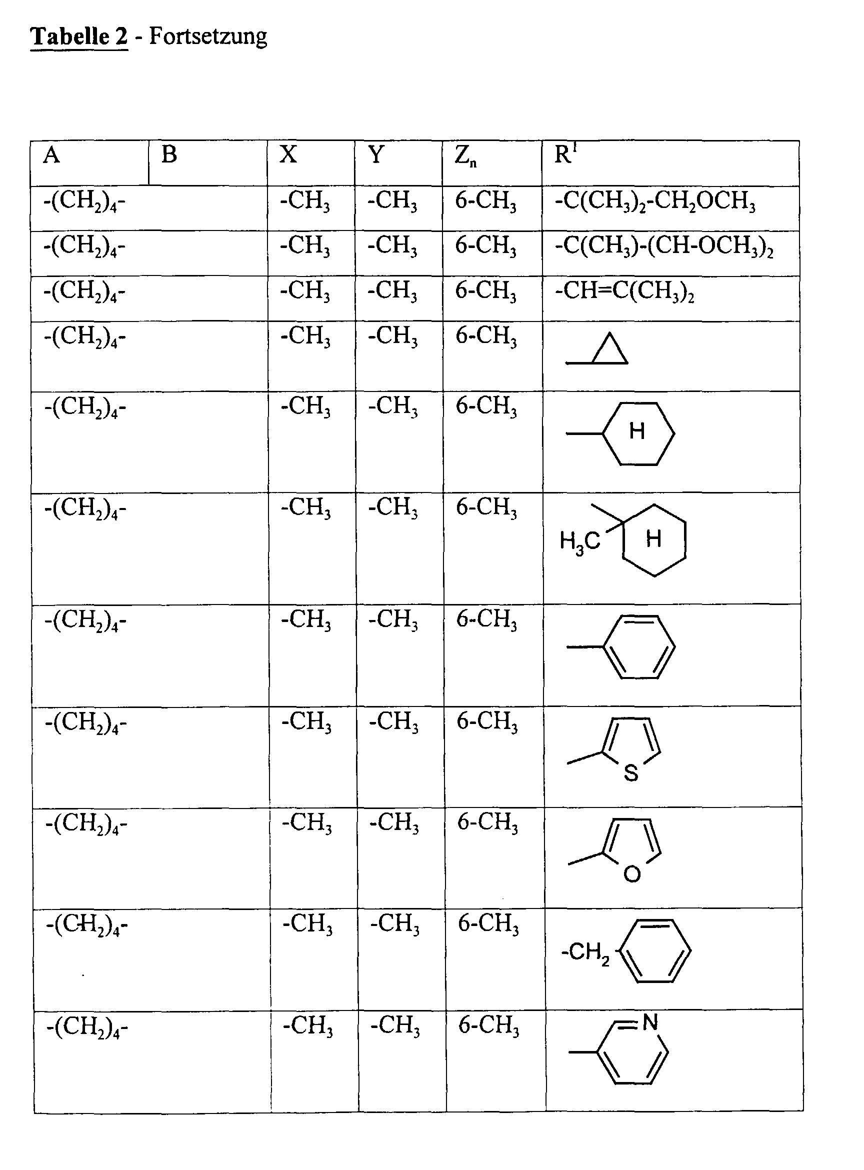

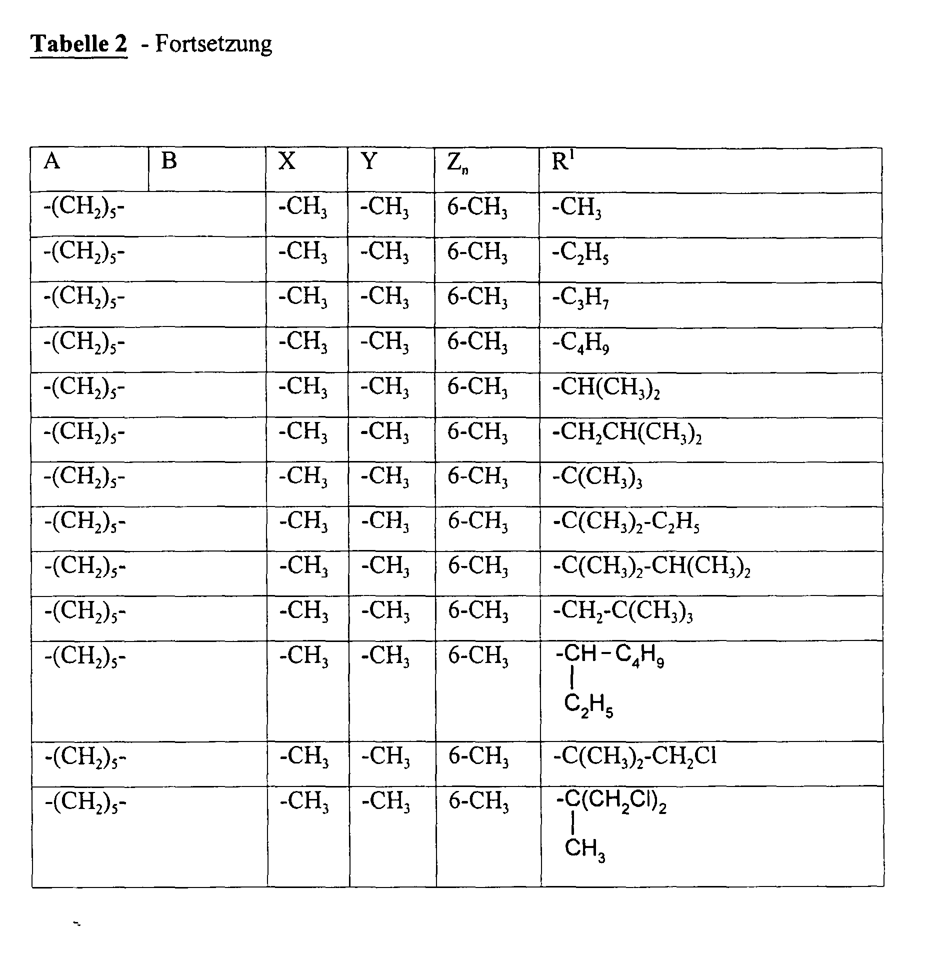

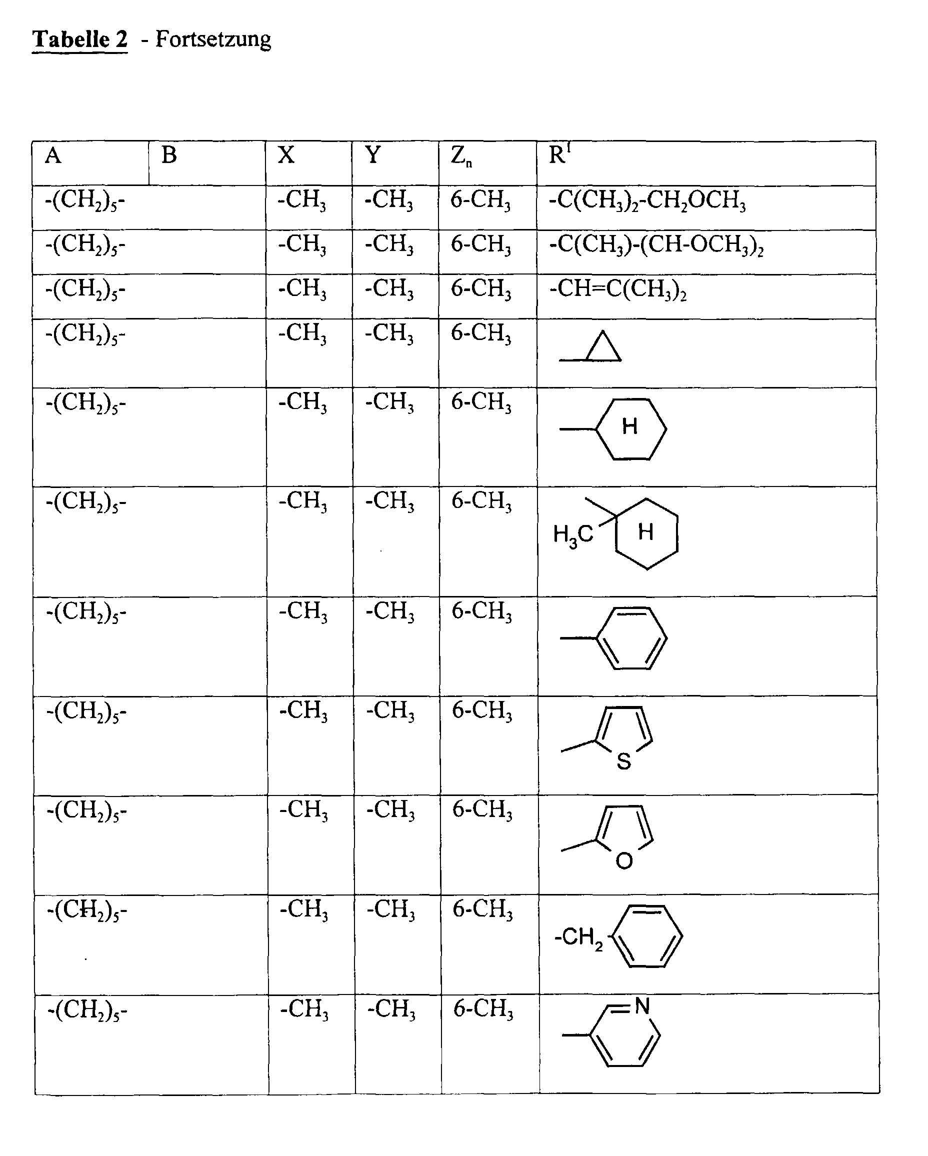

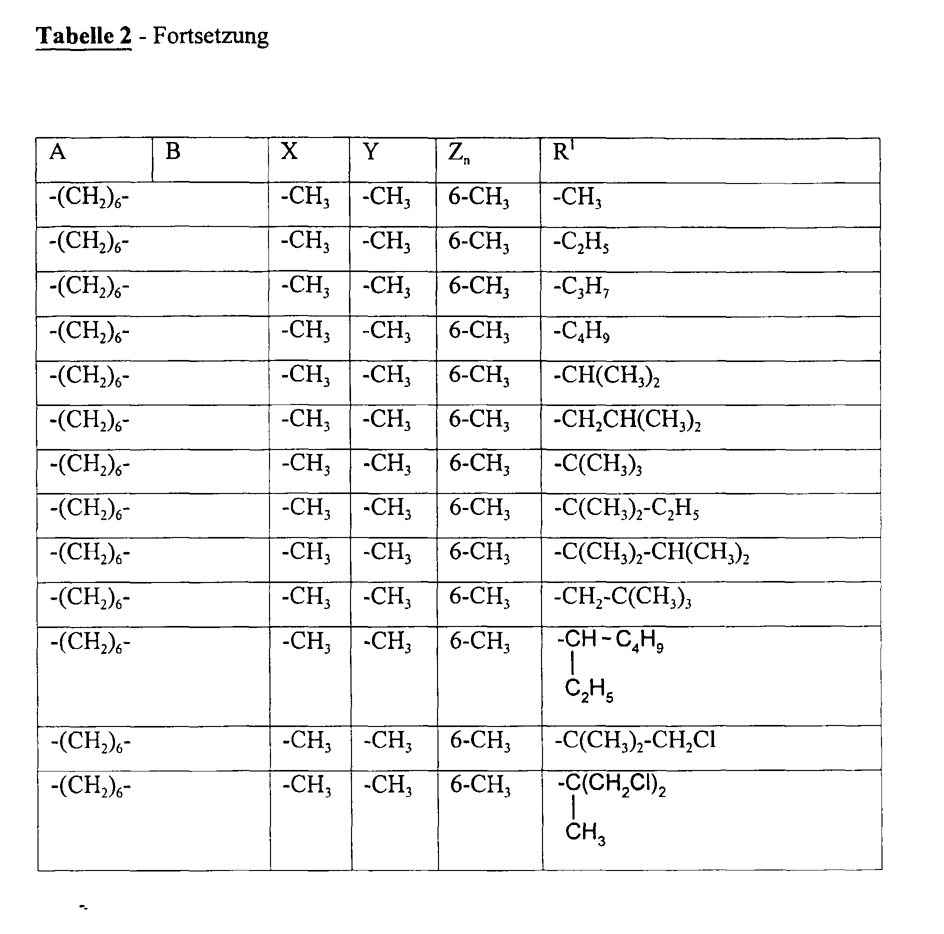

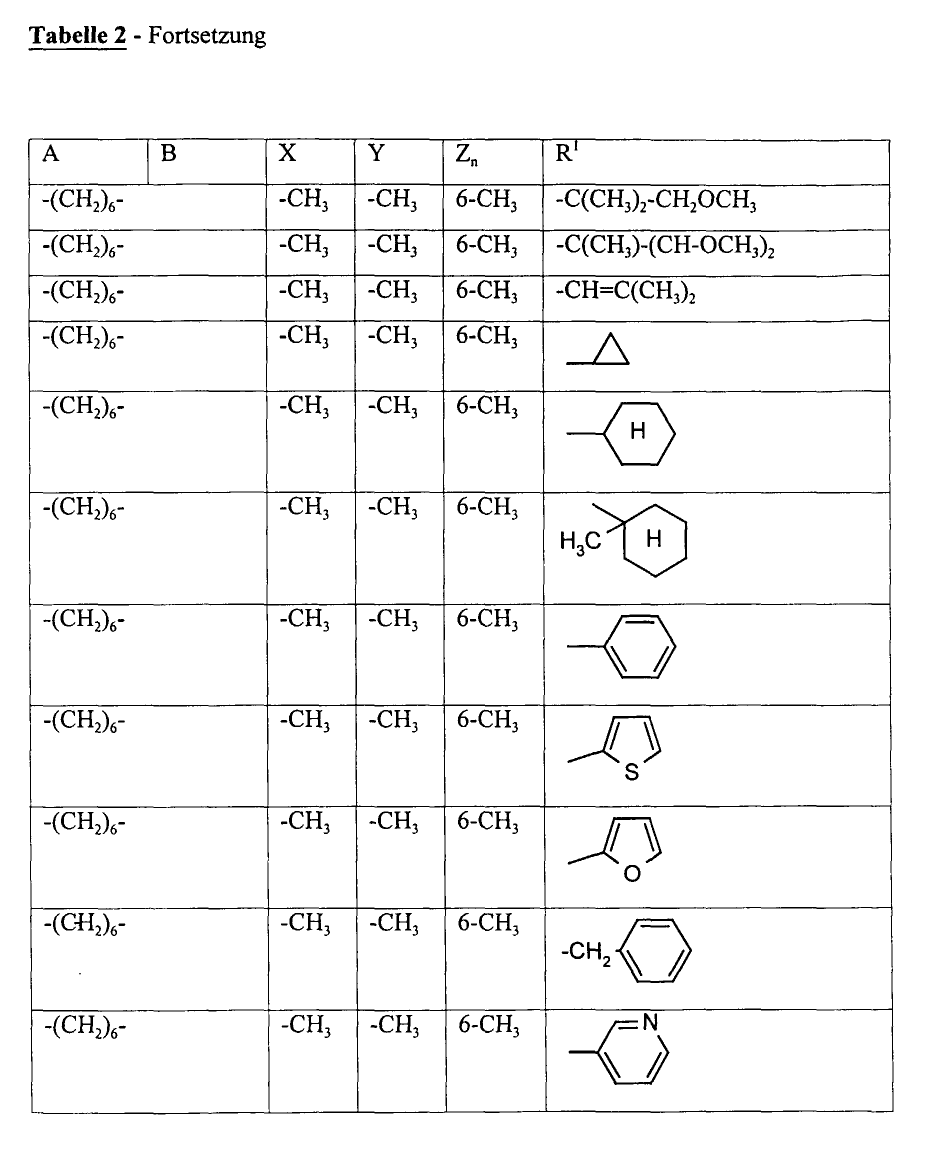

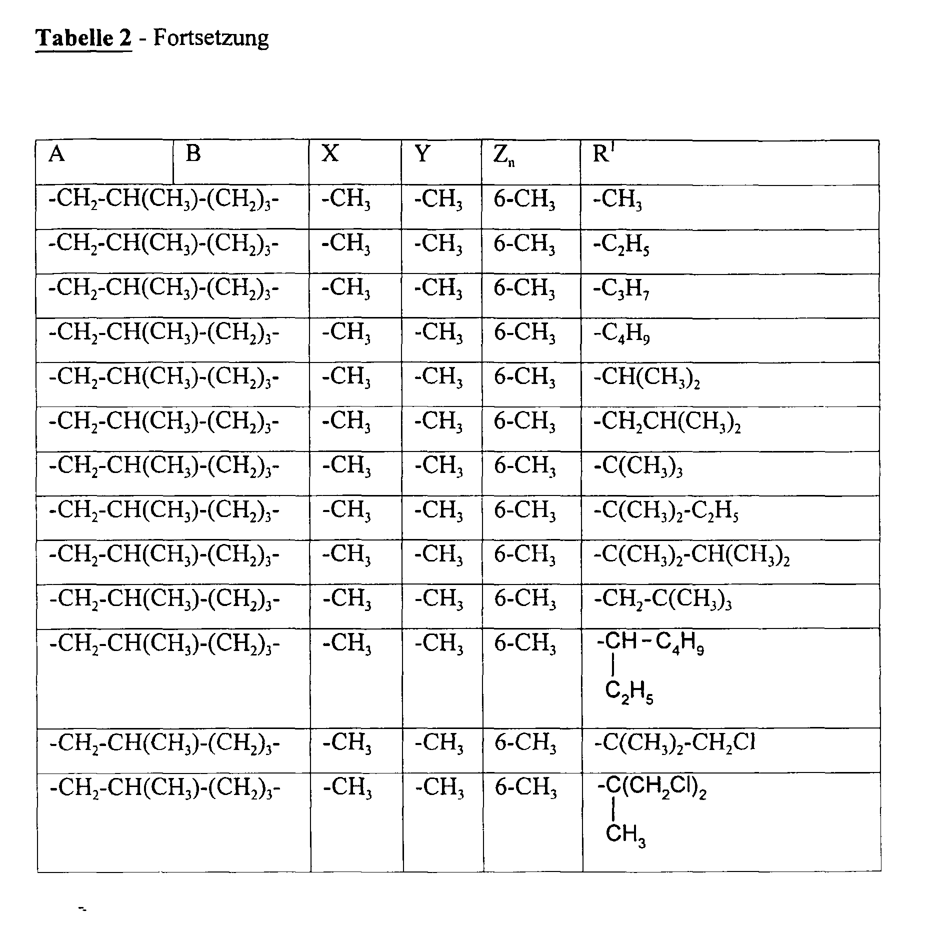

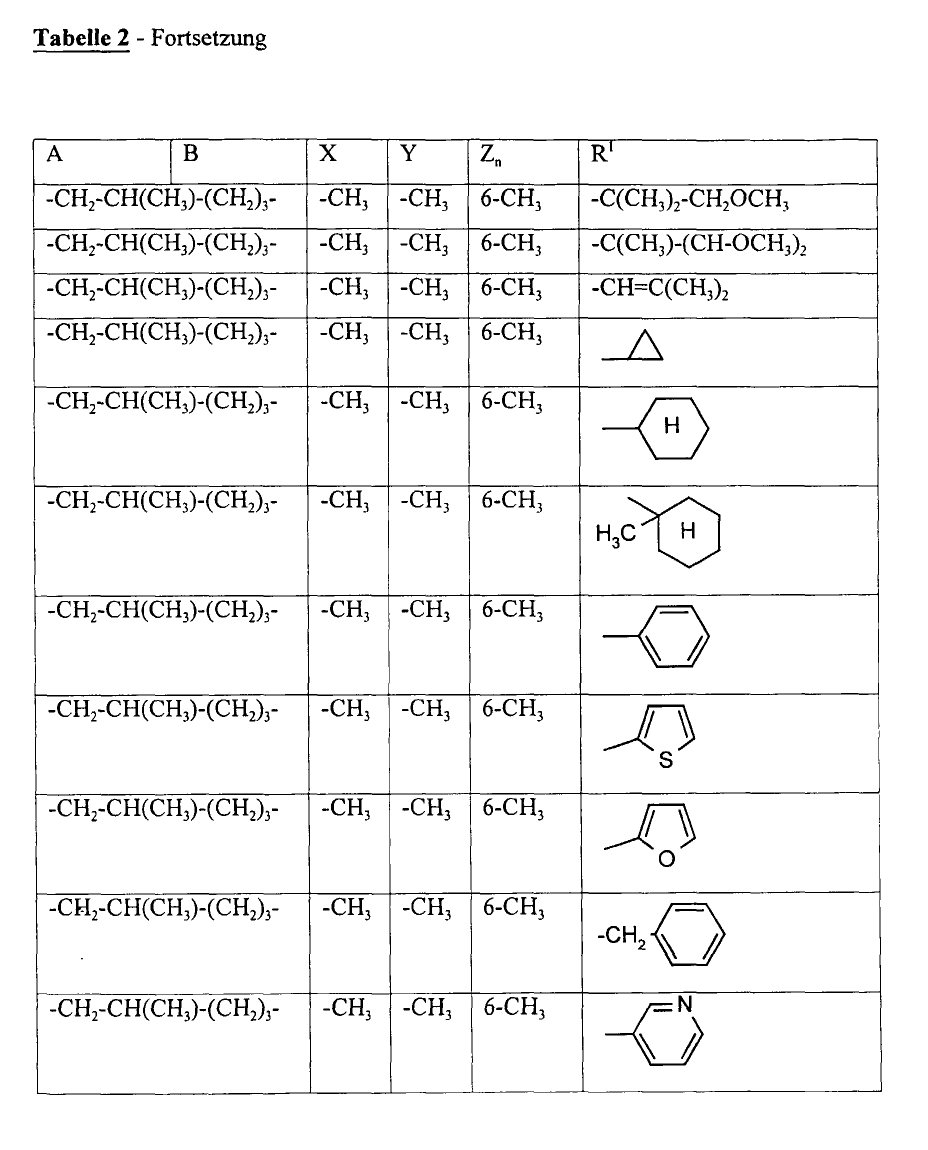

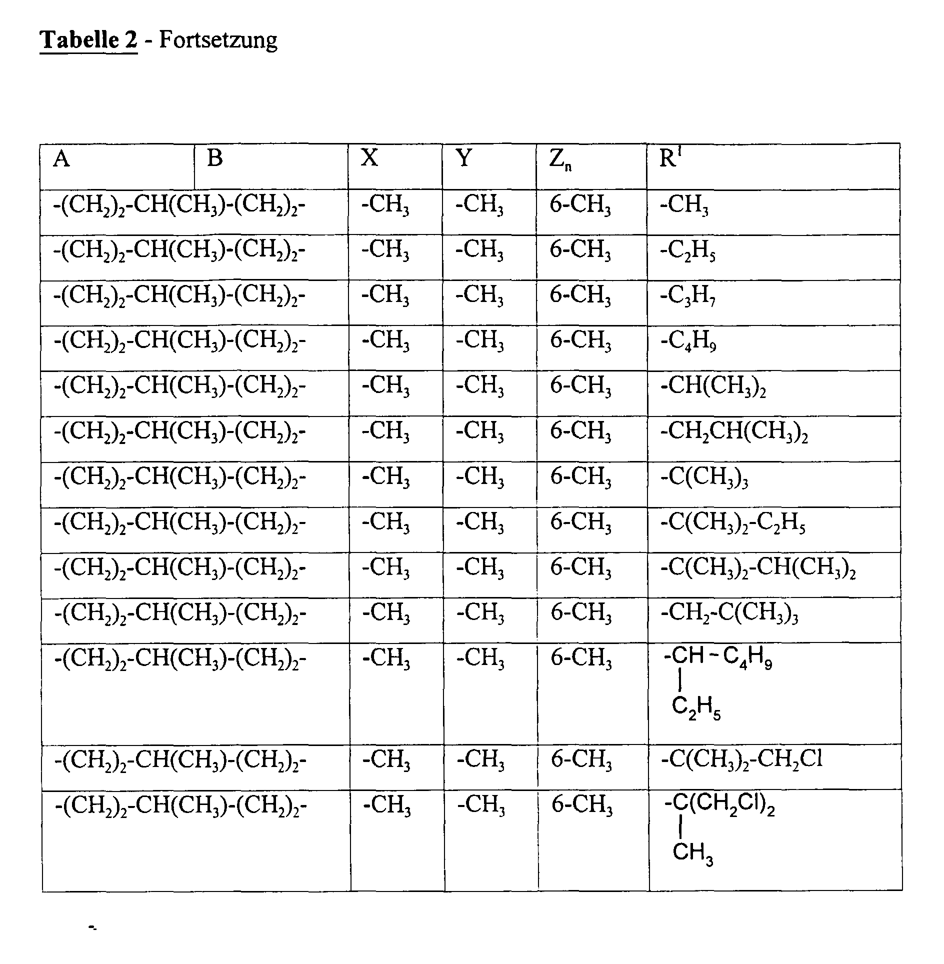

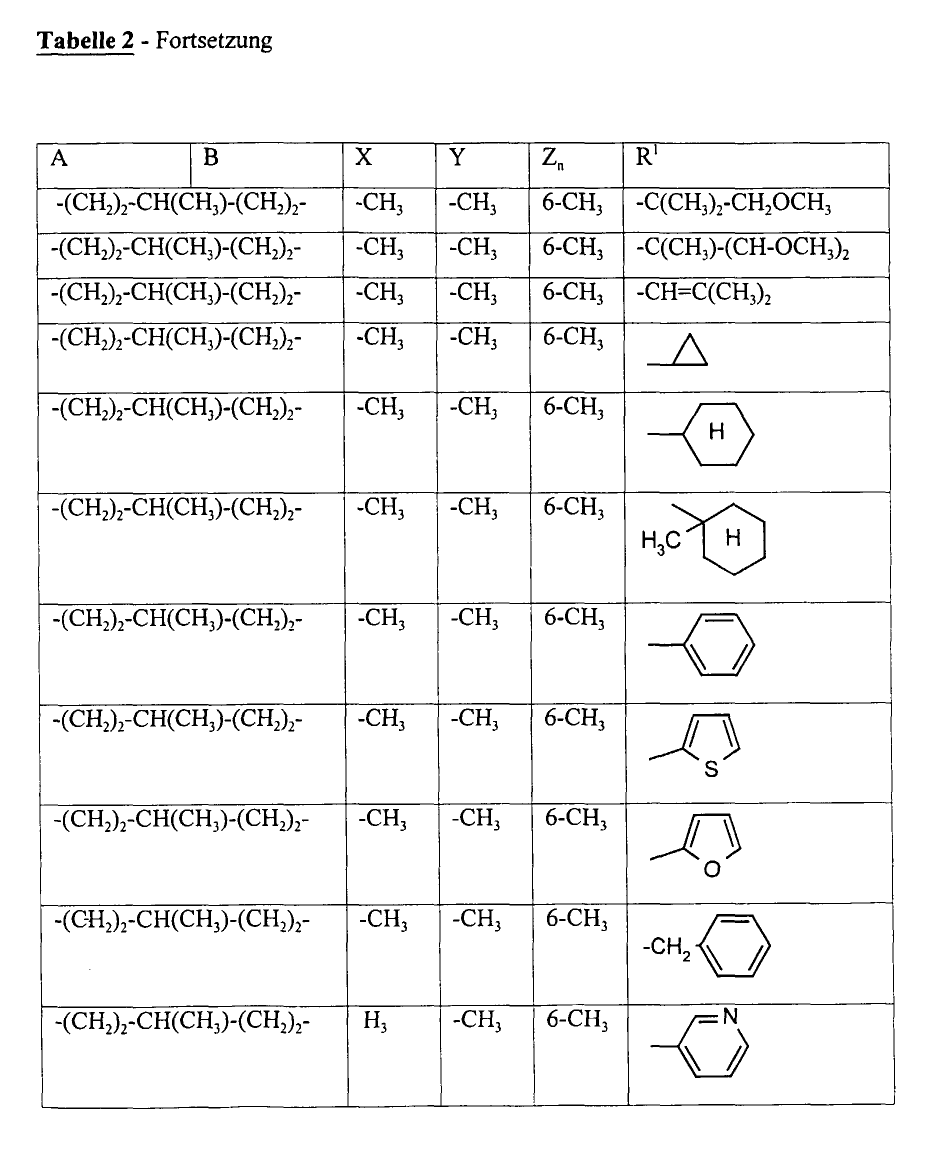

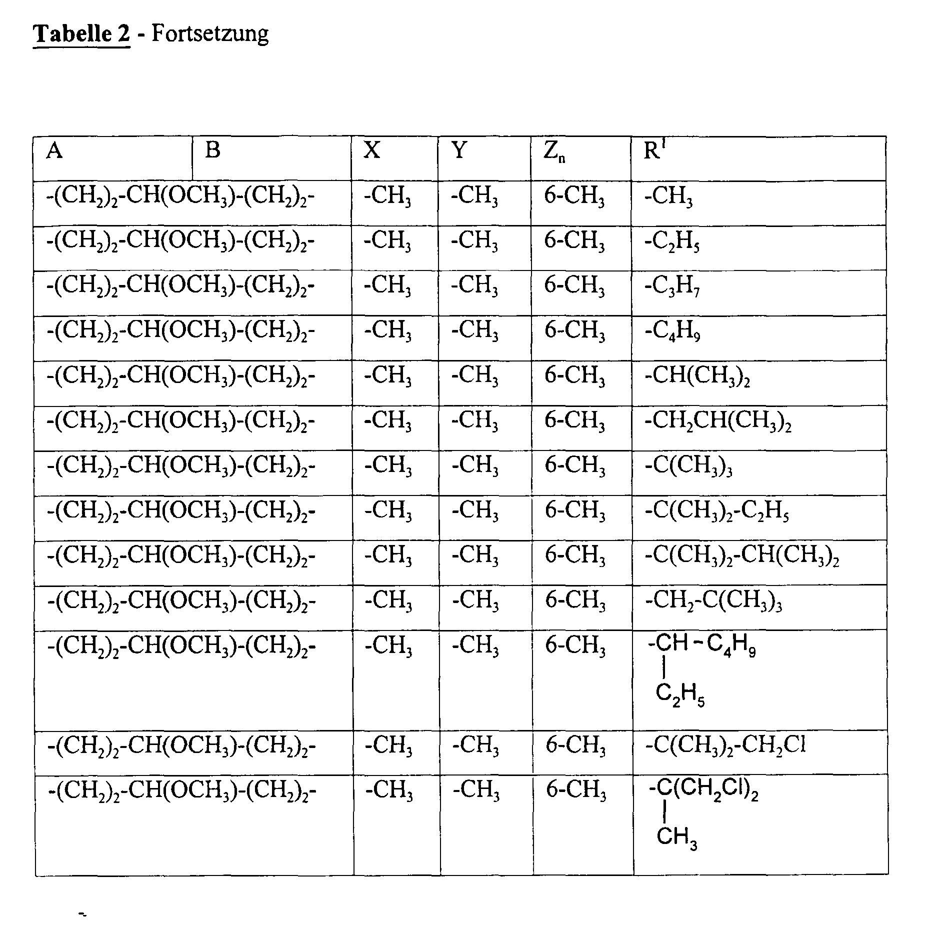

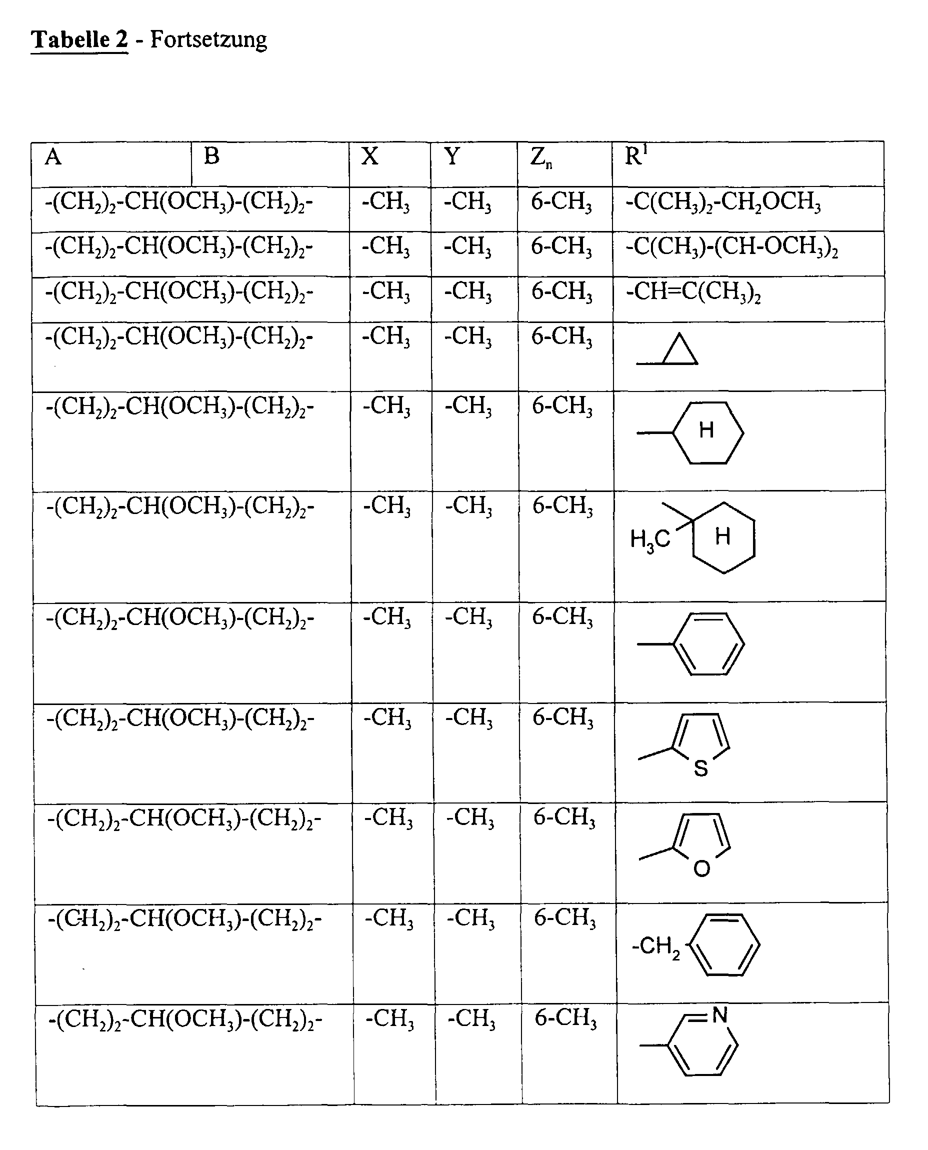

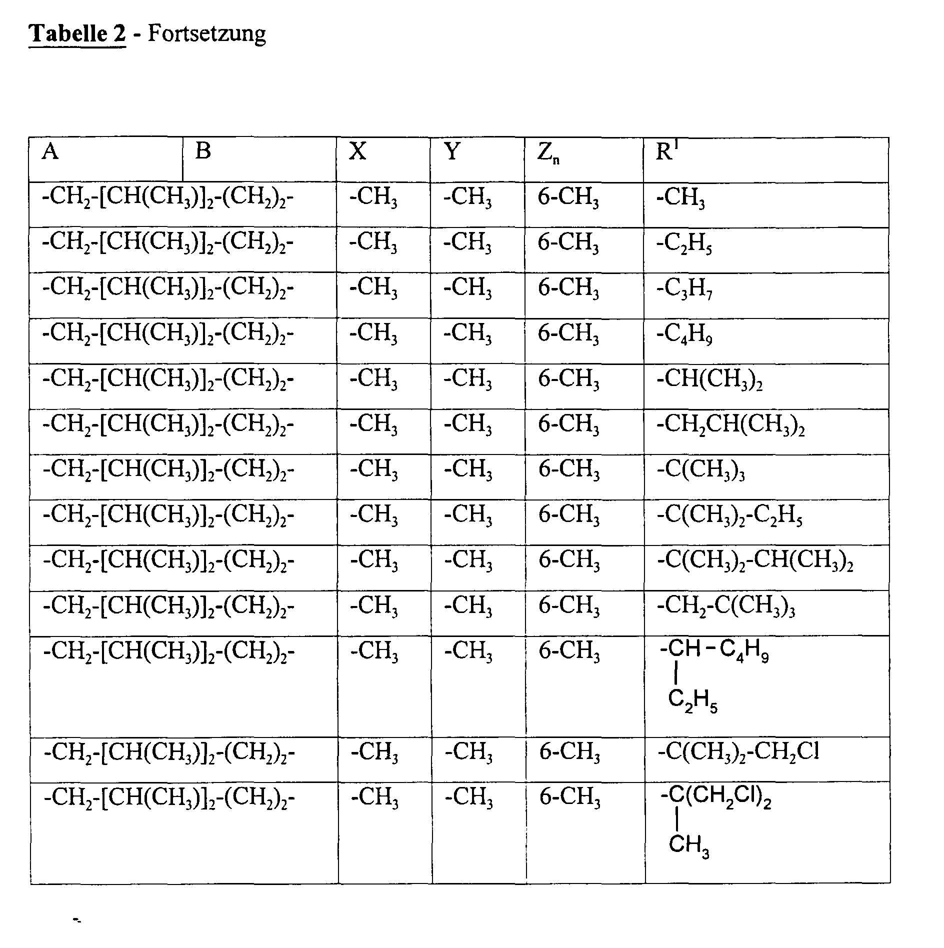

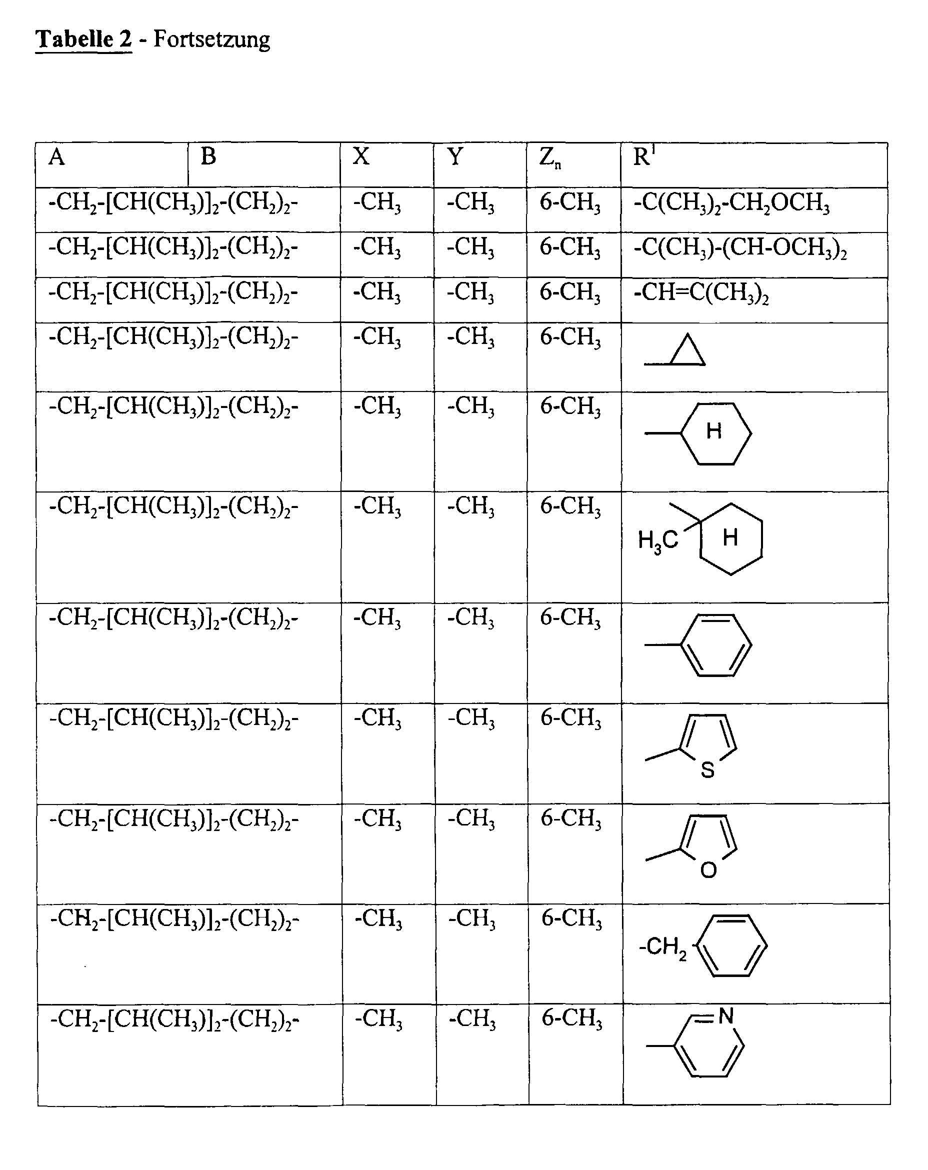

### Tabelle 3

Die Tabelle 3 enthält die Verbindungen der Formel (Ib), in welcher A, B und R¹ die in der Tabelle 2 genannten Bedeutungen haben und X und Y jeweils für Chlor stehen und Zₙ für Wasserstoffsteht.

### Tabelle 4

Die Tabelle 4 enthält die Verbindungen der Formel (Ib), in welcher A, B und R¹ die in der Tabelle 2 genannten Bedeutungen haben und X und Y jeweils für CH₃ stehen und Zₙ für Wasserstoff steht.

Im einzelnen seien außer den bei den Herstellungsbeispielen genannten Verbindungen die folgenden 3-Aryl-4-hydroxy-Δ³-dihydrothiophen-2-on-Derivate der Formel (Ic) genannt (Tabelle 5):

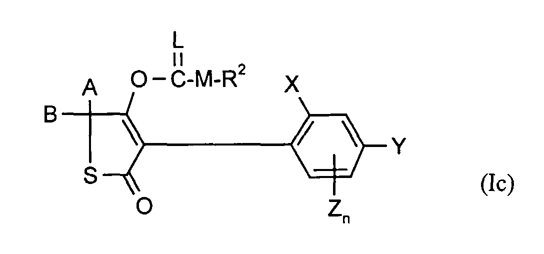

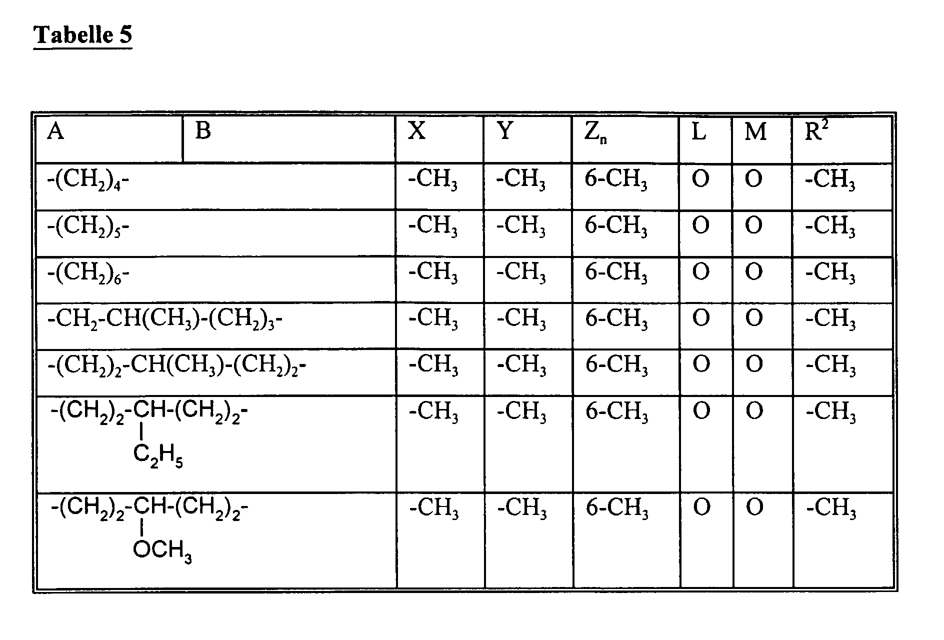

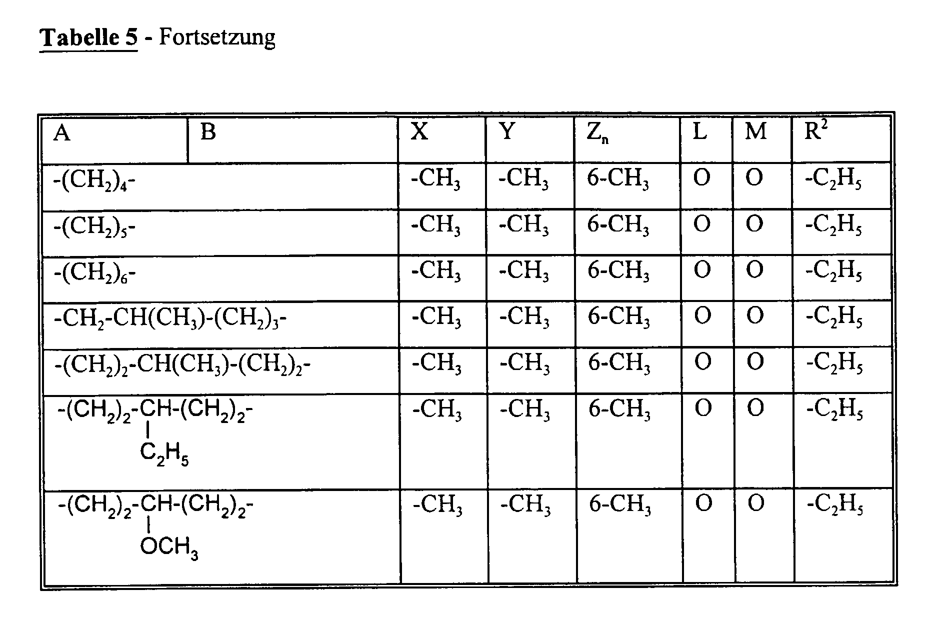

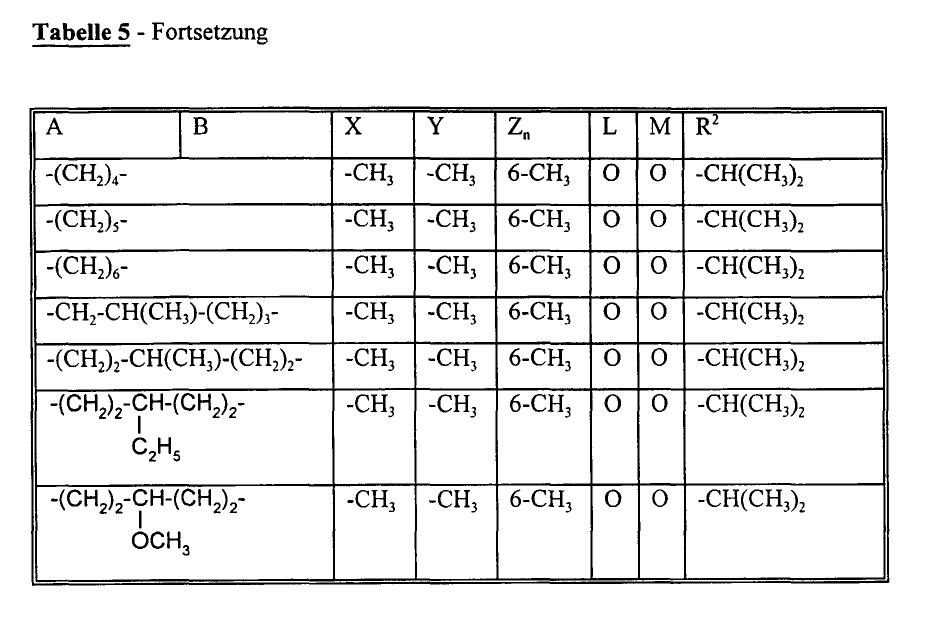

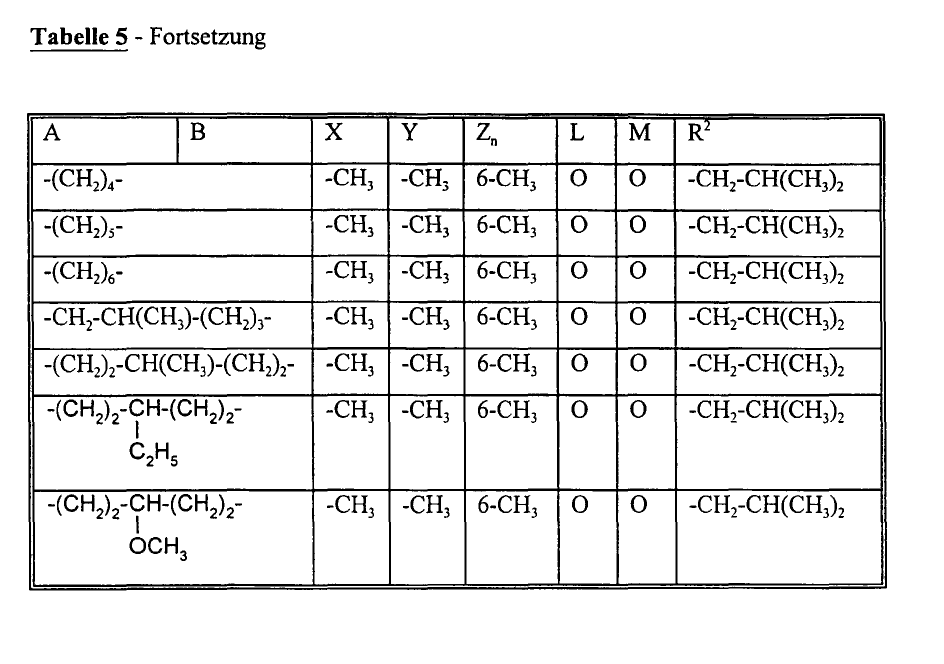

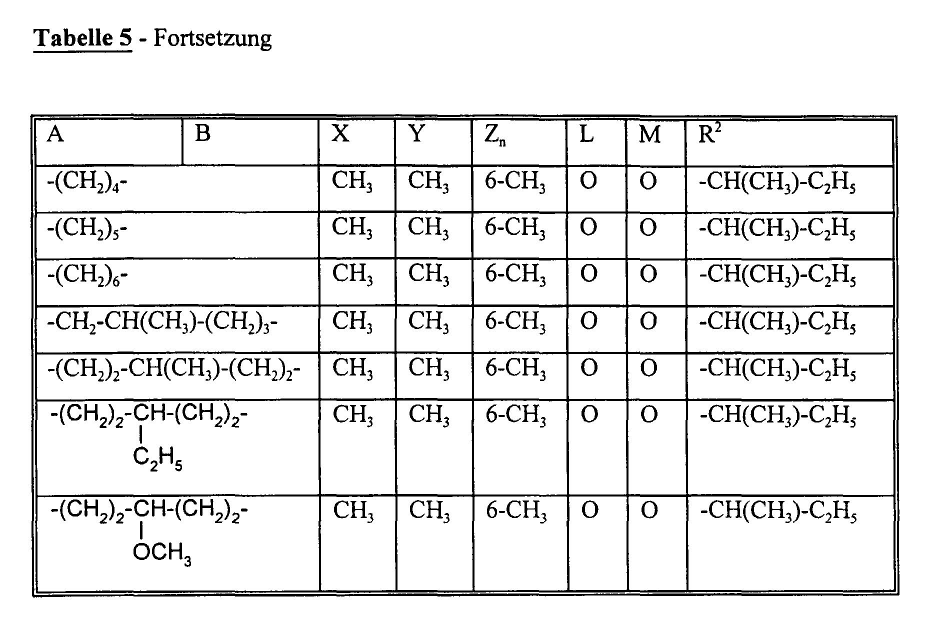

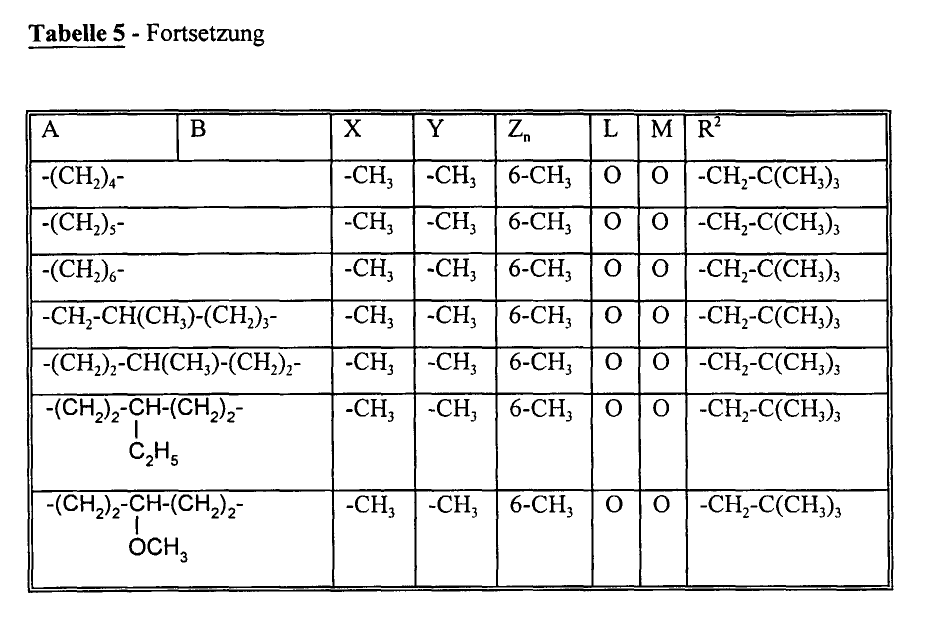

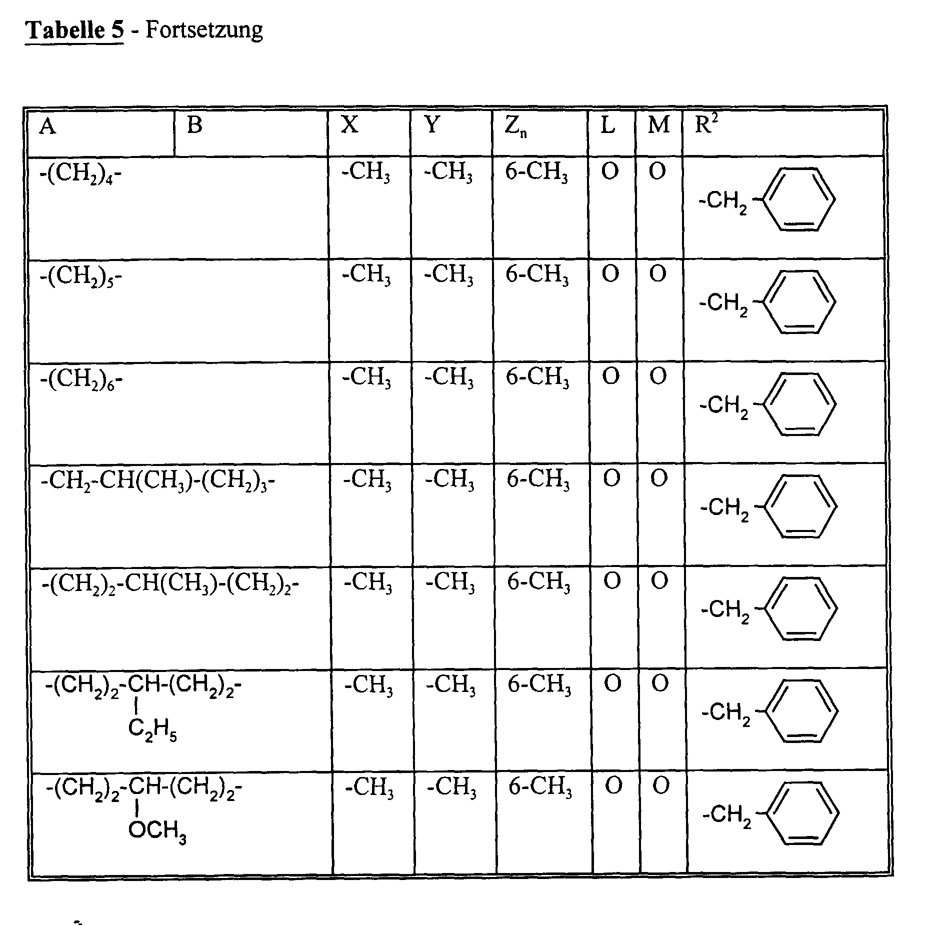

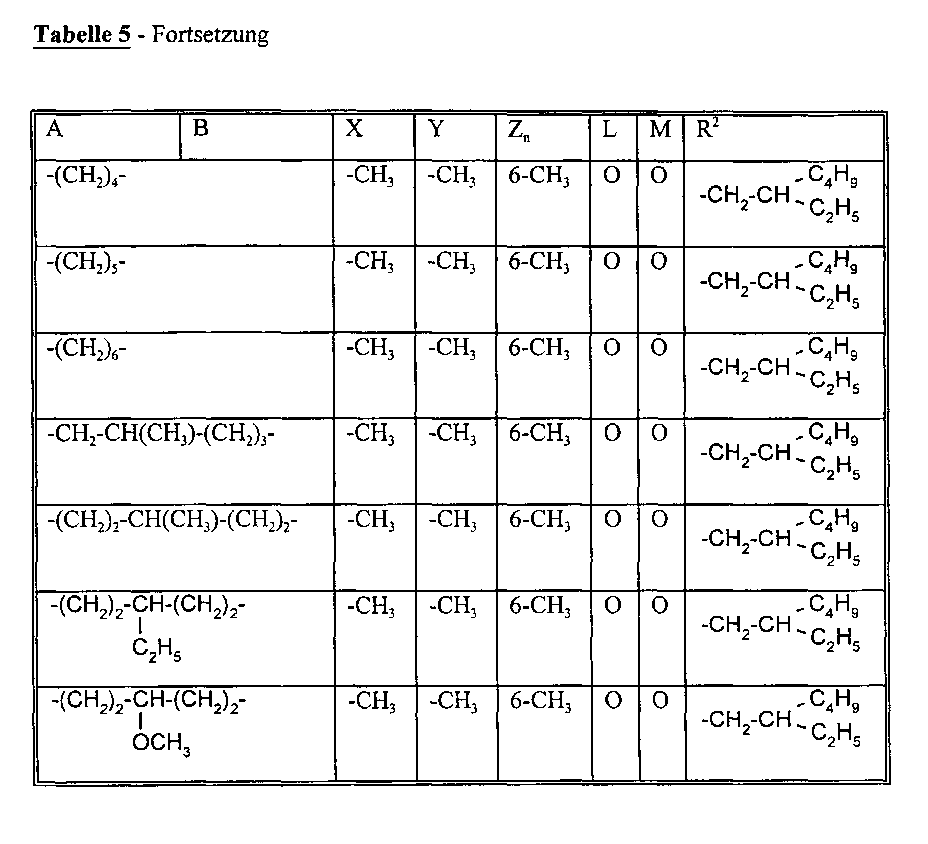

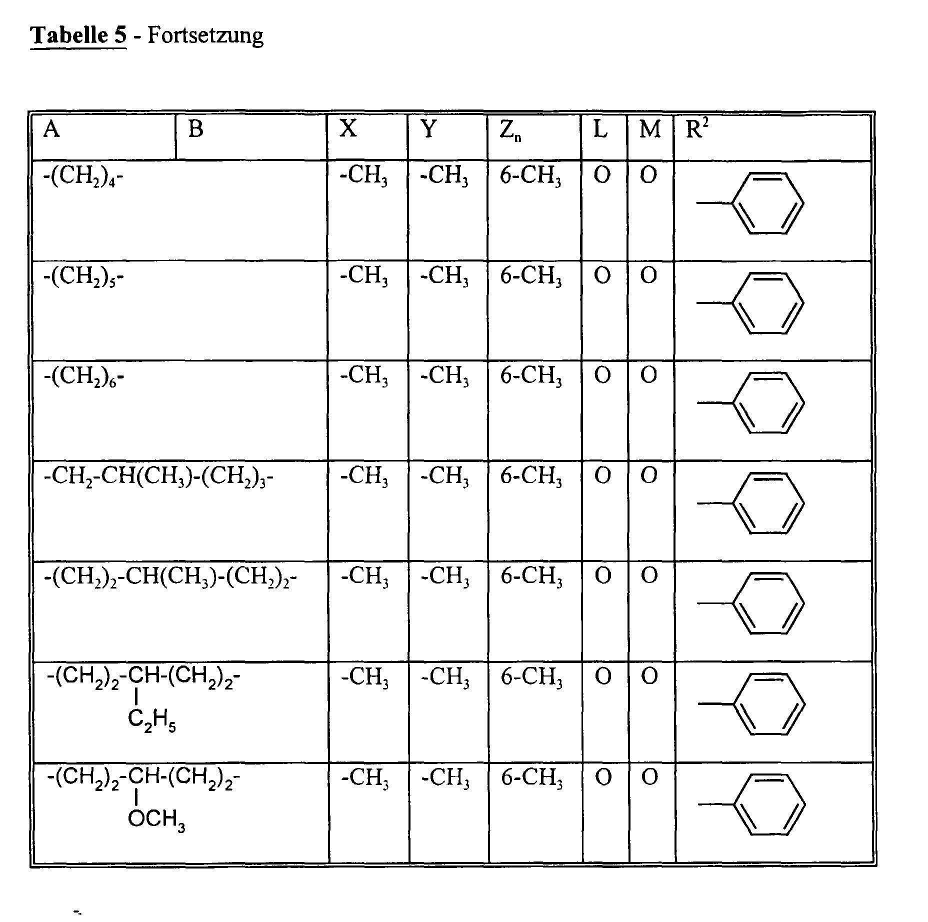

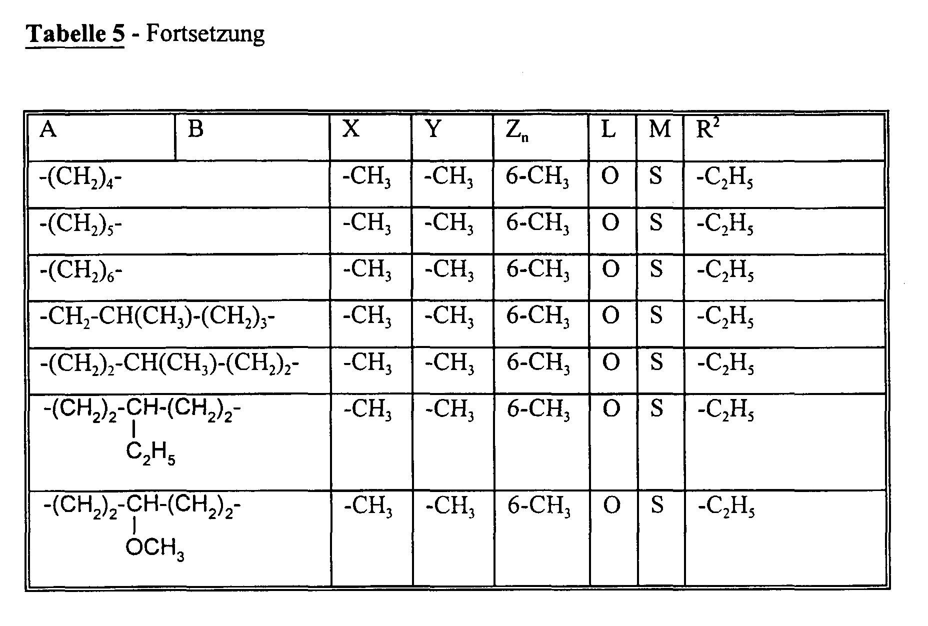

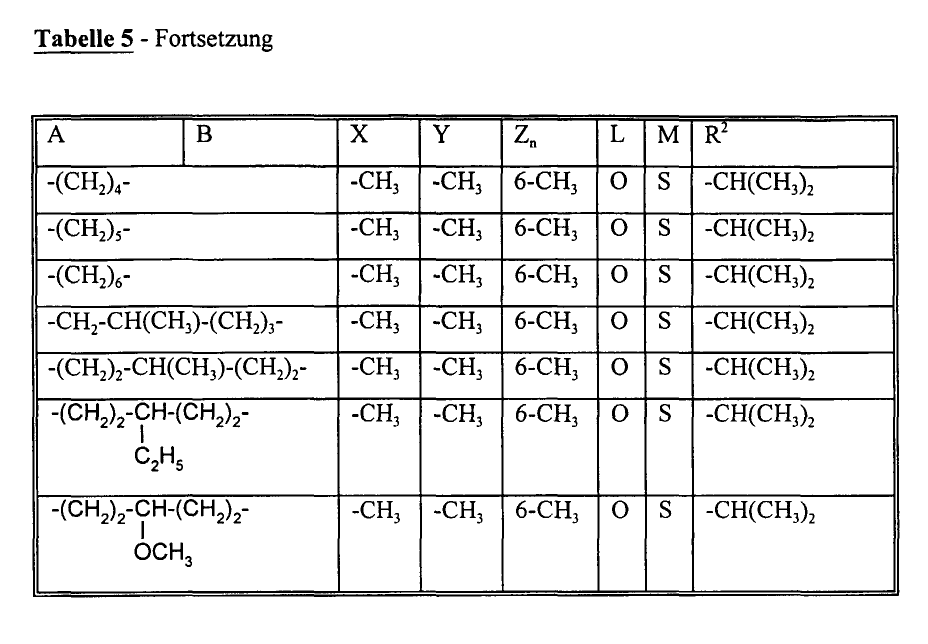

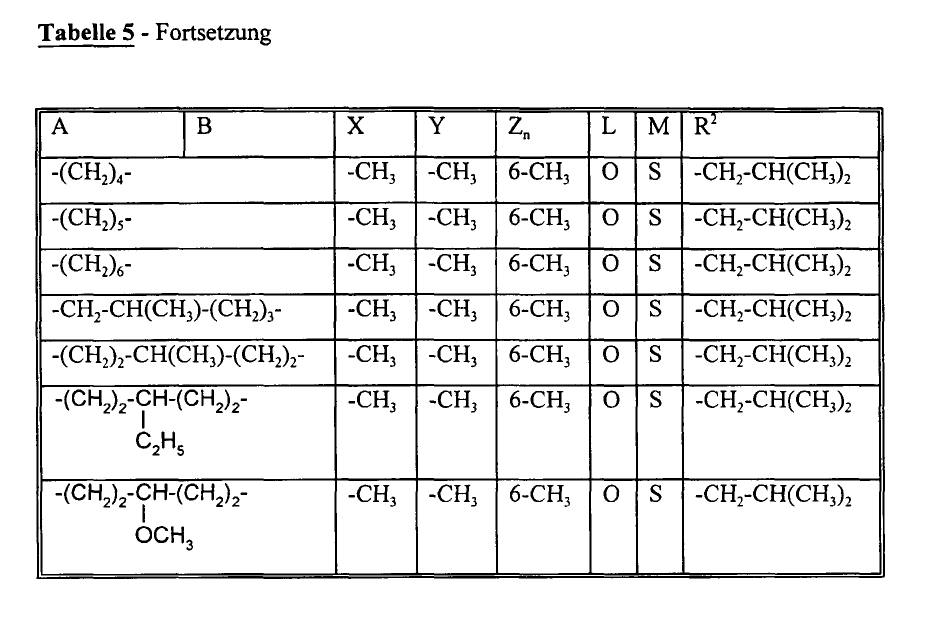

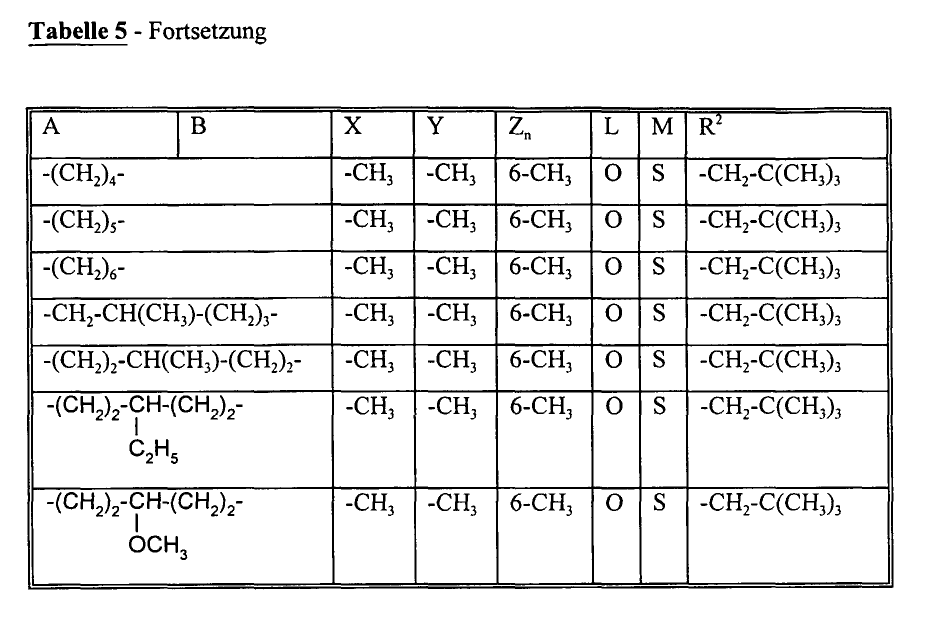

### Tabelle 6

Die Tabelle 6 enthält die Verbindungen der Formel (Ic), in welcher A, B, L, M und R² die in der Tabelle 5 genannten Bedeutungen haben und X und Y jeweils für Chlor stehen und Zₙ für Wasserstoff steht.

### Tabelle 7

Die Tabelle 7 enthält die Verbindungen der Formel (Ic), in welcher A, B, L, M und R² die in der Tabelle 5 genannten Bedeutungen haben und X und Y jeweils für CH₃ stehen und Zₙ für Wasserstoff steht.

Im einzelnen seien außer den bei den Herstellungsbeispielen genannten Verbindungen die folgenden 3-Aryl-4-hydroxy-Δ³-dihydrothiophen-2-on-Derivate der Formel (Id) genannt:

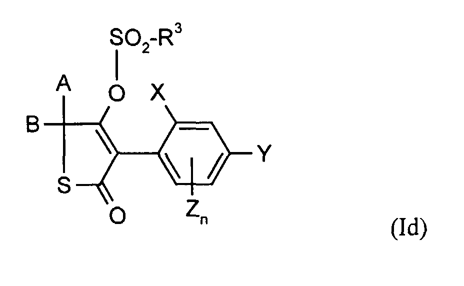

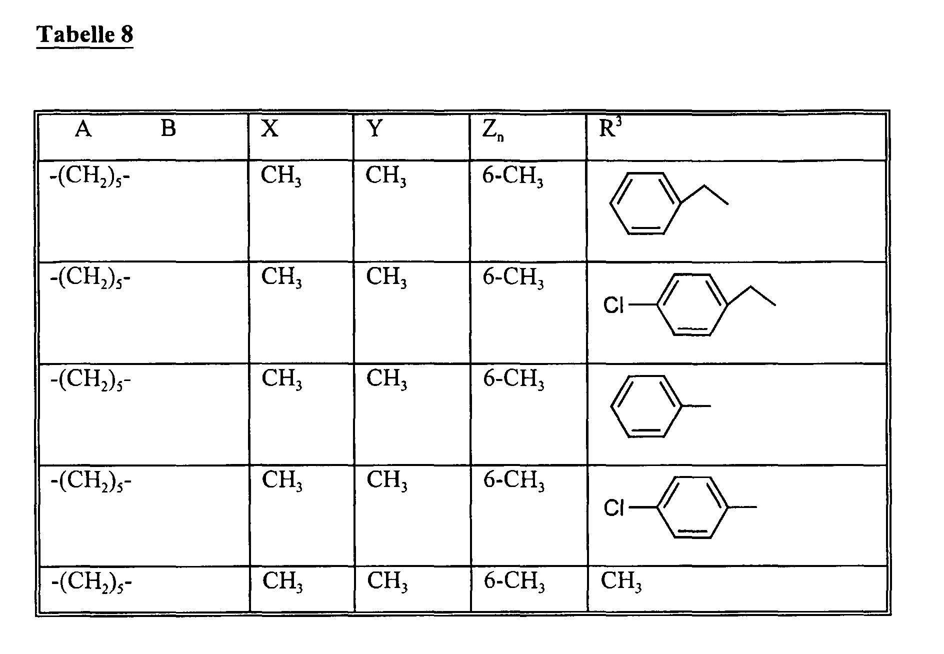

Im einzelnen seien außer den bei den Herstellungsbeispielen genannten Verbindungen die folgenden 3-Aryl-4-hydroxy-Δ³-dihydrothiophen-2-on-Derivate der Formel (Ie) genannt:

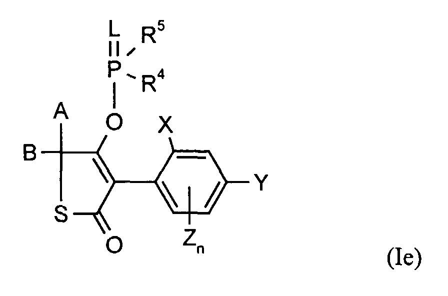

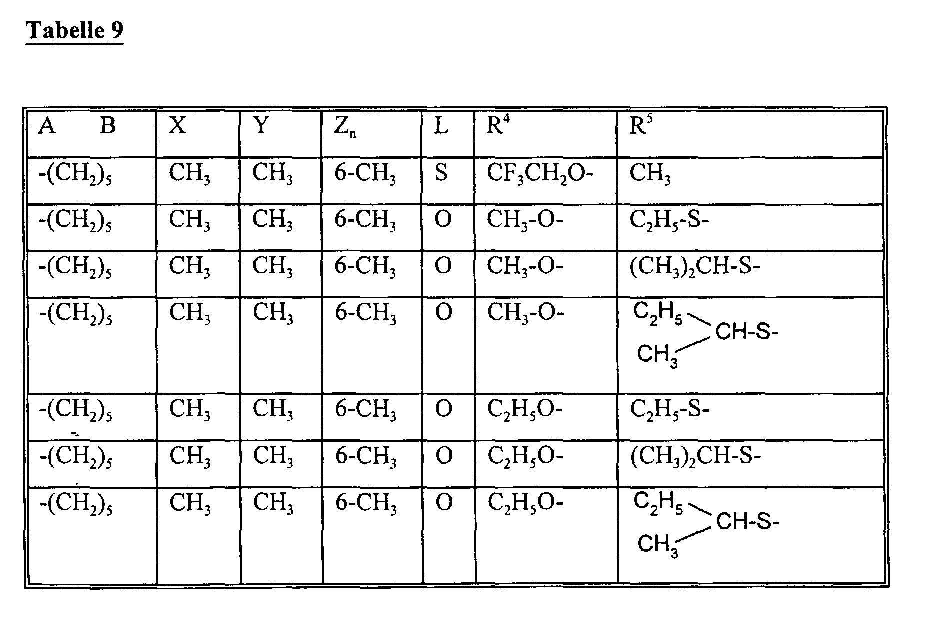

Im einzelnen seien außer den bei den Herstellungsbeispielen genannten Verbindungen die folgenden 3-Aryl-4-hydroxy-Δ³-dihydrothiophen-2-on-Derivate der Formel (If) genannt (Tabelle 10):

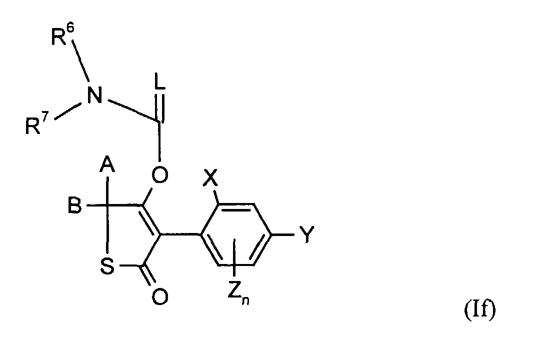

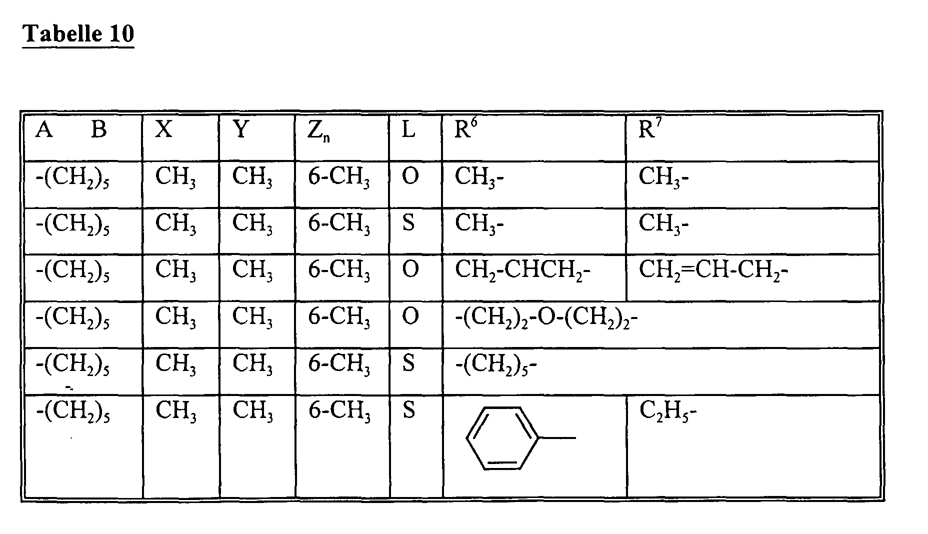

Im einzelnen seien außer den bei den Herstellungsbeispielen genannten Verbindungen die folgenden 3-Aryl-4-hydroxy-Δ³-dihydrothiophen-2-on-Derivate der Formel (Ig) genannt (Tabelle 11):

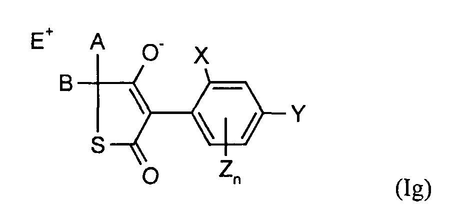

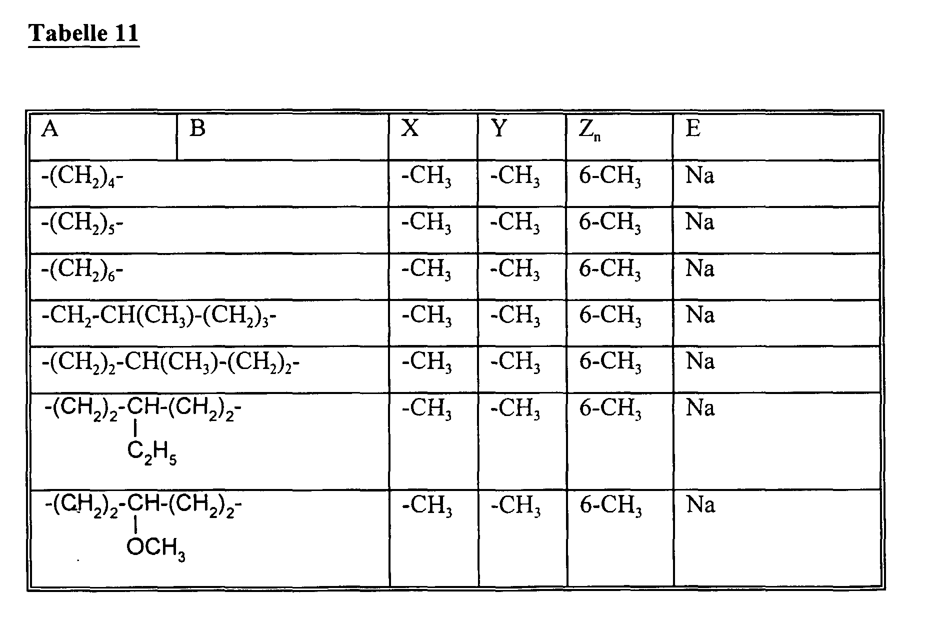

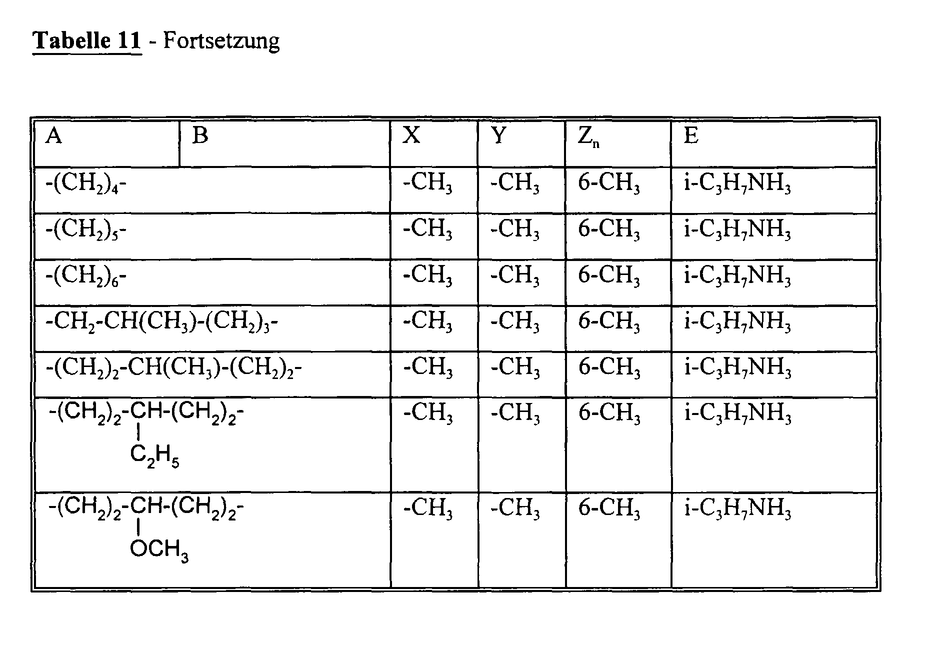

### Tabelle 12

Die Tabelle 12 enthält die Verbindungen der Formel (Ig), in welcher A, B und E die in der Tabelle 11 genannten Bedeutungen haben und X und Y jeweils für Chlor stehen und Zₙ für Wasserstoff steht.

### Tabelle 13

Die Tabelle 13 enthält die Verbindungen der Formel (Ig), in welcher A, B und E die in der Tabelle 11 genannten Bedeutungen haben und X und Y jeweils für CH₃ stehen und Zₙ für Wasserstoff steht.

Verwendet man gemäß Verfahren (A) 2-(2,6-Dichlorphenyl)-4-(4-methoxy)-benzylmercapto-3-oxo-4,4-tetramethylen-buttersäure-ethylester, so kann der Verlauf des erfindungsgemäßen Verfahrens durch folgendes Reaktionsschema wiedergegeben werden:

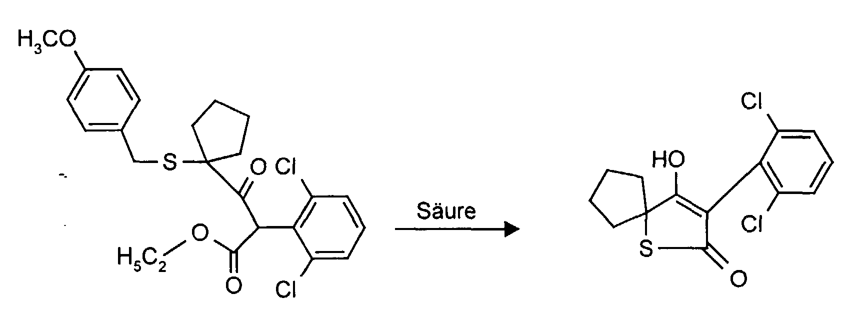

Verwendet man gemäß Verfahren (B) (Variante α) 3-(2,4,6-Trimethylphenyl)-4-hydroxy-5,5-tetramethylen-Δ³-dihydrothiophen-2-on und Pivaloylchlorid als Ausgangsstoff, so kann der Verlauf des erfindungsgemäßen Verfahrens durch folgendes Reaktionsschema wiedergegeben werden.

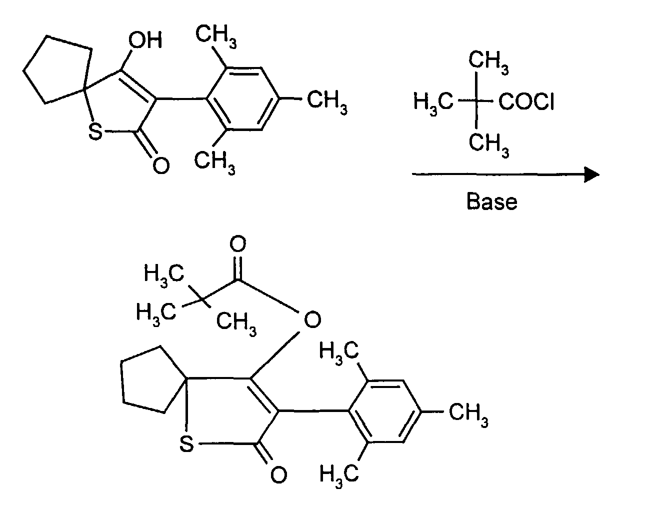

Verwendet man gemäß Verfahren C 3-(2,4-Dichlorphenyl)-4-hydroxy-5,5-tetramethylen-Δ³-dihydrothiophen-2-on und Chlorameisensäureethoxyethylester als Ausgangsverbindungen, so kann der Verlauf des erfindungsgemäßen Verfahrens durch folgendes Reaktionsschema wiedergegeben werden.

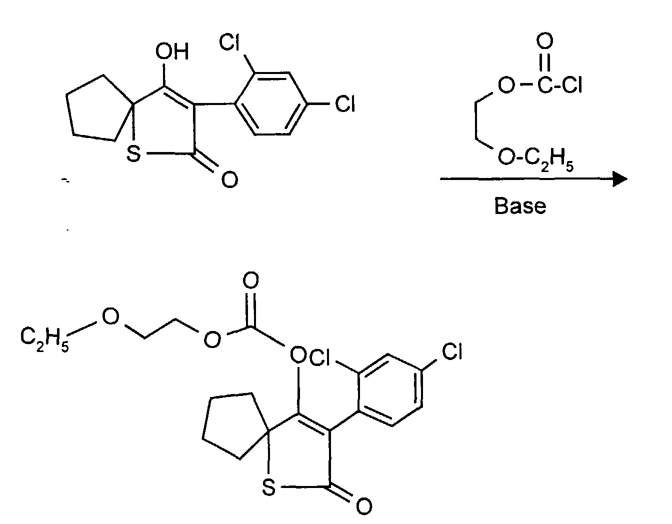

Verwendet man gemäß Verfahren (D_{α}) 3-(2,4,6-Trimethylphenyl)-4-hydroxy-5,5-tetramethylen-Δ³-dihydro-thiophen-2-on und Chlormonothioameisensäuremethylester als Ausgangsprodukte, so kann der Reaktionsverlauf wie folgt wiedergegeben werden:

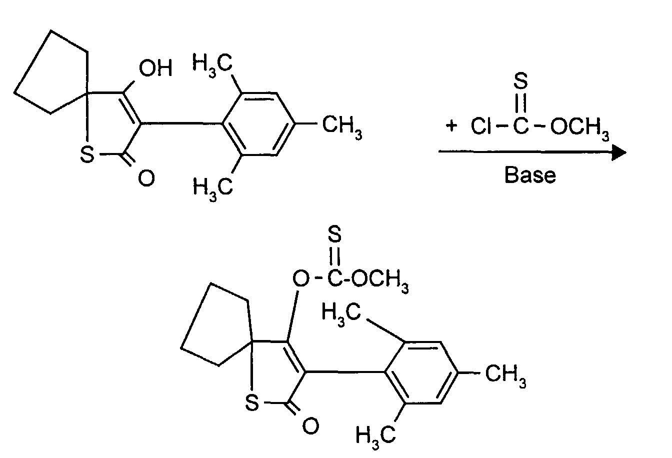

Verwendet man gemäß Verfahren (D_{β}) 3-(2,4,6-Trimethylphenyl)-4-hydroxy-5,5-pentamethylen-Δ³-dihydrothiophen-2-on, Schwefelkohlenstoff und Methyliodid als Ausgangskomponenten, so kann der Reaktionsverlauf wie folgt wiedergegeben werden:

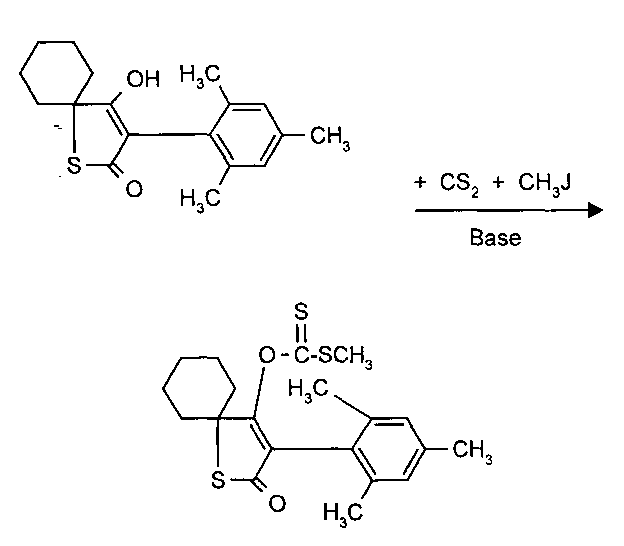

Verwendet man gemäß Verfahren (E) 3-(2,4,6-Trimethylphenyl)-4-hydroxy-5,5-(3-methoxy)-pentamethylen-Δ³-dihydrothiophen-2-on und Methansulfonsäurechlorid als Ausgangsprodukt, so kann der Reaktionsverlauf durch folgendes Reaktionsschema wiedergegeben werden:

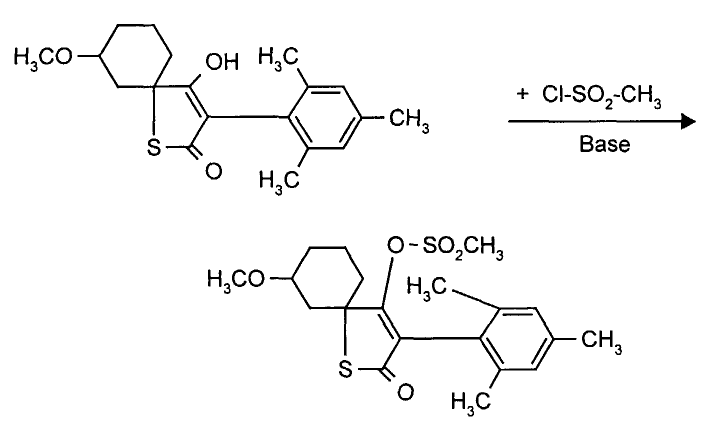

Verwendet man gemäß Verfahren (F) 3-(2,4,6-Trimethylphenyl)-4-hydroxy-5,5-tetramethylen-Δ³-dihydro-thiophen-2-on und Methanthio-phosphonsäurechlorid-(2,2,2-trifluorethylester) als Ausgangsprodukte, so kann der Reaktionsverlauf durch folgendes Reaktionsschema wiedergegeben werden:

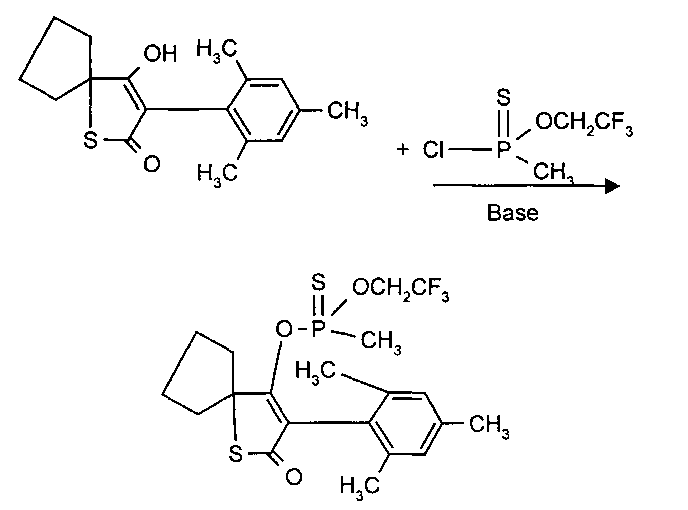

Verwendet man gemäß Verfahren (G_{α}) 3-(2,4,6-Trimethylphenyl)-4-hydroxy-5-tetramethylen-Δ³-dihydro-thiophen-2-on und Ethylisocyanat als Ausgangsprodukte, so kann der Reaktionsverlauf durch folgendes Reaktionsschema wiedergegeben werden:

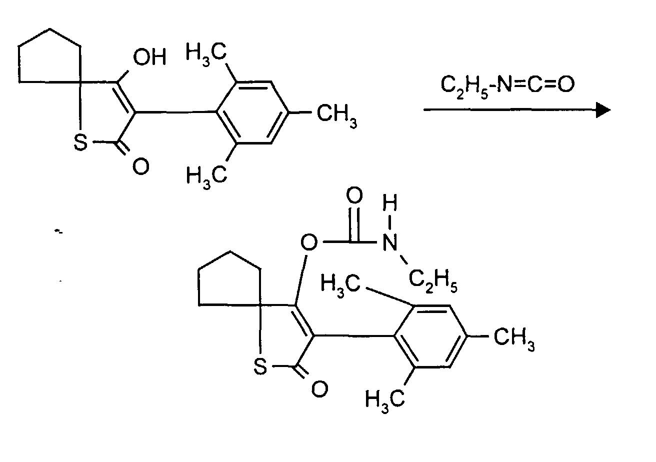

Verwendet man gemäß Verfahren (G_{β}) 3-(2,4,6-Trimethylphenyl)-4-hydroxy-5-trifluormethyl-5,5-tetramethylen-Δ³-dihydro-thiophen-2-on und Dimethylcarbamidsäurechlorid als Ausgangsprodukte, so kann der Reaktionsverlauf durch folgendes Schema wiedergegeben werden:

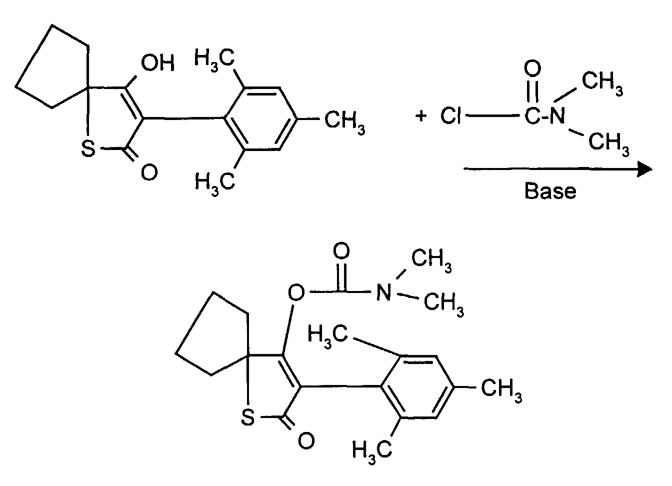

Verwendet man gemäß Verfahren (H) 3-(2,4,6-Trimethylphenyl)-4-hydroxy-5,5-tetramethylen-Δ³-dihydro-thiophen-2-on und NaOH als Komponenten, so kann der Verlauf des erfindungsgemäßen Verfahrens durch folgendes Reaktionsschema wiedergegeben werden:

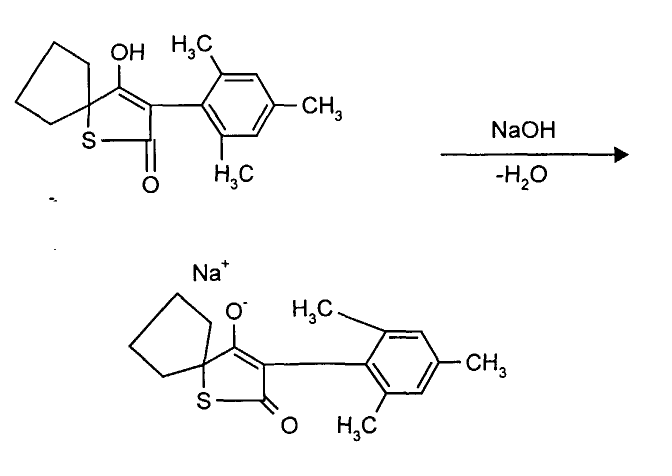

Die bei dem obigen Verfahren (A) als Ausgangsstoffe benötigten Verbindungen der Formel (II) 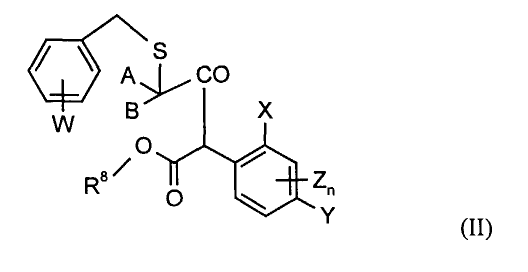 in welcher
A, B, W, X, Y, Z, n und R⁸ die oben angegebene Bedeutung haben, sind neu. Sie lassen sich nach im Prinzip bekannten Methoden herstellen. Man erhält z.B. 2-Aryl-4-S-benzyl-β-ketocarbonsäureester der Formel (II), wenn man Arylessigsäureester der Formel (XIV) 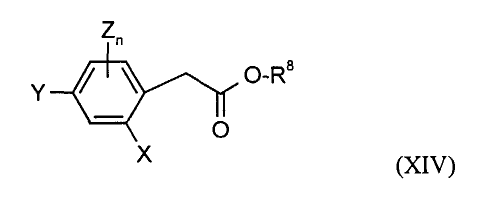 in welcher
X, Y, Z, R⁸ und n die oben angegebene Bedeutung haben,
mit 2-Benzylthio-carbonsäurehalogeniden der Formel (XV) 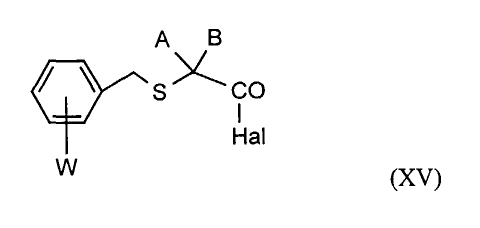 in welcher
- A, B und W: die oben angegebene Bedeutung haben und
- Hal: für Halogen, insbesondere Chlor oder Brom steht,
in Gegenwart von starken Basen acyliert (siehe z.B. M.S. Chambers, E.J. Thomas, D.J. Williams, J. Chem. Soc. Chem. Commun., (1987), 1228).

Die Verbindungen der Formel (XIV) sind literaturbekannt, leicht verfügbar, teilweise käufliche Verbindungen.

Die Benzylthio-carbonsäurehalogenide der Formel (XV) sind teilweise bekannt und/oder lassen sich nach bekannten Verfahren herstellen.

Die Benzylthio-carbonsäurehalogenide der Formel (XVa) 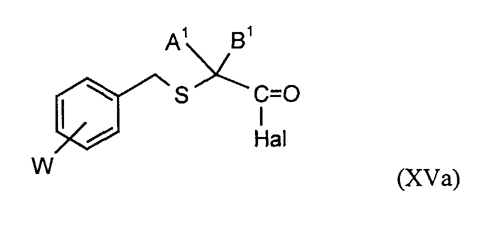 in welcher
- W: die oben angegebene Bedeutung hat
- Hal: für Halogen, insbesondere Chlor oder Brom steht und
- A¹ und B¹: gemeinsam mit dem Kohlenstoffatom, an das sie gebunden sind einen gesättigten oder ungesättigten, gegebenenfalls durch mindestens ein Heteroatom unterbrochenen und gegebenenfalls substituierten Cyclus bilden, oder worin
- A¹ und B¹: gemeinsam mit dem Kohlenstoffatom, an das sie gebunden sind für einen Cyclus stehen, bei dem Substituenten gemeinsam mit den Kohlenstoffatomen, an die sie gebunden sind, für einen gegebenenfalls einfach oder mehrfach, gleich oder verschieden durch Alkyl, Alkoxy oder Halogen substituierten gesättigten oder ungesättigten Cyclus stehen, der durch Sauerstoff oder Schwefel unterbrochen sein kann,
sind neu.

Man erhält die Verbindungen der Formel (XVa), wenn man Benzylthiocarbonsäuren der Formel (XVIa) 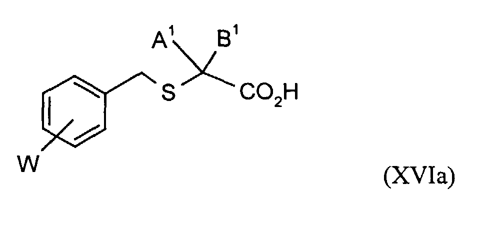 in welcher
- A¹, B¹ und W: die oben angegebenen Bedeutungen haben,
mit Halogenierungsmitteln, wie beispielsweise Phosgen, Phosphortri- oder -pentachlorid oder -bromid oder Thionylchlorid, gegebenenfalls in Gegenwart eines inerten Verdünnungsmittels, wie beispielsweise Kohlenwasserstoffen oder halogenierten Kohlenwasserstoffen bei Temperaturen zwischen -30°C und 150°C, bevorzugt zwischen -20°C und 100°C umsetzt (siehe z.B. J. Antibiotics (1983), 26, 1589).

Die Benzylthiocarbonsäuren der Formel (XVI) 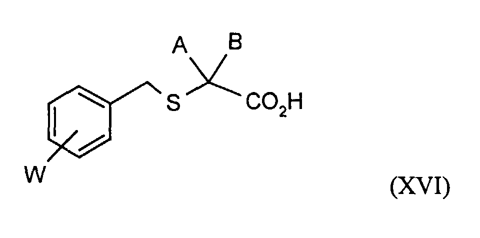 in welcher
- A, B und W: die oben angegebenen Bedeutungen haben,
sind zum Teil bekannt.

Man erhält die Benzylthiocarbonsäuren der Formel (XVI) beispielsweise, wenn man Carbonsäureester der Formel (XVII), 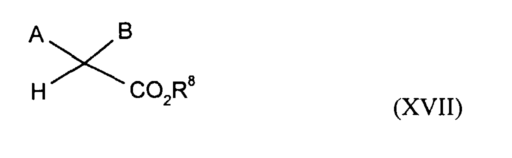 in welcher
- A, B und R⁸: die oben angegebenen Bedeutungen haben,
mit Disulfiden der Formel (XVIII) 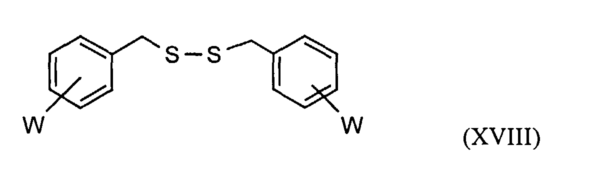 in welcher
- W: die oben angegebene Bedeutung hat,
gegebenenfalls in Gegenwart eines Verdünnungsmittels wie beispielsweise Tetrahydrofuran und/oder n-Hexan und in Gegenwart von Basen wie beispielsweise Natriumhydrid, Lithiumdiisopropylamid oder Kalium-tert.-butylat, umsetzt.

Dabei geht man im allgemeinen so vor, daß zunächst aus dem Ester der Formel (XVII) durch Reaktion mit der Base in einem geeigneten Verdünnungsmittel gegebenenfalls bei Temperaturen bis zu -80°C das entsprechende Anion gebildet wird. Dann setzt man das Disulfid der Formel (XVIII) zu und läßt die Reaktionsmischung bei Temperaturen von -20°C bis 50°C abreagieren (siehe z.B. J. Med. Chem. (1988), 31, 2199).

Die Verbindungen der Formeln (XVII) und (XVIII) sind allgemein bekannte Verbindungen der organischen Chemie.

Das Verfahren (A) ist dadurch gekennzeichnet, daß man Verbindungen der Formel (II) in welcher A, B, W, X, Y, Z, n und R⁸ die oben angegebene Bedeutung haben, in Gegenwart von Säuren intramolekular cyclisiert.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säuren können bei dem erfindungsgemäßen Verfahren (A) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die Säure z.B. in äquimolaren Mengen ein. Es ist jedoch gegebenenfalls auch möglich, die Säure als Lösungsmittel oder als Katalysator zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecan (DBU), Diazabicyclononen (DBN), Hünig-Base und N,N-Dimethylanilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im augemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Bei dem erfindungsgemäßen Verfahren (Bβ) können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calcium-oxid, außerdem Alkali- und Erdalkali-metall-carbonate, wie Natriumcarbonat, Kaliumcarbonat und Calciumcarbonat.

Als Verdünnungsmittel können bei dem erfindungsgemäßen Verfahren (C) alle gegenüber den Ausgangsverbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren D_{α} setzt man pro Mol Ausgangsverbindung der Formel (Ia) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren D_{β} setzt man pro Mol Ausgangsverbindung der Formel (II) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (II) durch Zusatz eines Deprotonierungsmittels (wie z.B. Kaliumtertiärbutylat oder Natriumhydrid) das entsprechende Salz herzustellen. Man setzt die Verbindung (II) solange mit Schwefelkohlenstoff um bis die Bildung der Zwischenverbindung abgeschlossen ist, z.B. nach mehrstündigem Rühren bei Raumtemperatur.

Die weitere Umsetzung mit dem Alkylhalogenid der Formel (VIII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren E) setzt man pro Mol Ausgangsverbindung der Formel (Ia) ca. 1 Mol Sulfonsäurechlorid (VIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren E) kann gegebenenfalls unter Phasen-Transfer-Bedingungen gearbeitet werden (W.J. Spillane et. al.; J. Chem. Soc., Perkin Trans I, (3) 677-9 (1982)). In diesem Fall setzt man pro Mol Ausgangsverbindung der Formel a) 0,3 bis 1,5 mol Sulfonsäurechlorid VIII, bevorzugt 0,5 mol bei 0° bis 150°C, vorzugsweise bei 20 bis 70°C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Beim Herstellungsverfahren F) setzt man zum Erhalt von Verbindungen der Formel (Ie) auf 1 Mol der Verbindung der Formel (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C.

Als gegebenenfalls zugesetzte Verdünnungsmittel kommen aller inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Dimethylsulfid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Beim Herstellungsverfahren G_{α}) setzt man pro Mol Ausgangsverbindung der Formel (Ia) ca. 1 Mol Isocyanat bzw. Isothiocyanat der Formel (X) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren G_{β}) setzt man pro Mol Ausgangsverbindung der Formel (Ia) ca. 1 Mol Carbamidsäurechlorid bzw. Thiocarbamidsäurechlorid der Formel (XI) bei 0 bis 150°C, vorzugsweise bei 20 bis 70°C um.

Als gegebenenfalls zugesetzte Verdünnungsmittel kommen aller inerten polaren organischen Lösungsmittel in Frage wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugswiese wird bei Normaldruck gearbeitet. Die Aufarbeitung geschieht nach üblichen Methoden.

Das Verfahren (H) ist dadurch gekennzeichnet, daß man Verbindungen der Formel (Ia) mit Metallverbindungen der Formel (XII) oder Aminen der Formel (XIII) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchgeführt. Die Reaktionstemperaturen liegen im allgemeinen zwischen -20°C und 100°C, vorzugsweise zwischen 0°C und 50°C.

Bei der Durchführung des erfindungsgemäßen Verfahrens (H) werden die Ausgangsstoffe der Formel (Ia) bzw. (XII) oder (XIII) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man zur Aufarbeitung das Reaktionsgemisch durch Abziehen des Verdünnungsmittel einengt.

Beispielhaft seien folgende Verbindungen der Formel (II) genannt:
2-(2,4-Dichlorphenyl)-4-(4-methoxybenzylmercapto)-3-oxo-4,4-tetramethylen-buttersäuremethylester
2-(2,4-Dichlorphenyl)-4-(4-methoxybenzylmercapto)-3-oxo-4,4-pentamethylen-buttersäuremethylester
2-(2,4-Dichlorphenyl)-4,4-hexamethylen-4-(4-methoxybenzylmercapto)-3-oxo-buttersäuremethylester
2-(2,4-Dichlorphenyl)-4,4-(2-methylpentamethylen)-4-(4-methoxybenzylmercapto)-3-oxo-buttersäuremethylester
2-(2,4-Dichlorphenyl)-4,4-(3-methylpentamethylen)-4-(4-methoxybenzylmercapto)-3-oxo-buttersäuremethylester
2-(2,4-Dichlorphenyl)-4,4-(3-ethylpentamethylen)-4-(4-methoxybenzylmercapto)-3-oxo-buttersäuremethylester
2-(2,4-Dichlorphenyl)-4,4-[3-(1-methyl)ethylpentamethylen]-4-(4-methoxybenzylmercapto)-3-oxo-buttersäuremethylester
2-(2,4-Dichlorphenyl)-4-(4-methoxybenzylmercapto)-4,4-(3-methoxypentamethylen)-3-oxo-buttersäuremethylester
2-(2,4-Dimethylphenyl)-4-(4-methoxybenzylmercapto)-3-oxo-4,4-tetramethylen-buttersäuremethylester
2-(2,4-Dimethylphenyl)-4-(4-methoxybenzylmercapto)-3-oxo-4,4-pentamethylen-buttersäuremethylester
2-(2,4-Dimethylphenyl)-4,4-hexamethylen-4-(4-methoxybenzylmercapto)-3-oxo-buttersäuremethylester
2-(2,4-Dimethylpheny)-4,4-(2-methylpentamethylen)-4-(4-methoxybenzylmercapto)-3-oxo-buttersäuremethylester
2-(2,4-Dimethylphenyl)-4,4-(3-methylpentamethylen)-4-(4-methoxybenzylmercapto)-3-oxo-buttersäuremethylester
2-(2,4-Dimethylphenyl)-4,4-(3-ethylpentamethylen)-4-(4-methoxybenzylmercapto)-3-oxo-buttersäuremethylester
2-(2,4-Dimethylphenyl)-4,4-[3-(1-methyl)ethylpentamethylen]-4-(4-methoxybenzylmercapto)-3-oxo-buttersäuremethylester
2-(2,4-Dimethylphenyl)-4-(4-methoxybenzylmercapto)-4,4-(3-methoxypentamethylen)-3-oxo-buttersäuremethylester
4-(4-Methoxybenzylmercapto)-3-oxo-4,4-tetramethylen-2-(2,4,6-trimethylphenyl)-buttersäuremethylester
4,4-Hexamethylen-4-(4-methoxybenzylmercapto)-3-oxo-2-(2,4,6-trimethylphenyl)-buttersäuremethylester
4-(4-Methoxybenzylmercapto)-3-oxo-4,4-pentamethylen-2-(2,4,6-trimethylphenyl)-buttersäuremethylester
4,4-(2-Methylpentamethylen)-4-(4-methoxybenzylmercapto)-3-oxo-2-(2,4,6-trimethylphenyl)-buttersäuremethylester
4,4-(3-Methylpentamethylen)-4-(4-methoxybenzylmercapto)-3-oxo-2-(2,4,6-trimethylphenyl)-buttersäuremethylester
4,4-(3-Ethylpentamethylen)-4-(4-methoxybenzylmercapto)-3-oxo-2-(2,4,6-trimethylphenyl)-buttersäuremethylester
4,4-[3-(1-Methyl)ethylpentamethylen]-4-(4-methoxybenzylmercapto)-3-oxo-2-(2,4,6-trimethylphenyl)-buttersäuremethylester
4-(4-Methoxybenzylmercapto)-4,4-(3-methoxypentamethylen)-3-oxo-2-(2,4,6-trimethylphenyl)-buttersäuremethylester.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Daraus pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Milben, wie beispielsweise gegen die gemeine Spinnmilbe oder die Bohnenspinnmilbe (Tetranychus urticae) oder gegen die Obstbaummilbe (Panonychus ulmi) einsetzen.

Die erfindungsgemäßen Wirkstoffe zeigen außerdem auch eine fungizide Wirksamkeit, beispielsweise gegen Pyricularia oryzae an Reis.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:
Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion,
Buprofezin, Chlorfluazuron, Diflubenzuron, Flucycloxuron, Flufenoxuron, Hexaflumuron, Pyriproxifen, Tebufenozide, Teflubenzuron, Triflumuron,
Imidacloprid, Nitenpyram, N-[(6-Chloro-3-pyridinyl)methyl]-N'-cyano-N-methylethanimidamid (NI-25),
Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril (AC 303630).

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Die Herstellung und die Verwendung der erfindungsgemäßen Stoffe werden durch die folgenden Beispiele veranschaulicht:

### Herstellungsbeispiele:

### Beispiel (Ia-1)

### 4-Hydroxy-1-thia-3-mesityl-spiro[4,5]dec-3-en-2-on

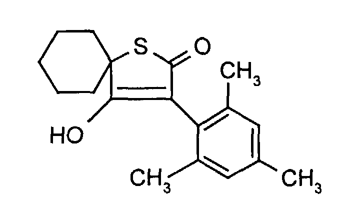

68 g 4-(4-Methoxybenzylmercapto)-3-oxo-4,4-pentamethylen-2-(2,4,6-trimethylphenyl)-buttersäure werden in 200 ml Trifluoressigsäure gelöst und unter Rückfluß eine Stunde gekocht. Dann wird das Lösungsmittel verdampft und der Rückstand (schwarzes Öl) in Ether (200 ml) und Wasser (1 l) aufgenommen. Man gibt dann NaOH bis pH 14 zu und dekantiert. Die wäßrige Phase wird mit konz. HCl angesäuert und mit Ether extrahiert (zweimal 200 ml). Die organisache Phase wird getrocknet (MgSO₄) und eingedampft. Der Rückstand kristallisiert im Hochvakuum. Die Kristalle werden mit wenig Cyclohexan gewaschen.
Weißer Feststoff, Fp.: 224°C
Ausbeute: 23,5 g (62 %)
¹H-NMR (100 MHz, CDCl₃): 1,25-2,00 (m, 8H); 2,15 (s, 6H); 2,20 (m, 2H); 2,30 (s, 3H); 6,95 (s, 2H).
¹³C NMR (CDCl₃): 19,6, 21,1, 24,7, 24,8, 35,9, 60,7, 114,5, 124,5, 128,7, 138,4, 138,8, 180,0, 193,5.

Analog zu Beispiel (Ia-1) und gemäß den allgemeinen Angaben zur Herstellung wurden folgende Verbindungen der Formel (Ia) hergestellt:

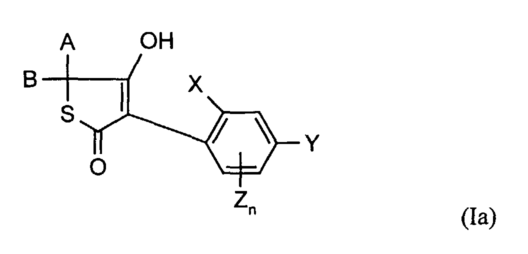

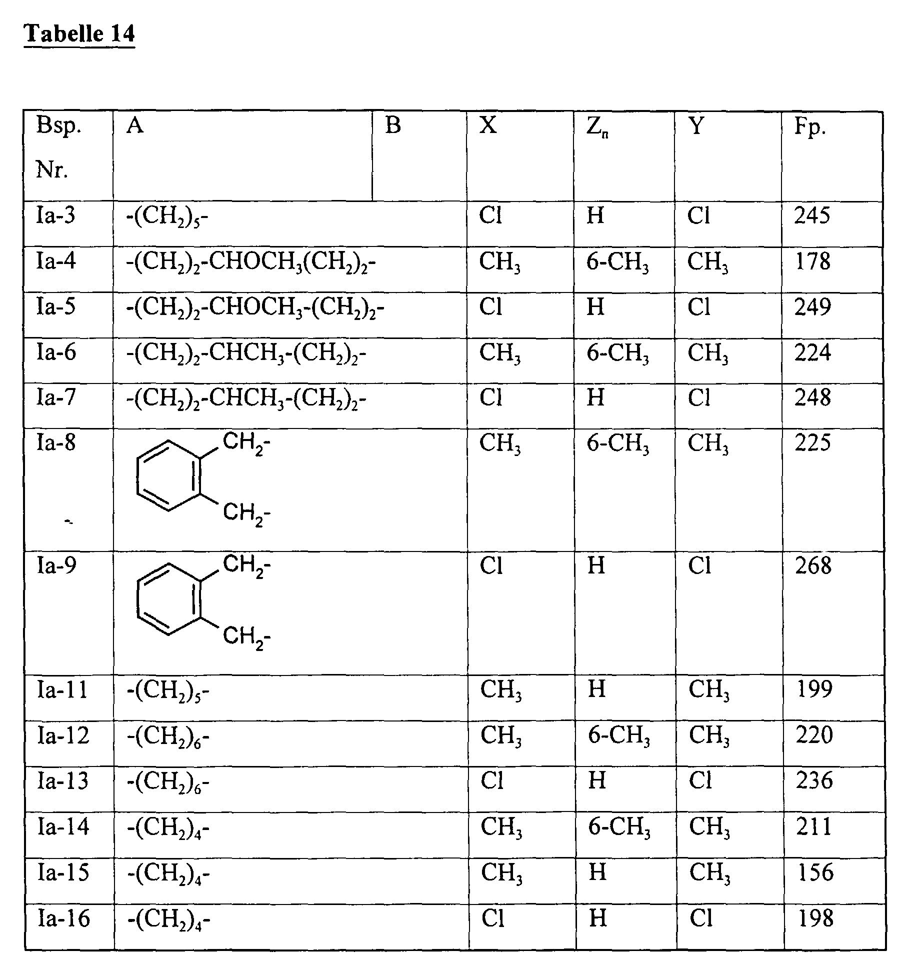

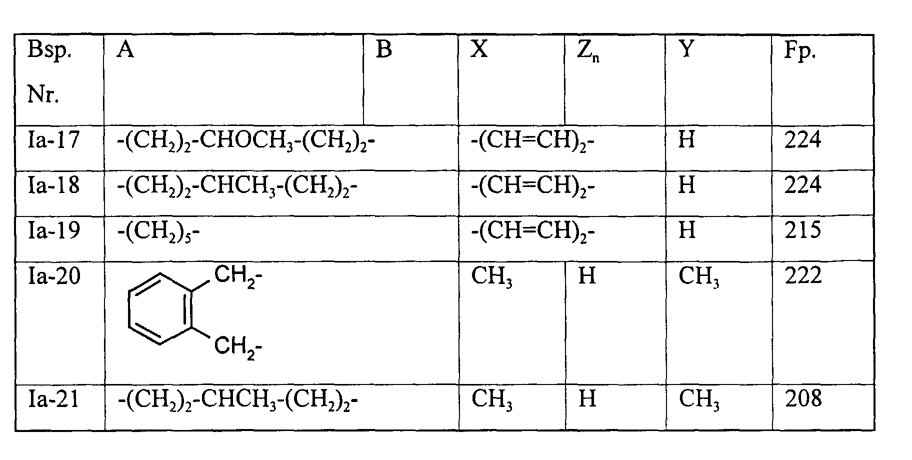

### Beispiel (Ib-1)

### 4-Acetoxy-1-thia-3-mesityl-spiro[4,5]dec-3-en-2-on

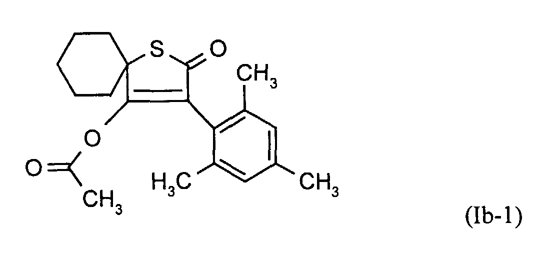

Eine Mischung aus der Verbindung gemäß Beispiel (Ia-1) (0,906 g), Triethylamin (1 ml) und Acetylchlorid (0,5 ml) in Toluol (10 ml) wird 2 Stunden unter Rückfluß gekocht und nach dem Abkühlen mit 100 ml Ethylether verdünnt. Die erhaltene Suspension wird über Kieselgel filtriert und eingeengt. Man erhält 848 mg (82 %) der oben gezeigten Verbindung vom Fp. = 150°C.

Analog zu Beispiel (Ib-1) und gemäß den allgemeinen Angaben zur Herstellung wurden folgende Verbindungen der Formel (Ib) hergestellt:

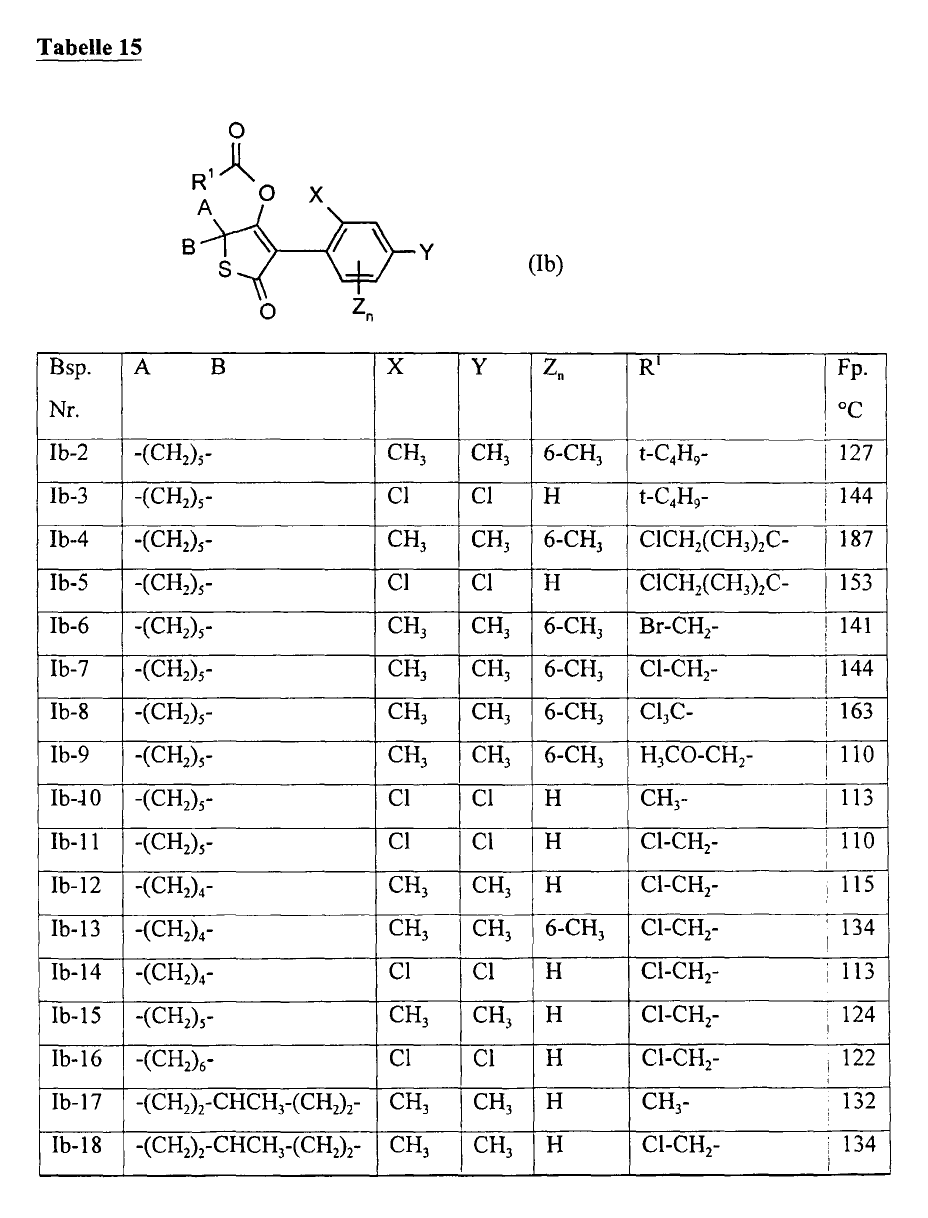

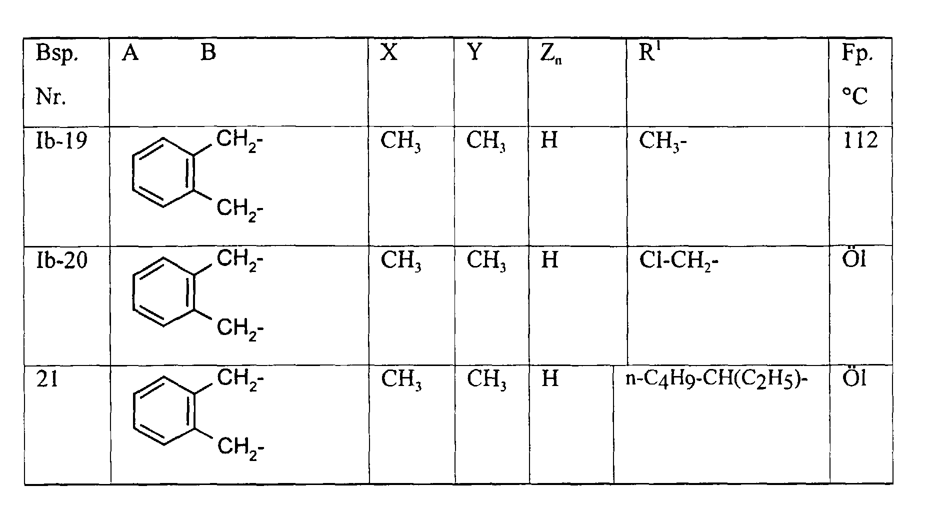

### Beispiel (Ic-1)

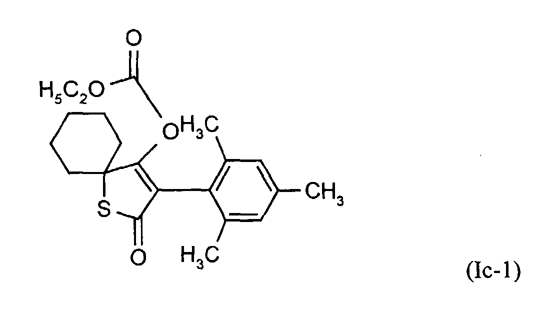

### 4-Ethoxycarbonyloxy-1-thia-3-mesityl-spiro[4,5]-dec-3-en-2-on.

Eine Mischung aus der Verbindung gemäß Beispiel (Ia-1) (0,906 g), Triethylamin (1 ml) und Chlorameisensäureethylester (0,6 ml) in Toluol (10 ml) wird 2 Stunden unter Rückfluß gekocht und nach dem Abkühlen mit 100 ml Ethylether verdünnt. Die erhaltene Suspension wird über Kieselgel filtriert und eingeengt. Man erhält 830 mg (74 %) der oben gezeigten Verbindung vom Fp. = 143°C.

Analog zu Beispiel (Ic-1) und gemäß den allgemeinen Angaben zur Herstellung wurden folgende Verbindungen der Formel (Ic) hergestellt:

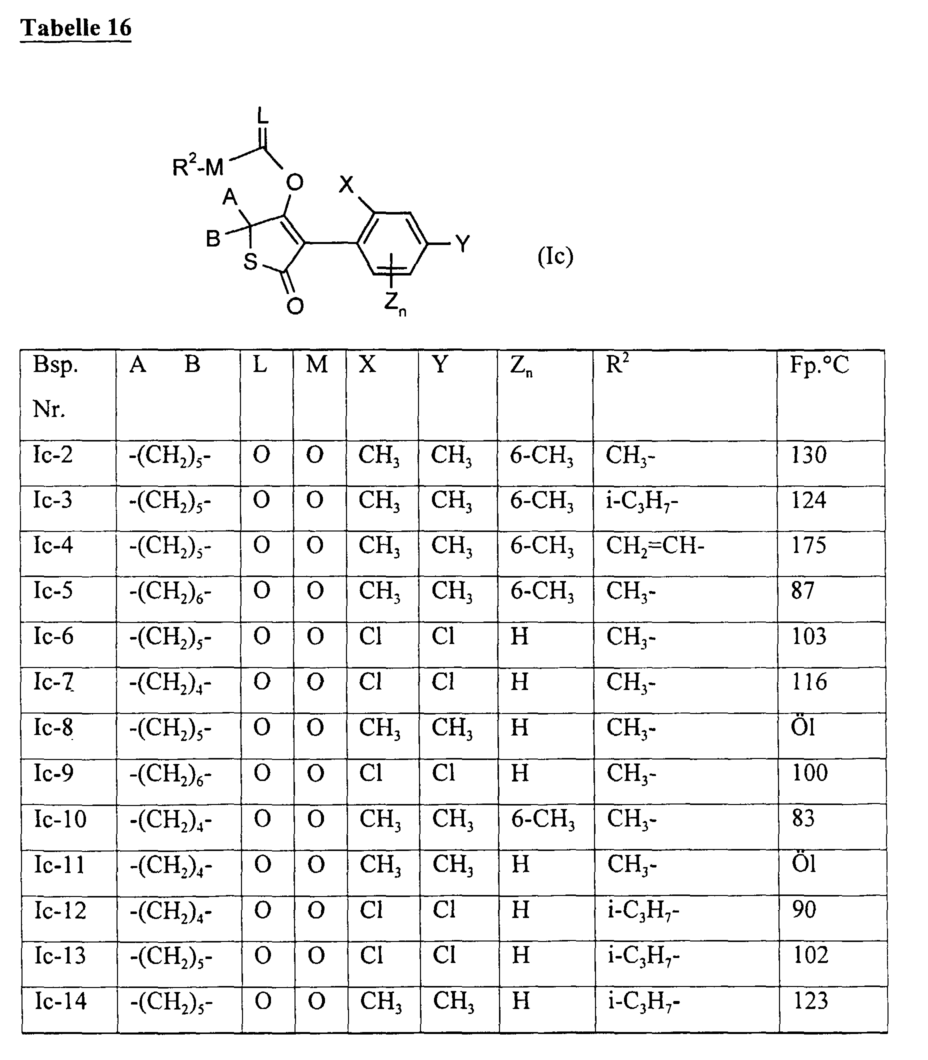

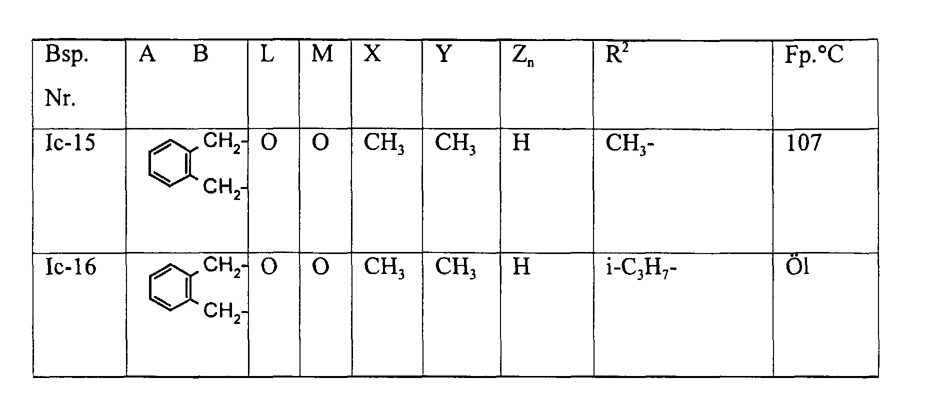

### Beispiel (Id-1)

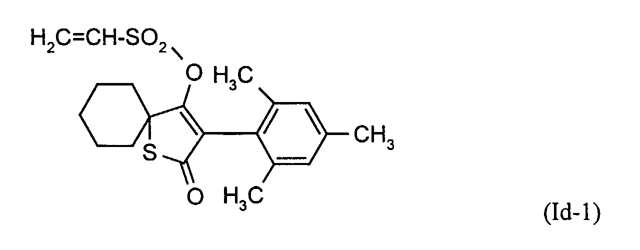

### 4-Vinylsulfonyloxy-1-thia-3-mesityl-spiro[4,5]-dec-3-en-2-on.

Eine Mischung aus der Verbindung gemäß Beispiel (Ia-1) (0,604 g), Triethylamin (1 ml) und 2-Chlorethylsulfonylchlorid (0,318 ml) in Toluol (10 ml) wird 2 Stunden unter Rückfluß gekocht, und nach dem Abkühlen mit 100 ml Ethylether verdünnt. Die erhaltene Suspension wird über Kieselgel filtriert und eingeengt. Man erhält 421 mg (54 %) der oben gezeigten Verbindung vom Fp. = 122°C.

### Herstellung der Ausgangsverbindungen

### Beispiel (XVI-1)

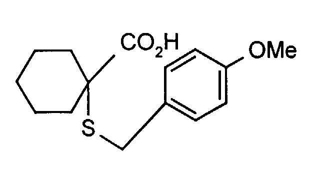

### 1-(4-Methoxybenzylmercapato)cyclohexan-1-carbonsäure:

Zu einer Lösung von Diisopropylamin (14,8 ml) in THF (80 ml) wird bei -78°C Butyllithium gegeben (1,6 M in Hexan, 60 ml). Nach wenigen Minuten wird Cyclohexancarbonsäuremethylester (11,4 g) zugetropft und die Lösung 1 Stunde bei -78°C gerührt. Dann wird Bis(4-methoxybenzyl)disulfid (24,3 g) zugegeben und die Temperatur auf 20°C erhöht. Die Mischung wird dann mit Ether (200 ml) verdünnt, mit Wasser (100 ml) gewaschen, getrocknet (MgSO₄) und dann eingeengt. Der feste Rückstand wird mit kaltem Petrolether gewaschen und in 200 ml Methanol gelöst. Man gibt 21 g Kaliumhydroxid zu. Die Mischung wird 2 Stunden unter Rückfluß erhitzt, dann eingeengt. Der feste Rückstand wird in Wasser aufgenommen und mit Ether gewaschen (zweimal 100 ml). Diese wäßrige Phase wird mit konzentriertem HCl angesäuert und mit Ether extrahiert (zweimal 300 ml). Die erhaltene organische Phase wird getrocknet (MgSO₄) und eingedampft. Der feste Rückstand wird mit Petroether gewaschen. Man erhält 14,5 g (86 %) der oben gezeugten Verbindung vom Fp. = 98°C.

Analog zu Beispiel XVI-1 und gemäß den allgemeinen Angaben zur Herstellung wurden folgende Verbindungen der Formel (XVI) hergestellt:

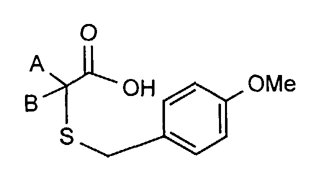

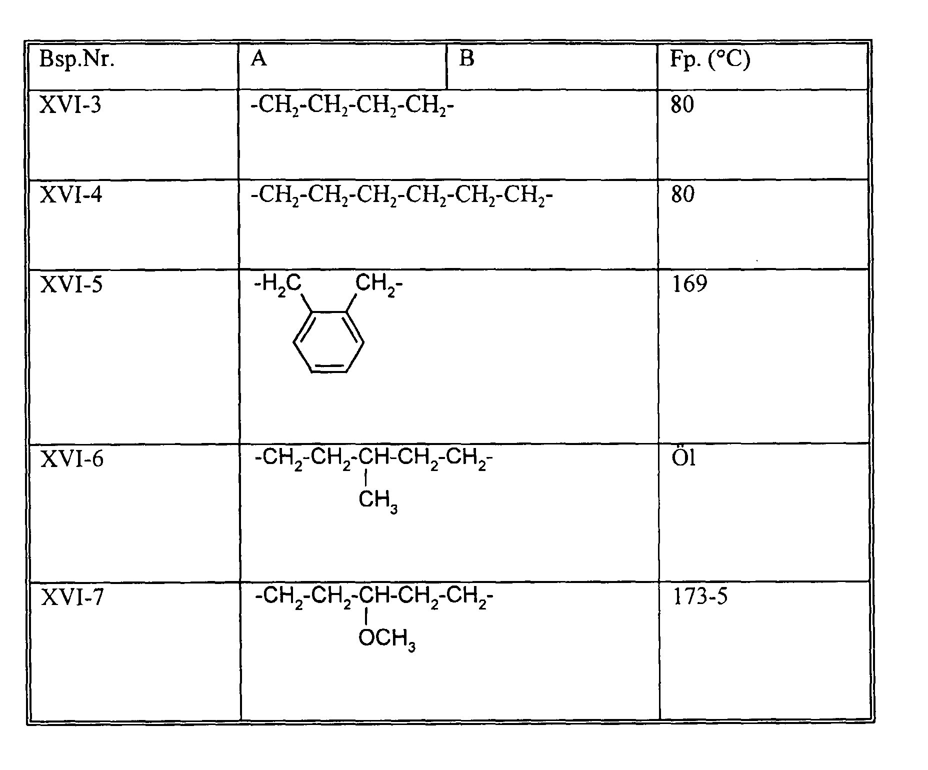

### Beispiel II-1

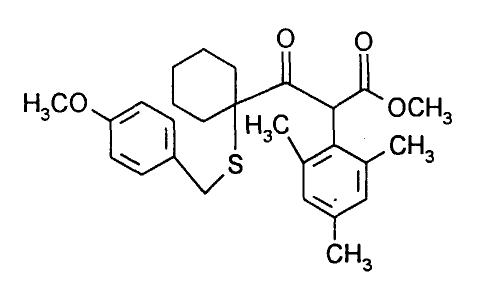

a) 1-(4-Methoxybenzylthio)-cyclohexancarbonsäurechlorid
   Zu einer Suspension von 11,2 g der Verbindung gemäß Beispiel (XVI-1) in 40 ml Toluol gibt man einen Tropfen DMF und 4,4 ml Thionylchlorid. Die Mischungwird nach wenigen Minuten auf 100°C erhitzt, bis die Gasentwicklung zu Ende ist (ca. 10 Minuten). Nach dem Eindampfen erhält man 12 g eines gelben Öls, das sofort weiter umgesetzt wird.
b) Zu einer Mischung von Diisopropylamin (14 ml) in THF (100 ml) gibt man bei 0°C Butyllithium (1,6 M in Hexan; 56 ml). Nach wenigen Minuten gibt man Mesitylessigsäuremethylester (15,4 g) zu und rührt 30 Minuten bei 0°C nach. Dann gibt man langsam 1-(4-Methoxybenzylthio)-cyclohexancarbonsäurechlorid aus a) zu und erwärmt auf Raumtemperatur. Nach einer Stunde verdünnt man das Reaktionsgemisch mit Ether (200 ml) und wäscht zweimal mit 10 %iger wäßriger Ammoniumchloridlösung, trocknet (MgSO₄) und dampft ein. Im Hochvakuum kristallisiert das Produkt langsam aus. Die Kristalle werden mit wenig Petrolether gewaschen. Fp. = 109°C
   Ausbeute 13,4 g (74 %)
   ¹H-NMR (100 MHz, CDCl₃): 1,20-1,80 (m, 8H); 1,90-2,00 (m, 2H); 2,30 (s, 3H); 2,40 (s, 6H); 3,20 (d, 1H, J = 12 Hz); 3,40 (d. 1H, J = 12 Hz); 3,80 (2s, 6H); 6,00 (s, 1H); 6,80 (d, 2H, J = 10 Hz); 6,90 (s, 2H); 7,05 (d, 2H, J = 10 Hz).

In Analogie zu Beispiel II-1 wurden die anderen Verbindungen der Formel (II) hergestellt, die sofort in Verfahren A eingesetzt und nicht analytisch charakterisiert wurden.

### Anwendungsbeispiele

### Beispiel A

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 (Gewichtsteil Wirkstoff mit der angegebenen Menge Lösungsmittel und der angegebenen Menge Emulgator und verdünnt das Konzentrat mit Wasser auf die gewünschte Konzentration

Kohlblätter (Brassica oleracea) werden durch Tauchen in die Wirkstoffzubereitung der gewünschten Konzentration behandelt und mit Meerrettichblattkäfer-Larven (Phaedon cochleariae) besitzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen (Ia-6), (Ia-7), (Ia-9), (Ia-11), (Ib-9) und (Ib-10) bei einer beispielhaften Wirkstoffkonzentration von 0,1 % eine Abtötung von 100 % nach 7 Tagen.

### Beispiel B

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen (Ia-6), (Ia-7), (Ia-9), (Ia-11), (Ib-3), (Ib-9) und (Ib-10) bei einer beispielhaften Wirkstoffkonzentration von 0,1 % eine Abtötung von 100 % nach 7 Tagen.

### Beispiel C

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 (Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen (Ia-6) und (Ia-11) bei einer beispielhaften Wirkstoffkonzentration von 0,1 % eine Abtötung von 100 % nach 6 Tagen.

### Beispiel D

### Spodoptera-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen (Ia-9), (Ia-11), (Ib-9) und (Ib-10) bei einer beispielhaften Wirkstoffkonzentration von 0,1 % eine Abtötung von mindestens 95 % nach 7 Tagen.

### Beispiel E

### Panonychus-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 (Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Ca. 30 cm hohe Pflaumenbäumchen (Prunus domestica), die stark von allen Stadien der Obstbaumspinnmilbe Panonychus ulmi befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test bewirkte z.B. die Verbindung gemäß dem Herstellungsbeispiel (Ib-7) bei einer beispielhaften Wirkstoffkonzentration von 0,02 % eine Abtötung von 100 % nach 14 Tagen.

### Beispiel F

### Tetranychus-Test (OP-resistent/Spritzbehandlung)

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 1OO %, daß alle Spinumilben abgetötet wurden; O % bedeutet, daß keine Spinnmilben abgetötet wurden.

Die Verbindungen gemäß den Herstellungsbeispielen (Ia-1), (Ia-3), (Ic-2) und (Ic-3) bewirkten bei einer beispielhaften Wirkstoffkonzentration von 0,02 % eine Abtötung von mindestens 95 % nach 7 Tagen.

### Beispiel G

### Blowfly-Larven-Test / Entwicklungshemmende Wirkung

- Testtiere:: Lucilia cuprina-Larven
- Lösungsmittel:: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 cm³ Pferdefleisch und 0,5 ml der zu testenden Wirkstoffzubereitung enthält.

Die Teströhrchen werden in Becher mit Sand-bedecktem Boden überführt. Nach 2 Tagen werden die Teströhrchen entfernt und die Puppen gezählt. Die Wirkung der Wirkstoffzubereitung wird nach der Zahl der geschlüpften Fliegen nach 1,5-facher Entwicklungsdauer einer unbehandelten Kontrolle beurteilt. Dabei bedeutet 100 %, daß keine Fliegen geschlüpft sind, 0 %, daß alle Fliegen normal geschlüpft sind.

In diesem Test hatte z.B. die Verbindung gemäß Herstellungsbeispiel (Ia-1) bei einer beispielhaften Wirkstoffkonzentration von 1000 ppm eine Wirkung von 100 %.

### Beispiel H

### Test mit Boophilus microplus resistent / SP resistenter Parkhurst-Stamm

- Testtiere:: Adulte gesogene Weibchen
- Lösungsmittel:: Dimethylsulfoxid

20 mg Wirkstoff werden in 1 ml Dimethylsulfoxid gelöst, geringere Konzentrationen werden durch Verdünnen mit dem gleichen Lösungsmittel hergestellt.

Der Test wird in 5-fach Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

In diesem Test haften z.B. die Verbindungen gemäß den Herstellungsbeispielen (Ia-1), (Ib-7) und (Ic-3) bei einer beispielhaften Wirkstoffkonzentration von 20 µg/Tier jeweils eine Wirkung von 100 %.

## Patentansprüche

1. 3-Aryl-4-hydroxy-Δ³-dihydrothiophenon-Derivate der Formel (I) 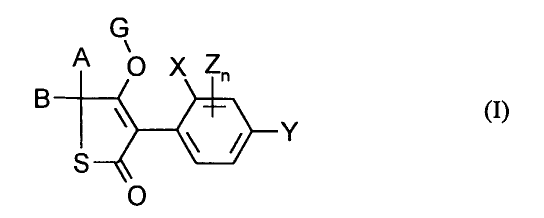 in welcher
X für C₁-C₆-Alkyl, Halogen, C₁-C₆-Alkoxy oder C₁-C₃-Halogenalkyl steht,
Y für Wasserstoff, C₁-C₆-Alkyl, Halogen, C₁-C₆-Alkoxy oder C₁-C₃-Halogenalkyl steht,
Z für C₁-C₆-Alkyl, Halogen oder C₁-C₆-Alkoxy steht,
n für eine Zahl von 0 bis 3 steht
oder die Reste X und Z gemeinsam mit dem Phenylrest an den sie gebunden sind, für den Naphthalinrest der Formel stehen, 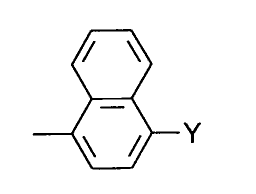 in welchem
Y die oben angegebene Bedeutung hat,
A und B gemeinsam mit dem Kohlenstoffatom, an das sie gebunden sind, für einen gesättigten oder ungesättigten, gegebenenfalls durch Sauerstoff und/oder Schwefel unterbrochenen und gegebenenfalls einfach oder mehrfach gleich oder verschieden durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls einfach oder mehrfach, gleich oder verschieden durch Halogen, C₁-C₆-Alkyl oder C₁-C₆-Alkoxy substituiertes Phenyl substituierten 3- bis 8-gliedrigen Ring stehen oder
A und B gemeinsam mit dem Kohlenstoffatom, an das sie gebunden sind, für einen C₃-C₈-gliedrigen Ring stehen, bei dem zwei Substituenten gemeinsam mit den Kohlenstoffatomen, an die sie gebunden sind, für einen gegebenenfalls einfach oder mehrfach gleich oder verschieden durch C₁-C₆-Alkyl, C₁-C₆-Alkoxy oder Halogen substituierten gesättigten oder einfach ungesättigten C₅-C₇-Ring stehen, der durch Sauerstoff oder Schwefel unterbrochen sein kann,
G für Wasserstoff (a) oder für eine der Gruppen 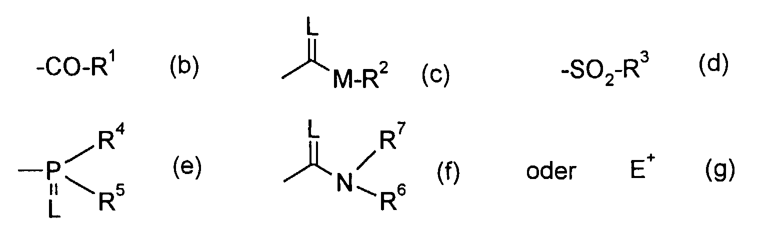 steht, in welchen
E⁺ für ein Metallionäquivalent oder ein Ammoniumion steht,
L für Sauerstoff oder Schwefel steht und
M für Sauerstoff oder Schwefel steht,
R¹ für jeweils gegebenenfalls einfach oder mehrfach, gleich oder verschieden durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, C₁-C₈-Polyalkoxy-C₂-C₈-alkyl oder gegebenenfalls durch Halogen oder C₁-C₆-Alkyl substituiertes Cycloalkyl mit 3 bis 8 Ringatomen, das durch mindestens ein Sauerstoff- und/oder Schwefelatom unterbrochen sein kann,
für gegebenenfalls einfach oder mehrfach, gleich oder verschieden durch Halogen, Nitro, C₁-C₆-Alkyl, C₁-C₆-Alkoxy, C₁-C₆-Halogenalkyl oder C₁-C₆-Halogenalkoxy substituiertes Phenyl,
für gegebenenfalls einfach oder mehrfach, gleich oder verschieden durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls einfach oder mehrfach, gleich oder verschieden durch Halogen oder C₁-C₆-Alkyl substituiertes 5- oder 6-gliedriges Hetaryl,
für gegebenenfalls einfach oder mehrfach, gleich oder verschieden durch Halogen oder C₁-C₆-Alkyl substituiertes Phenoxy-C₁-C₆-alkyl oder
für gegebenenfalls einfach oder mehrfach, gleich oder verschieden durch Halogen, Amino oder C₁-C₆-Alkyl substituiertes 5- oder 6-gliedriges Hetaryloxy-C₁-C₆-alkyl steht,
R² für jeweils gegebenenfalls einfach oder mehrfach, gleich oder verschieden durch Halogen substituiertes C₁-C₂₀-Alkyl, C₂-C₂₀-Alkenyl, C₁-C₈-Alkoxy-C₂-C₈-alkyl oder C₁-C₈-Polyalkoxy-C₂-C₈-alkyl oder für gegebenenfalls durch Halogen, C₁-C₄-Alkyl oder C₁-C₄-Alkoxy substituiertes C₃-C₈-Cycloalkyl oder
für jeweils gegebenenfalls einfach oder mehrfach, gleich oder verschieden durch Halogen, Nitro, C₁-C₆-Alkyl, C₁-C₆-Alkoxy oder C₁-C₆-Halogenalkyl substituiertes Phenyl oder Benzyl steht,
R³, R⁴ und R⁵ unabhängig voneinander für jeweils gegebenenfalls einfach oder mehrfach, gleich oder verschieden durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alkinylthio, C₃-C₇-Cycloalkylthio oder für jeweils gegebenenfalls einfach oder mehrfach, gleich oder verschieden durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Benzyl, Phenoxy oder Phenylthio stehen und
R⁶ und R⁷ unabhängig voneinander für Wasserstoff, jeweils gegebenenfalls einfach oder mehrfach, gleich oder verschieden durch Halogen substituiertes C₁-C₂₀-Alkyl, C₁-C₂₀-Alkoxy, C₂-C₈-Alkenyl oder C₁-C₂₀-Alkoxy-C₁-C₂₀-alkyl, für jeweils gegebenenfalls einfach oder mehrfach, gleich oder verschieden durch Halogen, C₁-C₂₀-Halogenalkyl, C₁-C₂₀-Alkyl oder C₁-C₂₀-Alkoxy substituiertes Phenyl oder Benzyl oder zusammen für einen gegebenenfalls durch Sauerstoff unterbrochenen C₂-C₆-Alkylenrest stehen.

2. Verbindungen der Formel (I) gemäß Anspruch 1, in welcher
X für C₁-C₆-Alkyl, Halogen, C₁-C₆-Alkoxy oder C₁-C₂-Halogenalkyl steht,
Y für Wasserstoff C₁-C₆-Alkyl, Halogen, C₁-C₆-Alkoxy oder C₁-C₂-Halogenalkyl steht,
Z für C₁-C₄-Alkyl, Halogen oder C₁-C₄-Alkoxy steht,
n für eine Zahl von 0 bis 2 steht
oder die Reste X und Z gemeinsam mit dem Phenylrest an den sie gebunden sind, für den Naphthalinrest der Formel 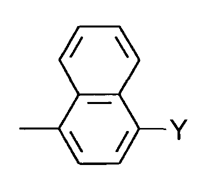 stehen, in welchem
Y die oben angegebene Bedeutung hat,
A und B gemeinsam mit dem Kohlenstoffatom, an das sie gebunden sind, für einen gesättigten oder ungesättigten, gegebenenfalls durch Sauerstoff und/oder Schwefel unterbrochen und gegebenenfalls einfach oder mehrfach, gleich oder verschieden durch Fluor, Chlor, C₁-C₅-Alkyl, C₁-C₅-Alkoxy, C₁-C₃-Halogenalkyl, C₁-C₄-Halogenalkoxy, C₁-C₃-Alkylthio oder gegebenenfalls einfach oder mehrfach, gleich oder verschieden durch Fluor, Chlor, Brom, C₁-C₄-Alkyl oder C₁-C₄-Alkoxy substituiertes Phenyl substituierten 3- bis 8-gliedrigen Ring stehen, oder
A und B gemeinsam mit dem Kohlenstoffatom, an das sie gebunden sind, für einen C₄-C₇-gliedrigen Ring stehen, bei dem zwei Substituenten gemeinsam mit den Kohlenstoffatomen, an die sie gebunden sind, für einen gegebenenfalls einfach oder mehrfach, gleich oder verschieden durch C₁-C₃-Alkyl, C₁-C₃-Alkoxy, Fluor oder Chlor substituierten gesättigten oder ungesättigten C₅-C₆-Ring stehen, der durch Sauerstoff oder Schwefel unterbrochen sein kann,
G für Wasserstoff (a) oder für eine der Gruppen 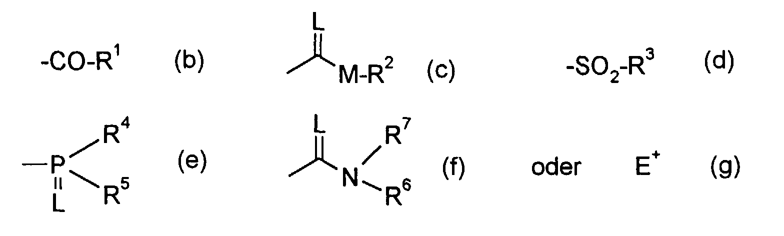 steht, in welchen
E⁺ für ein Metallionäquivalent oder ein Ammoniumion steht,
L für Sauerstoff oder Schwefel steht,
M für Sauerstoff oder Schwefel steht,
R¹ für jeweils gegebenenfalls einfach oder mehrfach, gleich oder verschieden durch Halogen substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl, C₁-C₆-Polyalkoxy-C₂-C₆-alkyl oder gegebenenfalls durch Fluor, Chlor, C₁-C₄-Alkyl substituiertes Cycloalkyl mit 3 bis 7 Ringatomen, das durch 1 bis 2 Sauerstoff- und/oder Schwefelatome unterbrochen sein kann,
für gegebenenfalls einfach bis fünffach gleich oder verschieden durch Halogen, Nitro, C₁-C₄-Alkyl, C₁-C₄-Alkoxy, C₁-C₃-Halogenalkyl oder C₁-C₃-Halogenalkoxy substituiertes Phenyl,
für gegebenenfalls einfach bis fünffach, gleich oder verschieden durch Halogen, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für gegebenenfalls einfach bis vierfach, gleich oder verschieden durch Halogen oder C₁-C₆-Alkyl substituiertes Pyridyl, Thienyl, Furanyl, Pyrimidyl, Thiazolyl oder Pyrazolyl,
für gegebenenfalls einfach bis fünffach, gleich oder verschieden durch Halogen oder C₁-C₄-Alkyl substituiertes Phenoxy-C₁-C₅-alkyl,
für gegebenenfalls einfach bis vierfach, gleich oder verschieden durch Halogen, Amino oder C₁-C₄-Alkyl substituiertes Pyridyloxy-, Pyrimidyloxy-, Thiazolyloxy-C₁-C₅-alkyl steht,
R² für jeweils gegebenenfalls einfach oder mehrfach, gleich oder verschieden durch Halogen substituiertes C₁-C₁₆-Alkyl, C₂-C₁₆-Alkenyl, C₁-C₆-Alkoxy-C₂-C₆-alkyl oder C₁-C₆-Polyalkoxy-C₂-C₆-alkyl oder für gegebenenfalls durch Fluor, Chlor, C₁-C₃-Alkyl, C₁-C₃-Alkoxy substituiertes C₃-C₇-Cycloalkyl oder
für jeweils gegebenenfalls einfach bis fünffach, gleich oder verschieden durch Halogen, Nitro, C₁-C₄-Alkyl, C₁-C₃-Alkoxy oder C₁-C₃-Halogenalkyl substituiertes Phenyl oder Benzyl steht,
R³, R⁴ und R⁵ unabhängig voneinander für jeweils gegebenenfalls einfach oder mehrfach, gleich oder verschieden durch Halogen substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄-Alkenylthio, C₂-C₄-Alkinylthio, C₃-C₆-Cycloalkylthio oder für jeweils gegebenenfalls einfach bis fünffach, gleich oder verschieden durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Benzyl, Phenoxy oder Phenylthio stehen und
R⁶ und R⁷ unabhängig voneinander für Wasserstoff, jeweils gegebenenfalls einfach oder mehrfach, gleich oder verschieden durch Halogen substituiertes C₁-C₂₀-Alkyl, C₁-C₂₀-Alkoxy, C₂-C₈-Alkenyl oder C₁-C₂₀-Alkoxy-C₁-C₂₀-alkyl, für gegebenenfalls einfach bis fünffach gleich oder verschieden durch Halogen, C₁-C₅-Halogenalkyl, C₁-C₅-Alkyl oder C₁-C₅-Alkoxy substituiertes Phenyl oder Benzyl oder zusammen für einen gegebenenfalls durch Sauerstoff oder Schwefel unterbrochenen C₄-C₆-Alkylenrest stehen.

3. Verbindungen der Formel (I) gemäß Anspruch 1, in welcher
X für Methyl, Ethyl, Propyl, i-Propyl, Fluor, Chlor, Brom, Methoxy, Ethoxy oder Trifluormethyl steht,
Y für Wasserstoff Methyl, Ethyl, Propyl, i-Propyl, Butyl, i-Butyl, tert.-Butyl, Fluor, Chlor, Brom, Methoxy, Ethoxy oder Trifluormethyl steht,
Z für Methyl, Ethyl, i-Propyl, Butyl, i-Butyl, tert-Butyl, Fluor, Chlor, Brom, Methoxy oder Ethoxy steht,
n für eine Zahl 0 oder 1 steht,
oder die Reste X und Z gemeinsam mit dem Phenylrest an den sie gebunden sind für den Rest der Formel 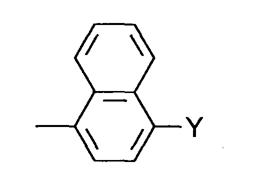 stehen, in welchem Y die oben angegebene Bedeutung hat,
A und B gemeinsam mit dem Kohlenstoffatom, an das sie gebunden sind für einen gesättigten oder ungesättigten, gegebenenfalls durch Sauerstoff und/oder Schwefel unterbrochenen und gegebenenfalls einfach bis fünffach, gleich oder verschieden durch Fluor, Chlor, C₁-C₄-Alkyl, C₁-C₄-Alkoxy, Trifluormethyl, C₁-C₂-Alkylthio oder gegebenenfalls einfach bis dreifach, gleich oder verschieden durch Fluor, Chlor, Methyl oder Methoxy substituiertes Phenyl substituierten 3- bis 8-gliedrigen Ring stehen oder
A und B gemeinsam mit dem Kohlenstoffatom, an das sie gebunden sind, für einen C₃-C₆-gliedrigen Ring stehen, bei dem zwei Substituenten gemeinsam mit den Kohlenstoffatomen, an die sie gebunden sind, für einen gegebenenfalls einfach bis fünffach, gleich oder verschieden durch Methyl, Ethyl, Methoxy, Ethoxy, Fluor oder Chlor substituierten gesättigten oder ungesättigten C₅-C₆-Ring stehen, der durch Sauerstoff oder Schwefel unterbrochen sein kann,
G für Wasserstoff (a) oder für eine der Gruppen 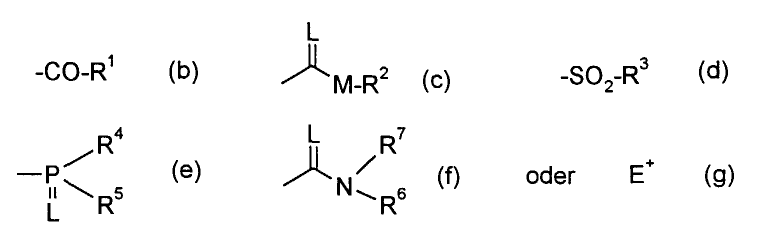 steht, in welchen
E⁺ für ein Metallionäquivalent oder ein Ammoniumion steht,
L für Sauerstoff oder Schwefel steht,
M für Sauerstoff oder Schwefel steht,
R¹ für jeweils gegebenenfalls einfach bis neunfach, gleich oder verschieden durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₂-C₄-alkyl oder gegebenenfalls durch Fluor, Chlor, Methyl oder Ethyl substituiertes Cycloalkyl mit 3 bis 6 Ringatomen, das durch 1-2 Sauerstoff- und/oder Schwefelatome unterbrochen sein kann,
für gegebenenfalls einfach bis dreifach, gleich oder verschieden durch Fluor, Chlor, Brom, Methyl, Ethyl, Propyl, i-Propyl, Methoxy, Ethoxy, Trifluormethyl, Trifluormethoxy oder Nitro substituiertes Phenyl,
für gegebenenfalls einfach bis dreifach, gleich oder verschieden durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl,
für jeweils gegebenenfalls einfach bis dreifach, gleich oder verschieden durch Fluor, Chlor, Brom, Methyl oder Ethyl substituiertes Pyridyl, Thienyl oder Furanyl,
für gegebenenfalls einfach bis dreifach, gleich oder verschieden durch Fluor, Chlor, Methyl, Ethyl substituiertes Phenoxy-C₁-C₄-alkyl oder
für jeweils gegebenenfalls einfach bis dreifach, gleich oder verschieden durch Fluor, Chlor, Amino, Methyl oder Ethyl substituiertes Pyridyloxy-C₁-C₄-alkyl, Pyrimidyloxy-C₁-C₄-alkyl und Thiazolyloxy-C₁-C₄-alkyl steht,
R² für jeweils gegebenenfalls einfach bis neunfach, gleich oder verschieden durch Fluor oder Chlor substituiertes C₁-C₁₄-Alkyl, C₂-C₁₄-Alkenyl, C₁-C₄-Alkoxy-C₂-C₆-alkyl oder C₁-C₄-Polyalkoxy-C₂-C₆-alkyl, für gegebenenfalls durch Fluor, Chlor, Methyl, Ethyl, Methoxy oder Ethoxy substituiertes C₃-C₆-Cycloalkyl
oder für jeweils gegebenenfalls einfach bis dreifach, gleich oder verschieden durch Fluor, Chlor, Nitro, Methyl, Ethyl, Propyl, i-Propyl, Methoxy, Ethoxy oder Trifluormethyl substituiertes Phenyl oder Benzyl steht,
R³, R⁴ und R⁵ unabhängig voneinander für jeweils gegebenenfalls einfach bis fünffach, gleich oder verschieden durch Fluor oder Chlor substituiertes C₁-C₄-Alkyl, C₁-C₄-Alkoxy, C₁-C₄-Alkylamino, Di-(C₁-C₄-Alkyl)-amino, C₁-C₄-Alkylthio, für jeweils gegebenenfalls einfach bis dreifach, gleich oder verschieden durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio oder C₁-C₃-Alkyl substituiertes Phenyl, Benzyl, Phenoxy oder Phenylthio stehen und
R⁶ und R⁷ unabhängig voneinander für Wasserstoff, jeweils gegebenenfalls einfach bis neunfach, gleich oder verschieden durch Fluor, Chlor oder Brom substituiertes C₁-C₁₀-Alkyl, C₁-C₁₀-Alkoxy oder C₁-C₁₀-Alkoxy-(C₁-C₁₀)alkyl, für gegebenenfalls einfach bis dreifach, gleich oder verschieden durch Fluor, Chlor, Brom, C₁-C₂-Halogenalkyl, C₁-C₂-Alkyl oder C₁-C₄-Alkoxy substituiertes Phenyl, für gegebenenfalls einfach bis dreifach, gleich oder verschieden durch Fluor, Chlor, Brom, C₁-C₄-Alkyl, C₁-C₄-Halogenalkyl oder C₁-C₄-Alkoxy substituiertes Benzyl oder zusammen für einen gegebenenfalls durch Sauerstoff oder Schwefel unterbrochenen C₄-C₆-Alkylenrest stehen.

4. Verfahren zur Herstellung von Verbindungen der Formel (I) gemäß Anspruch 1, dadurch gekennzeichnet,
A)
daß man zum Erhalt von 3-Aryl-4-hydroxy-Δ³-dihydrothiophenon-Derivaten der Formel (Ia) 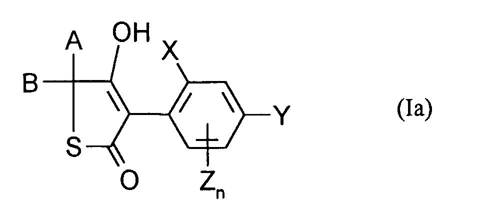 in welcher
A, B, X, Y, Z und n die in Anspruch 1 angegebene Bedeutung haben,
β-Ketocarbonsäureester der Formel (II) 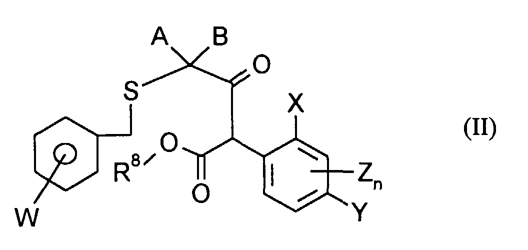 in welcher
A, B, X, Y, Z und n die oben angegebene Bedeutung haben,
W für Wasserstoff, Halogen, C₁-C₈-Alkyl oder C₁-C₈-Alkoxy,
und
R⁸ für C₁-C₈-Alkyl steht,
gegebenenfalls in Gegenwart eines Verdünnungsmittels und in Gegenwart einer Säure intramolekular cyclisiert oder
(B)
daß man zum Erhalt von Verbindungen der Formel (Ib) 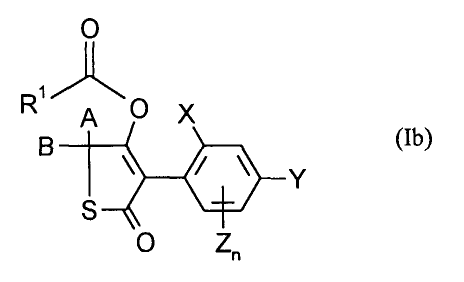 in welcher
A, B, X, Y, Z, R¹ und n die in Anspruch 1 angegebene Bedeutung haben,
Verbindungen der Formel (Ia) 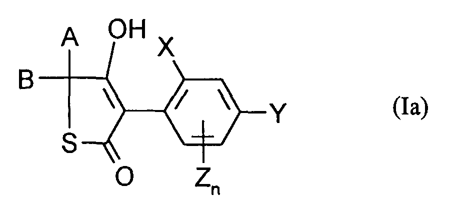 in welcher
A, B, X, Y, Z und n die oben angegebene Bedeutung haben,
α) mit Säurehalogeniden der Formel (III) 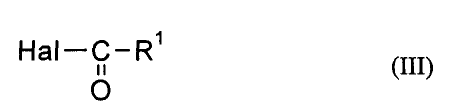 in welcher
R¹ die oben angegebene Bedeutung hat
und
Hal für Halogen steht,
gegebenenfalls in Gegenwart eines Verdünnungsmittels und gegebenenfalls in Gegenwart eines Säurebindemittels umsetzt
oder
β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt oder
(C)
daß man zum Erhalt von Verbindungen der Formel (Ic) 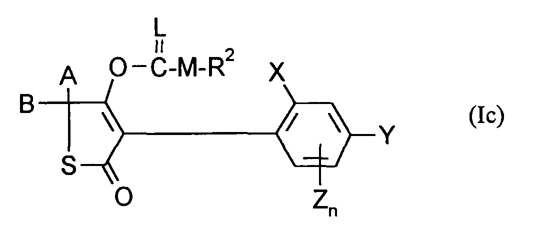 in welcher
A, B, X, Y, Z, R² und n die in Anspruch 1 angegebene Bedeutung haben,
L für Sauerstoff und
M für Sauerstoff oder Schwefel steht,
Verbindungen der Formel (Ia) 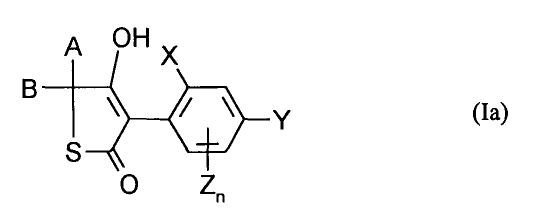 in welcher
A, B, X, Y, Z und n die oben angegebene Bedeutung haben,
mit Chlorameisensäureester oder Chlorameisensäurethiolester der Formel (V)
R²-M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt, oder
(D)
daß man zum Erhalt von Verbindungen der Formel (Ic) 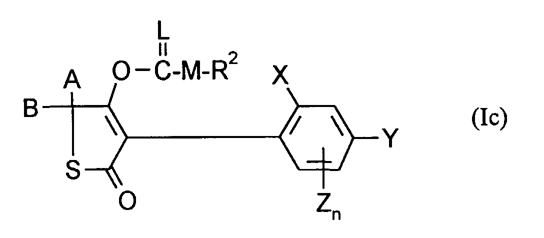 in welcher
A, B, R², X, Y, Z und n die oben angegebene Bedeutung haben,
L für Schwefel und
M für Sauerstoff oder Schwefel steht,
Verbindungen der Formel (Ia) 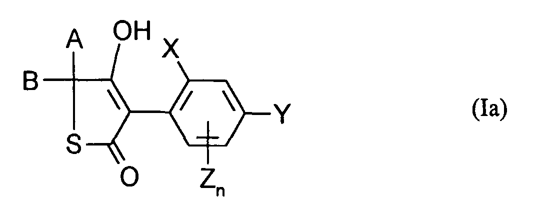 in welcher
A, B, X, Y, Z und n die oben angegebene Bedeutung haben,
α) mit Chlormonothioameisensäureestern oder Chlordithioameisensäureestern der Formel (VI) 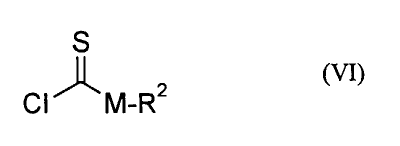 in welcher
M und R² die oben angegebene Bedeutung haben
gegebenenfalls in Gegenwart eines Verdünnungsmittels und gegebenenfalls in Gegenwart eines Säurebindemittels umsetzt, oder
β) mit Schwefelkohlenstoff und anschließend mit Alkylhalogeniden der Formel (VII)
R²-Hal (VII)
in welcher
R² die oben angegebene Bedeutung hat
und
Hal für Chlor, Brom oder Iod steht, umsetzt oder
(E)
daß man zum Erhalt von Verbindungen der Formel (Id) 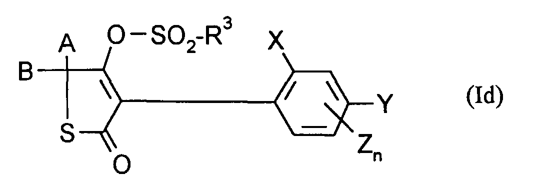 in welcher
A, B, X, Y, Z, R³ und n die in Anspruch 1 angegebene Bedeutung haben,
Verbindungen der Formel (Ia) 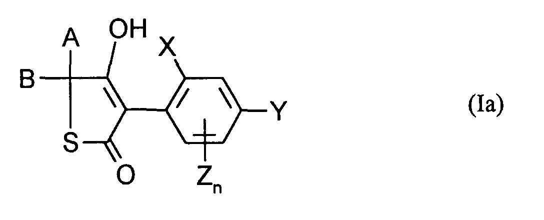 in welcher
A, B, X, Y, Z und n die oben angegebene Bedeutung haben,
mit Sulfonsäurechloriden der Formel (VIII)
R³-SO₂-Cl (VIII)
in welcher
R³ die oben angegebene Bedeutung hat
gegebenenfalls in Gegenwart eines Verdünnungsmittels und gegebenenfalls in Gegenwart eines Säurebindemittels,
umsetzt, und
(F)
daß man zum Erhalt von Verbindungen der Formel (Ie) 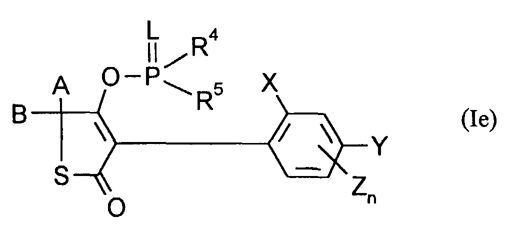 in welcher
A, B, L, X, Y, Z, R⁴, R⁵ und n die in Anspruch 1 angegebene Bedeutung haben,
Verbindungen der Formel (Ia) 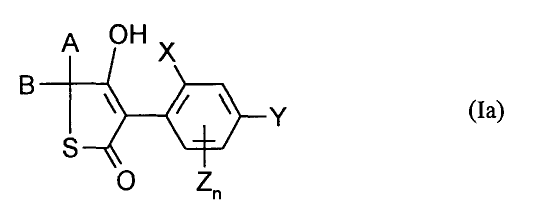 in welcher
A, B, X, Y, Z und n die oben angegebene Bedeutung haben
mit Phosphorverbindungen der Formel (IX) 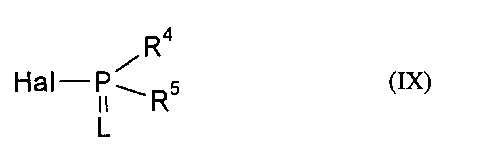 in welcher
L, R⁴ und R⁵ die oben angegebene Bedeutung haben und
Hal für Halogen steht,
gegebenenfalls in Gegenwart eines Verdünnungsmittels und gegebenenfalls in Gegenwart eines Säurebindemittels umsetzt, oder
(G)
daß man zum Erhalt von Verbindungen der Formel (If) 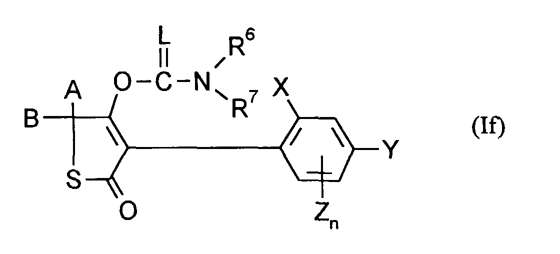 in welcher
A, B, L, X, Y, Z, R⁶, R⁷ und n die in Anspruch 1 angegebene Bedeutung haben,
Verbindungen der Formel (Ia) 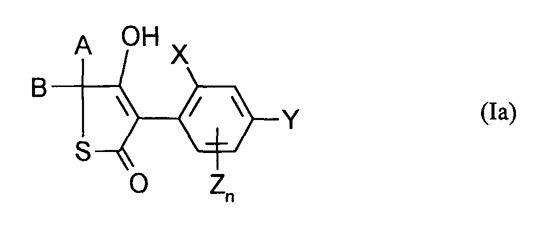 in welcher
A, B, X, Y, Z und n die oben angegebene Bedeutung haben
α) mit Isocyanaten oder Isothiocyanaten der Formel (X)
R⁶-N=C=L (X)
in welcher
R⁶ und L die oben angegebene Bedeutung hat
gegebenenfalls in Gegenwart eines Verdünnungsmittels und gegebenenfalls in Gegenwart eines Katalysators umsetzt,
oder
β) mit Carbamidsäurechloriden oder Thiocarbamidsäurechloriden der Formel (XI) 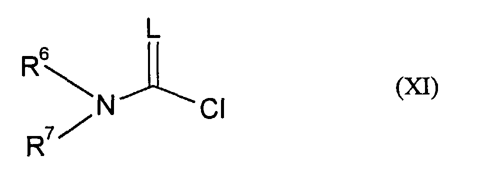 in welcher
L, R⁶ und R⁷ die oben angegebene Bedeutung haben,
gegebenenfalls in Gegenwart eines Verdünnungsmittels und gegebenenfalls in Gegenwart eines Säurebindemittels,
umsetzt, oder
(H)
daß man zum Erhalt von Verbindungen der Formel (Ig) 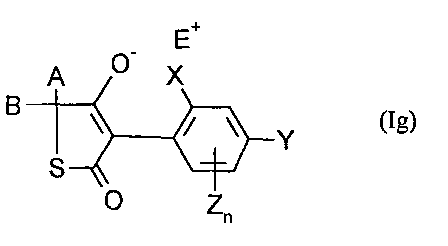 in welcher
X, Y, Z, A, B und n die oben angegebene Bedeutung haben,
und E⁺ für ein Metallionäquivalent oder für ein Ammoniumion steht,
Verbindungen der Formel (Ia) 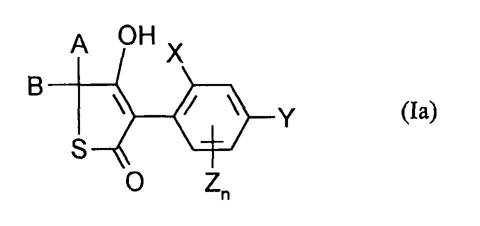 in welcher
X, Y, Z, A, B und n die oben angegebene Bedeutung haben,
mit Metallhydroxiden, Metallalkoxiden oder Aminen der Formeln (XII) und (XIII)
MeOR⁹ₜ (XII) 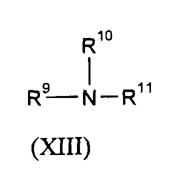
in welchen
Me für ein- oder zweiwertige Metallionen und
t für die Zahl 1 oder 2 steht und
R⁹, R¹⁰ und R¹¹ unabhängig voneinander für Wasserstoff oder Alkyl stehen,
gegebenenfalls in Gegenwart eines Verdünnungsmittels, umsetzt.

5. Verbindungen der Formel (II) 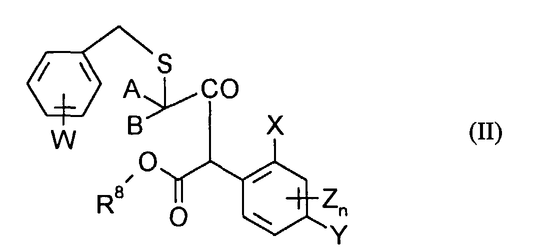 in welcher
A, B, X, Y, Z und n die in Anspruch 1 angegebene Bedeutung haben, R⁸ für C₁-C₈-Alkyl steht und W für Wasserstoff, Halogen, C₁-C₈-Alkyl oder C₁-C₈-Alkoxy steht.

6. Verfahren zur Herstellung von Verbindungen der Formel (II) gemäß Anspruch 5, dadurch gekennzeichnet, daß man Arylessigsäureester der Formel (XIV) 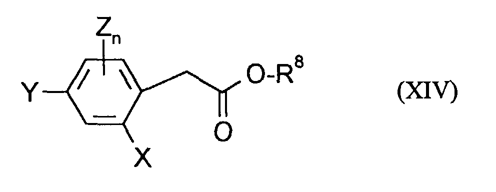 in welcher
X, Y, Z, R⁸ und n die in Anspruch 5 angegebene Bedeutung haben,
mit 2-Benzylthio-carbonsäurehalogeniden der Formel (XV) 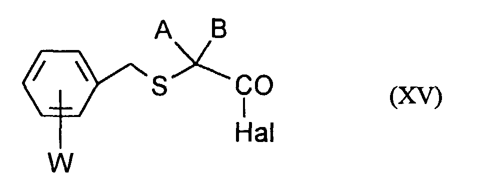 in welcher
A, B und W die in Anspruch 5 angegebene Bedeutung haben und
Hal für Halogen steht,
in Gegenwart von starken Basen acyliert.

7. Benzylthio-carbonsäurehalogenide der Formel (XVa) 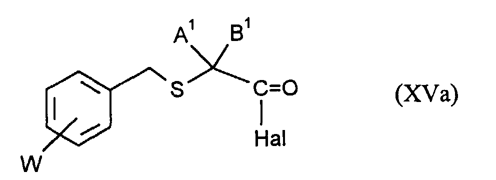 in welcher
W die in Anspruch 5 angegebene Bedeutung hat
Hal für Halogen steht und
A¹ und B¹ gemeinsam mit dem Kohlenstoffatom, an das sie gebunden sind einen gesättigten oder ungesättigten, gegebenenfalls durch mindestens ein Heteroatom unterbrochenen und gegebenenfalls substituierten Cyclus bilden, oder worin
A¹ und B¹ gemeinsam mit dem Kohlenstoffatom, an das sie gebunden sind für einen Cyclus stehen, bei dem Substituenten gemeinsam mit den Kohlenstoffatomen, an die sie gebunden sind, für einen gegebenenfalls einfach oder mehrfach, gleich oder verschieden durch Alkyl, Alkoxy oder Halogen substituierten gesättigten oder ungesättigten Cyclus stehen, der durch Sauerstoff oder Schwefel unterbrochen sein kann.

8. Verfahren zur Herstellung von Verbindungen der Formel (XVa) gemäß Anspruch 7, dadurch gekennzeichnet, daß man Benzylthiocarbonsäuren der Formel (XVIa) 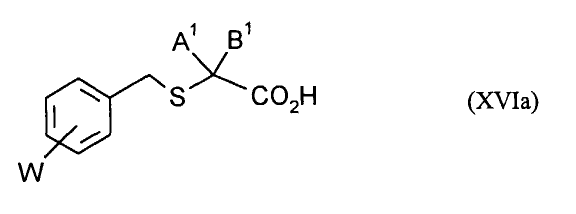 in welcher
A¹, B¹ und W die in Anspruch 7 angegebenen Bedeutungen haben,
mit Halogenierungsmitteln bei Temperaturen zwischen -30°C und 150°C, bevorzugt zwischen -20°C und 100°C umsetzt.

9. Schädlingsbekämpfungsmittel, gekennzeichnet durch einen Gehalt an mindestens einer Verbindung der Formel (I) gemäß Anspruch 1.

10. Verwendung von Verbindungen der Formel (I) gemäß Anspruch 1 zur Bekämpfung von tierischen Schädlingen.

11. Verfahren zur Bekämpfung von tierischen Schädlingen, dadurch gekennzeichnet, daß man Verbindungen der Formel (I) gemäß Anspruch 1 auf die Schädlinge und/oder deren Lebensraum ausbringt.

12. Verfahren zur Herstellung von Mitteln zur Bekämpfung tierischer Schädlinge, dadurch gekennzeichnet, daß man Verbindungen der Formel (I) gemäß Anspruch 1 mit Streckmitteln und/oder oberflächenaktiven Stoffen vermischt.

## Claims

1. 3-Aryl-4-hydroxy-Δ³-dihydrothiophenone derivatives of the formula (I) 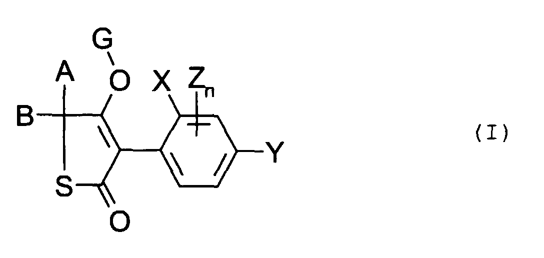 in which
X represents C₁-C₆-alkyl, halogen, C₁-C₆-alkoxy or C₁-C₃-halogenoalkyl,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number from 0 to 3
or the radicals X and Z represent, together with the phenyl radical to which they are bonded, the naphthalene radical of the formula 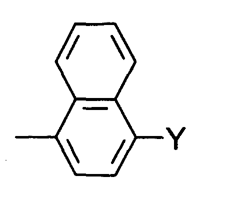 in which
Y has the abovementioned meaning.,
A and B represent, together with the carbon atom to which they are bonded, a saturated or unsaturated 3- to 8-membered ring which is optionally interrupted by oxygen and/or sulphur and optionally substituted identically or differently once or more than once by halogen, C₁-C₆-alkyl, C₁-C₆-alkoxy, C₁-C₄-halogenoalkyl, C₁-C₄-halogenoalkoxy, C₁-C₄-alkylthio or phenyl which is optionally substituted identically or differently once or more than once by halogen, C₁-C₆-alkyl or C₁-C₆-alkoxy, or
A and B represent, together with the carbon atom to which they are bonded, a C₃-C₈-membered ring in which two substituents, together with the carbon atoms to which they are bonded, represent a saturated or mono-unsaturated C₅-C₇ ring which is optionally substituted identically or differently once or more than once by C₁-C₆-alkyl, C₁-C₆-alkoxy or halogen and which can be interrupted by oxygen or sulphur,
G represents hydrogen (a) or represents one of the groups 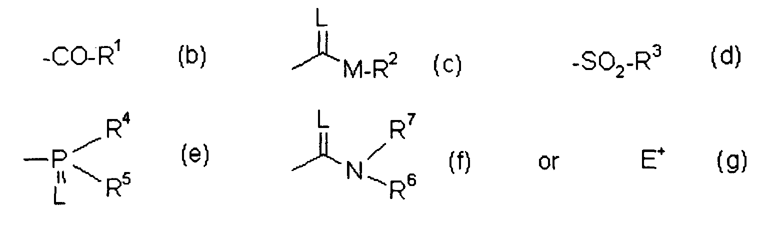 in which
E⁺ represents a metal ion equivalent or an ammoium ion,
L represents oxygen or sulphur, and
M represents oxygen or sulphur,
R¹ represents C₁-C₂₀-alkyl, C₂-C₂₀-alkenyl, C₁-C₈-alkoxy-C₁-C₈-alkyl, C₁-C₈-alkylthio-C₁-C₈-alkyl or C₁-C₈-polyalkoxy-C₂-C₈-alkyl which are in each case optionally substituted identically or differently once or more than once by halogen, or cycloalkyl having 3 to 8 ring atoms which is optionally substituted by halogen or C₁-C₆-alkyl and can be interrupted by at least one oxygen and/or sulphur atom,
represents phenyl which is optionally substituted identically or differently once or more than once by halogen, nitro, C₁-C₆-alkyl, C₁-C₆-alkoxy, C₁-C₆-halogenoalkyl or C₁-C₆-halogenoalkoxy,
represents phenyl-C₁-C₆-alkyl which is optionally substituted identically or differently once or more than once by halogen, C₁-C₆-alkyl, C₁-C₆-alkoxy, C₁-C₆-halogenoalkyl or C₁-C₆-halogenoalkoxy,
represents 5- or 6-membered hetaryl which is optionally substituted identically or differently once or more than once by halogen or C₁-C₆-alkyl,
represents phenoxy-C₁-C₆-alkyl which is optionally substituted identically or differently once or more than once by halogen or C₁-C₆-alkyl, or
represents 5- or 6-membered hetaryloxy-C₁-C₆-alkyl which is optionally substituted identically or differently once or more than once by halogen, amino or C₁-C₆-alkyl,
R² represents C₁-C₂₀-alkyl, C₂-C₂₀-alkenyl, C₁-C₈-alkoxy-C₂-C₈-alkyl or C₁-C₈-polyalkoxy-C₂-C₈-alkyl which are in each case optionally substituted identically or differently once or more than once by halogen, or represents C₃-C₈-cycloalkyl which is optionally substituted by halogen, C₁-C₄-alkyl or C ₁-C₄-alkoxy, or represents phenyl or benzyl which are in each case optionally substituted identically or differently once or more than once by halogen, nitro, C₁-C₆-alkyl, C₁-C₆-alkoxy or C₁-C₆-halogenoalkyl,
R³, R⁴ and R⁵ represent, independently of each other, C₁-C₈-alkyl, C₁-C₈-alkoxy, C₁-C₈-alkylamino, di-(C₁-C₈)-alkylamino, C₁-C₈-alkylthio, C₂-C₅-alkenylthio, C₂-C₅-alkinylthio or C₃-C₇-cycloalkylthio which are in each case optionally substituted identically or differently once or more than once by halogen, or represent phenyl, benzyl, phenoxy or phenylthio which are in each case optionally substituted identically or differently once or more than once by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl, and
R⁶ and R⁷ represent, independently of each other, hydrogen, or C₁-C₂₀-alkyl, C₁-C₂₀-alkoxy, C₂-C₈-alkenyl or C₁-C₂₀-alkoxy-C₁-C₂₀-alkyl which are in each case optionally substituted identically or differently once or more than once by halogen, represent phenyl or benzyl which are in each case optionally substituted identically or differently once or more than once by halogen, C₁-C₂₀-halogenoalkyl, C₁-C₂₀-alkyl or C₁-C₂₀-alkoxy, or together represent a C₂-C₆-alkylene radical which is optionally interrupted by oxygen.

2. Compounds of the formula (I) according to Claim 1, in which
X represents C₁-C₆-alkyl, halogen, C₁-C₆-alkoxy or C₁-C₂-halogenoalkyl,
Y represents hydrogen, C₁-C₆-alkyl, halogen, C₁-C₆-alkoxy or C₁-C₂-halogenoalkyl,
Z represents C₁-C₄-alkyl, halogen or C₁-C₄-alkoxy,
n represents a number from 0 to 2
or the radicals X and Z represent, together with the phenyl radical to which they are bonded, the naphthalene radical of the formula 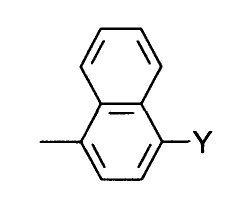 in which
Y has the abovementioned meaning.
A and B represent, together with the carbon atom to which they are bonded, a saturated or unsaturated 3- to 8-membered ring which is optionally interrupted by oxygen and/or sulphur and which is optionally substituted identically or differently once or more than once by fluorine, chlorine, C₁-C₅-alkyl, C₁-C₅-alkoxy, C₁-C₃-halogenoalkyl, C₁-C₄-halogenoalkoxy, C₁-C₃-alkylthio or phenyl which is optionally substituted identically or differently once or more than once by fluorine, chlorine, bromine, C₁-C₄-alkyl or C₁-C₄alkoxy, or
A and B represent, together with the carbon atom to which they are bonded, a C₄-C₇-membered ring in which two substituents, together with the carbon atoms to which they are bonded, represent a saturated or unsaturated C₅-C₆ ring which is optionally substituted identically or differently once or more than once by C₁-C₃-alkyl, C₁-C₃-alkoxy, fluorine or chlorine, and which can be interrupted by oxygen or sulphur,
G represents hydrogen (a) or represents one of the groups 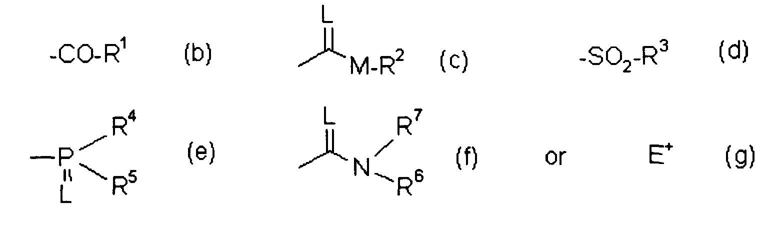 in which
E⁺ represents a metal ion equivalent or an ammonium ion,
L represents oxygen or sulphur,
M represents oxygen or sulphur,
R¹ represents C₁-C₁₆-alkyl, C₂-C₁₆-alkenyl, C₁-C₆-alkoxy-C₁-C₆-alkyl, C₁-C₆-alkylthio-C₁-C₆-alkyl or C₁-C₆-polyalkoxy-C₂-C₆-alkyl which are in each case optionally substituted identically or differently once or more than once by halogen, or cycloalkyl having 3 to 7 ring atoms which is optionally substituted by fluorine, chlorine or C₁-C₄-alkyl and can be interrupted by 1 to 2 oxygen and/or sulphur atoms,
represents phenyl which is optionally substituted identically or differently once to five times by halogen, nitro, C₁-C₄-alkyl, C₁-C₄-alkoxy, C₁-C₃-halogenoalkyl or C₁-C₃-halogenoalkoxy,
represents phenyl-C₁-C₄-alkyl which is optionally substituted identically or differently once to five times by halogen, C₁-C₄-alkyl, C₁-C₄-alkoxy, C₁-C₃-halogenoalkyl or C₁-C₃-halogenoalkoxy,
represents pyridyl, thienyl, furanyl, pyrimidyl, thiazolyl or pyrazolyl which are optionally substituted identically or differently once to four times by halogen or C₁-C₆-alkyl,
represents phenoxy-C₁-C₅-alkyl which is optionally substituted identically or differently once to five times by halogen or C₁-C₄-alkyl,
represents pyridyloxy-C₁-C₅-alkyl, pyrimidyloxy-C₁-C₅-alkyl and thiazolyloxy-C₁-C₅-alkyl which are optionally substituted identically or differently once to four times by halogen, amino or C₁-C₄-alkyl,
R² represents C₁-C₁₆-alkyl, C₂-C₁₆-alkenyl, C₁-C₆-alkoxy-C₂-C₆-alkyl or C₁-C₆-polyalkoxy-C₂-C₆-alkyl which are in each case optionally substituted identically or differently once or more than once by halogen, or represents C₃-C₇-cycloalkyl which is optionally substituted by fluorine, chlorine, C₁-C₃-alkyl, C₁-C₃-alkoxy, or
represents phenyl or benzyl which are in each case optionally substituted identically or differently once to five times by halogen, nitro, C₁-C₄-alkyl, C₁-C₃-alkoxy or C₁-C₃-halogenoalkyl,
R³, R⁴ and R⁵ represent, independently of each other, C₁-C₆-alkyl, C₁-C₆-alkoxy, C₁-C₆-alkylamino, di-(C₁-C₆)-alkylamino, C₁-C₆-alkylthio, C₃-C₄-alkenylthio, C₂-C₄-alkinylthio or C₃-C₆-cycloalkylthio which are in each case optionally substituted identically or differently once or more than once by halogen, or represent phenyl, benzyl, phenoxy or phenylthio which are in each case optionally substituted identically or differently once to five times by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl, and
R⁶ and R⁷ represent, independently of each other, hydrogen, or C₁-C₂₀-alkyl, C₁-C₂₀-alkoxy, C₂-C₈-alkenyl or C₁-C₂₀-alkoxy-C₁-C₂₀-alkyl which are in each case optionally substituted identically or differently once or more than once by halogen, represent phenyl or benzyl which is optionally substituted identically or differently once to five times by halogen, C₁-C₅-halogenoalkyl, C₁-C₅-alkyl or C₁-C₅-alkoxy, or together represent a C₄-C₆-alkylene radical which is optionally interrupted by oxygen or sulphur.

3. Compounds of the formula (I) according to Claim 1, in which
X represents methyl, ethyl, propyl, i-propyl, fluorine, chlorine, bromine, methoxy, ethoxy or trifluoromethyl,
Y represents hydrogen, methyl, ethyl, propyl, i-propyl, butyl, i-butyl, tert-butyl, fluorine, chlorine, bromine, methoxy, ethoxy or trifluoromethyl,
Z represents methyl, ethyl, i-propyl, butyl, i-butyl, tert-butyl, fluorine, chlorine, bromine, methoxy or ethoxy,
n represents a number 0 or 1
or the radicals X and Z represent, together with the phenyl radical to which they are bonded, the radical of the formula 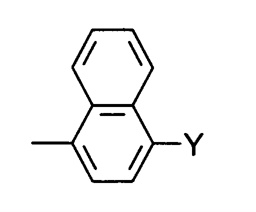
in which Y has the abovementioned meaning,
A and B represent, together with the carbon atom to which they are bonded, a saturated or unsaturated 3- to 8-membered ring which is optionally interrupted by oxygen and/or sulphur and is optionally substituted identically or differently once to five times by fluorine, chlorine, C₁-C₄-alkyl, C₁-C₄-alkoxy, trifluoromethyl, C₁-C₂-alkylthio or phenyl which is optionally substituted identically or differently once to three times by fluorine, chlorine, methyl or methoxy, or
A and B represent, together with the carbon atom to which they are bonded, a C₃-C₆-membered ring in which two substituents, together with the carbon atoms to which they are bonded, represent a saturated or unsaturated C₅-C₆-ring which is optionally substituted identically or differently once to five times by methyl, ethyl, methoxy, ethoxy, fluorine or chlorine, and which can be interrupted by oxygen or sulphur,
G represents hydrogen (a) or represents one of the groups 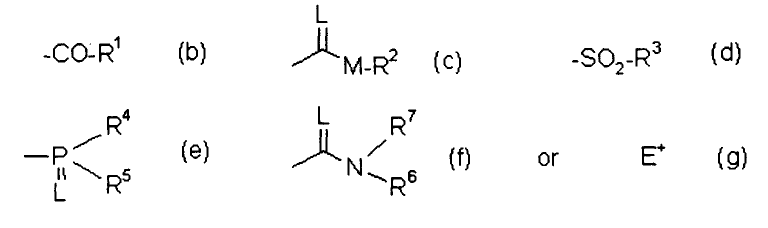 in which
E⁺ represents a metal ion equivalent or an ammonium ion,
L represents oxygen or sulphur,
M represents oxygen or sulphur,
R¹ represents C₁-C₁₄-alkyl, C₂-C₁₄-alkenyl, C₁-C₄-alkoxy-C₁-C₆-alkyl, C₁-C₄-alkylthio-C₁-C₆-alkyl or C₁-C₄-polyalkoxy-C₂-C₄-alkyl which are in each case optionally substituted identically or differently once to nine times by fluorine or chlorine, or cycloalkyl which has 3 to 6 ring atoms, which is optionally substituted by fluorine, chlorine, methyl or ethyl, and can be interrupted by 1-2 oxygen and/or sulphur atoms,
represents phenyl which is optionally substituted identically or differently once to three times by fluorine, chlorine, bromine, methyl, ethyl, propyl, i-propyl, methoxy, ethoxy, trifluoromethyl, trifluoromethoxy or nitro,
represents phenyl-C₁-C₃-alkyl which is optionally substituted identically or differently once to three times by fluorine, chlorine, bromine, methyl, ethyl, propyl, i-propyl, methoxy, ethoxy, trifluoromethyl or trifluormethoxy,
represents pyridyl, thienyl or furanyl which are in ease case optionally substituted identically or differently once to three times by fluorine, chlorine, bromine, methyl or ethyl,
represents phenoxy-C₁-C₄-alkyl which is optionally substituted identically or differently once to three times by fluorine, chlorine, methyl or ethyl, or
represents pyridyloxy-C₁-C₄-alkyl, pyrimidyloxy-C₁-C₄-alkyl and thiazolyloxy-C₁-C₄-alkyl which are in each case optionally substituted identically or differently once to three times by fluorine, chlorine, amino, methyl or ethyl,
R² represents C₁-C₁₄-alkyl, C₂-C₁₄-alkenyl, C₁-C₄-alkoxy-C₂-C₆-alkyl or C₁-C₄-polyalkoxy-C₂-C₆-alkyl which are in each case optionally substituted identically or differently once to nine times by fluorine or chlorine, or represents C₃-C₆-cycloalkyl which is optionally substituted by fluorine, chlorine, methyl, ethyl, methoxy or ethoxy,
or represents phenyl or benzyl which are in each case optionally substituted identically or differently once to three times by fluorine, chlorine, nitro, methyl, ethyl, propyl, i-propyl, methoxy, ethoxy or trifluoromethyl,
R³, R⁴ and R⁵ represent, independently of each other, C₁-C₄-alkyl, C₁-C₄-alkoxy, C₁-C₄-alkylamino, di-(C₁-C₄-alkyl)-amino or C₁-C₄-alkylthio which are in each case optionally substituted identically or differently once to five times by fluorine or chlorine, represent phenyl, benzyl, phenoxy or phenylthio which are in each case optionally substituted identically or differently once to three times by fluorine, chlorine, bromine, nitro, cyano, C₁-C₂-alkoxy, C₁-C₄-fluoroalkoxy, C₁-C₂-chloroalkoxy, C₁-C₂-alkylthio, C₁-C₂-fluoroalkylthio, C₁-C₂-chloroalkylthio or C₁-C₃-alkyl and
R⁶ and R⁷ represent, independently of each other, hydrogen, or C₁-C₁₀-alkyl, C₁-C₁₀-alkoxy or C₁-C₁₀-alkoxy-(C₁-C₁₀)alkyl which are in each case optionally substituted identically or differently once to nine times by fluorine, chlorine or bromine, represent phenyl which is optionally substituted identically or differently once to three times by fluorine, chlorine, bromine, C₁-C₂-halogenoalkyl, C₁-C₂-alkyl or C₁-C₄-alkoxy, represent benzyl which is optionally substituted identically or differently once to three times by fluorine, chlorine, bromine, C₁-C₄-alkyl, C₁-C₄-halogenoalkyl or C₁-C₄-alkoxy, or together represent a C₄-C₆-alkylene radical which is optionally interrupted by oxygen or sulphur.

4. Process for preparing compounds of the formula (I) according to Claim 1, characterized
A)
in that, in order to obtain 3-aryl-4-hydroxy-Δ³-dihydrothiophenone derivatives of the formula (Ia), 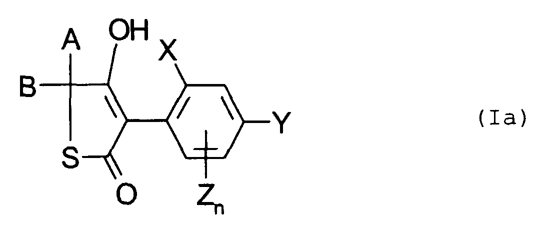 in which
A, B, X, Y, Z and n have the meaning given in Claim 1,
β-ketocarboxylic esters of the formula (II) 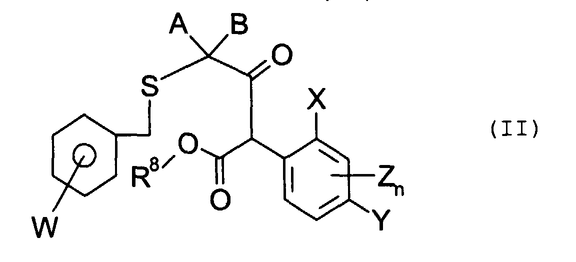 in which
A, B, X, Y, Z and n have the abovementioned meaning,
W represents hydrogen, halogen, C₁-C₈-alkyl or C₁-C₈-alkoxy,
and
R⁸ represents C₁-C₈-alkyl,
are subjected to intramolecular cyclization, optionally in the presence of a diluent and in the presence of an acid, or
(B)
in that, in order to obtain compounds of the formula (Ib), 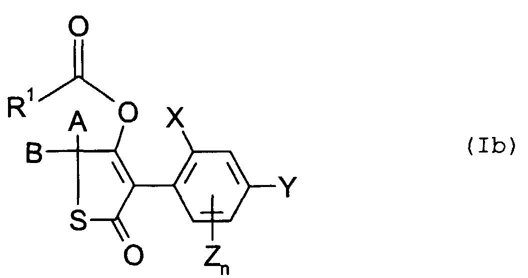 in which
A, B, X, Y, Z, R¹ and n have the meaning given in Claim 1,
compounds of the formula (Ia) 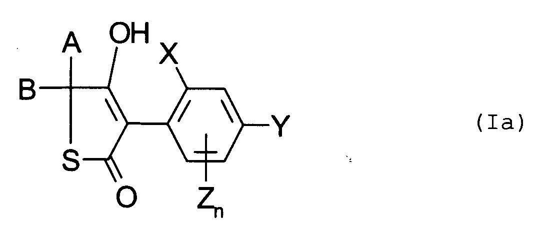 in which
A, B, X, Y, Z and n have the abovementioned meaning,
α) are reacted with acid halides of the formula (III) 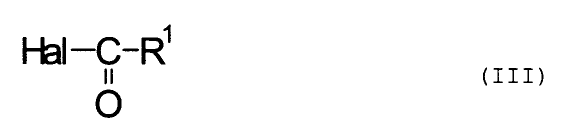 in which
R¹ has the abovementioned meaning,
and
Hal represents halogen,
optionally in the presence of a diluent and optionally in the presence of an acid binding agent,
or
β) are reacted with carboxylic anhydrides of the formula (IV)
R¹-CO-O-CO-R¹ (IV)
in which
R¹ has the abovementioned meaning,
optionally in the presence of a diluent and optionally in the presence of an acid binding agent,
or
(C)
in that, in order to obtain compounds of the formula (Ic), 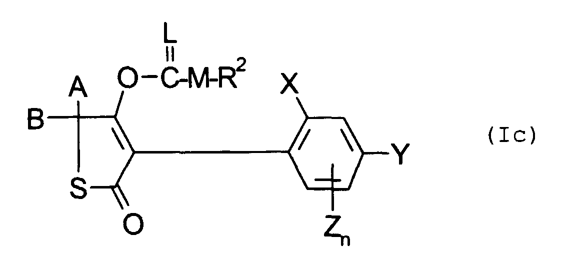 in which
A, B, X, Y, Z, R² and n have the meaning given in Claim 1,
L represents oxygen, and
M represents oxygen or sulphur,
compounds of the formula (Ia) 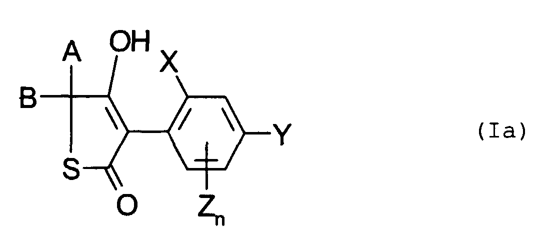 in which
A, B, X, Y, Z and n have the abovementioned meaning,
are reacted with chloroformic esters or chloroformic thioesters of the formula (V)
R²-M-CO-Cl (V)
in which
R² and M have the abovementioned meaning,
optionally in the presence of a diluent and optionally in the presence of an acid binding agent, or
(D)
in that, in order to obtain compounds of the formula (Ic) 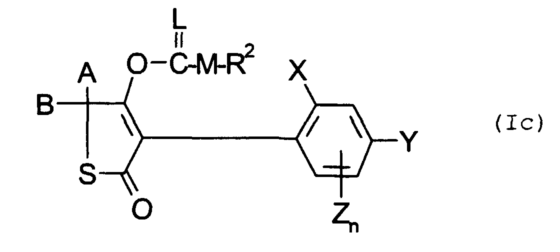 in which
A, B, R², X, Y, Z and n have the abovementioned meaning,
L represents sulphur, and
M represents oxygen or sulphur,
compounds of the formula (Ia) 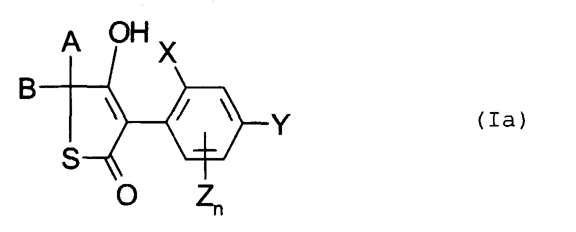 in which
A, B, X, Y, Z and n have the abovementioned meaning,
α) are reacted with chloromonothioformic esters or chlorodithioformic esters of the formula (VI) 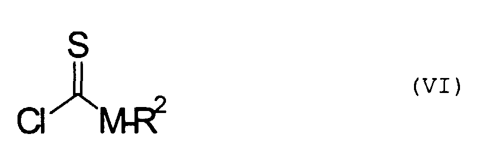 in which
M and R² have the abovementioned meaning,
optionally in the presence of a diluent and optionally in the presence of an acid binding agent, or
β) are reacted with carbon disulphide and then with alkyl halides of the formula (VII)
R²-Hal (VII)
in which
R² has the abovementioned meaning,
and
Hal represents chlorine, bromine or iodine, or
(E)
in that, in order to obtain compounds of the formula (Id) 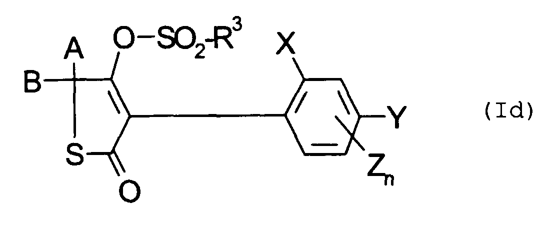 in which
A, B, X, Y, Z, R³ and n have the meaning given in Claim 1,
compounds of the formula (Ia) 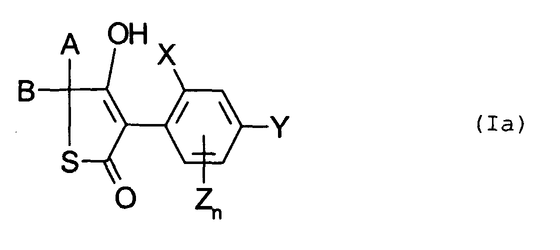 in which
A, B, X, Y, Z and n have the abovementioned meaning,
are reacted with sulphonyl chlorides of the formula (VIII)
R³-SO₂-Cl (VIII)
in which
R³ has the abovementioned meaning,
optionally in the presence of a diluent and optionally in the presence of an acid binding agent,
and
(F)
in that, in order to obtain compounds of the formula (Ie) 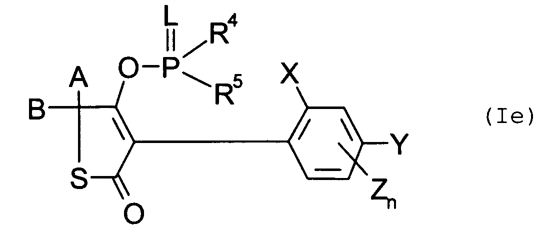 in which
A, B, L, X, Y, Z, R⁴, R⁵ and n have the meaning given in Claim 1,
compounds of the formula (Ia) 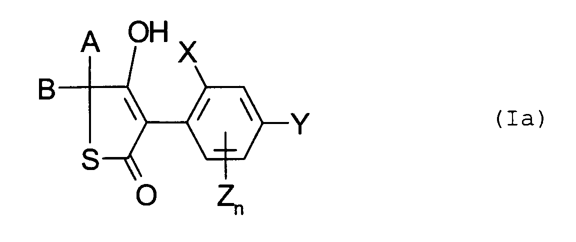 in which
A, B, X, Y, Z and n have the abovementioned meaning,
are reacted with phosphorus compounds of the formula (IX) 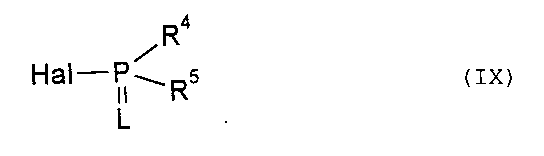 in which
L, R⁴ and R⁵ have the abovementioned meaning,
and
Hal represents halogen,
optionally in the presence of a diluent and optionally in the presence of an acid binding agent, or
(G)
in that, in order to obtain compounds of the formula (If), 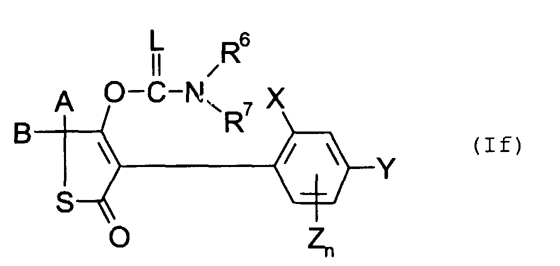 in which
A, B, L, X, Y, Z, R⁶, R⁷ and n have the meaning given in Claim 1,
compounds of the formula (Ia) 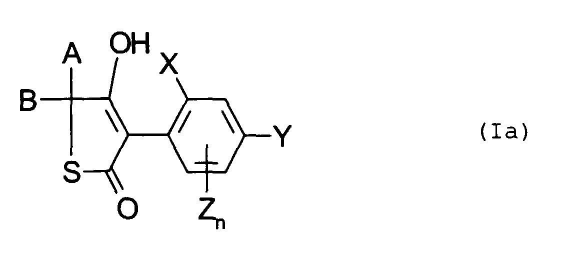 in which
A, B, X, Y, Z and n have the abovementioned meaning,
α) are reacted with isocyanates or isothiocyanates of the formula (X)
R⁶-N=C=L (X)
in which
R⁶ and L have the abovementioned meaning,
optionally in the presence of a diluent and optionally in the presence of a catalyst,
or
β) are reacted with carbamoyl chlorides or thiocarbamoyl chlorides of the formula (XI) 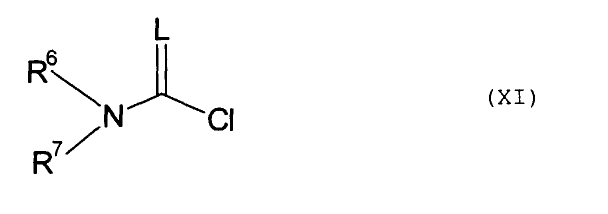 in which
L, R⁶ and R⁷ have the abovementioned meaning,
optionally in the presence of a diluent and optionally in the presence of an acid binding agent,
or
(H)
in that, in order to obtain compounds of the formula (Ig) 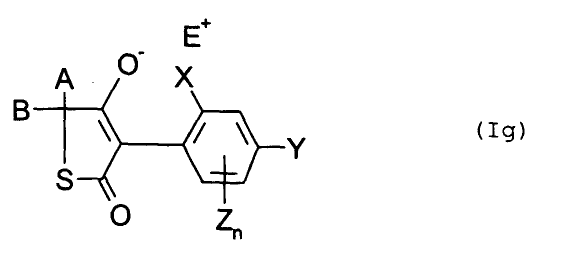 in which
X, Y, Z, A, B and n have the abovementioned meaning,
and E⁺ represents a metal ion equivalent or represents an ammonium ion,
compounds of the formula (Ia) 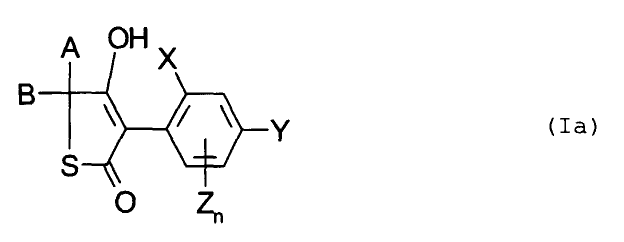 in which
X, Y, Z, A, B and n have the abovementioned meaning,
are reacted with metal hydroxides, metal alkoxides or amines of the formulae (XII) and (XIII)
MeOR⁹ₜ (XII) 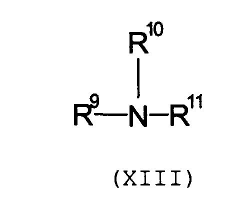
in which
Me represents singly charged or doubly charged metal ions, and
t represents the number 1 or 2, and
R⁹, R¹⁰ and R¹¹, independently of each other, represent hydrogen or alkyl,
optionally in the presence of a diluent.

5. Compounds of the formula (II) 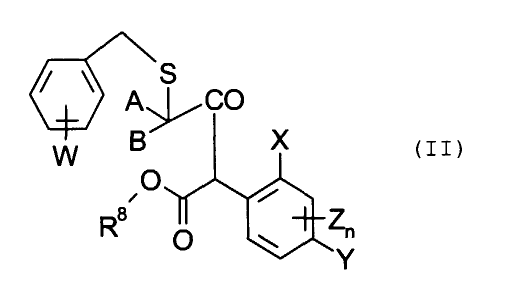 in which
A, B, X, Y, Z and n have the meaning given in Claim 1, R⁸ represents C₁-C₈-alkyl and W represents hydrogen, halogen, C₁-C₈-alkyl or C₁-C₈-alkoxy.

6. Process for preparing compounds of the formula (II) according to Claim 5, characterized in that arylacetic esters of the formula (XIV) 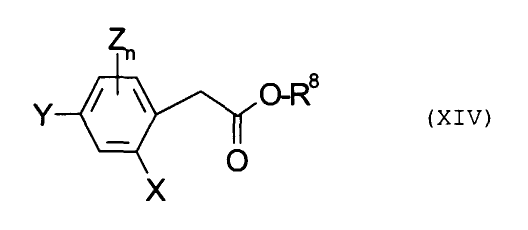 in which
X, Y, Z, R⁸ and n have the meaning given in Claim 5,
are acylated with 2-benzylthio-carbonyl halides of the formula (XV) 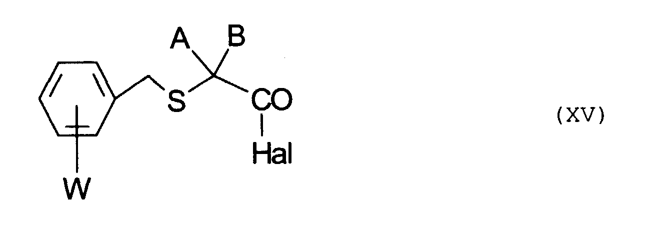 in which
A, B and W have the meaning given in Claim 5,
and
Hal represents halogen,
in the presence of strong bases.

7. Benzylthio-carbonyl halides of the formula (XVa) 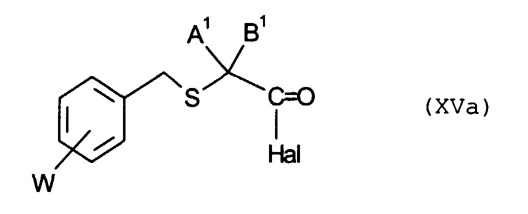 in which
W has the meaning given in Claim 5,
Hal represents halogen, and
A¹ and B¹, together with the carbon atom to which they are bonded, form a saturated or unsaturated ring which is optionally interrupted by at least one heteroatom and which is optionally substituted, or in which
A¹ and B¹, together with the carbon atom to which they are bonded, represent a ring in which substituents, together with the carbon atoms to which they are bonded, represent a saturated or unsaturated ring which is optionally substituted identically or differently once or more than once by alkyl, alkoxy or halogen and which can be interrupted by oxygen or sulphur.

8. Process for preparing compounds of the formula (XVa) according to Claim 7, characterized in that benzylthiocarboxylic acids of the formula (XVIa) 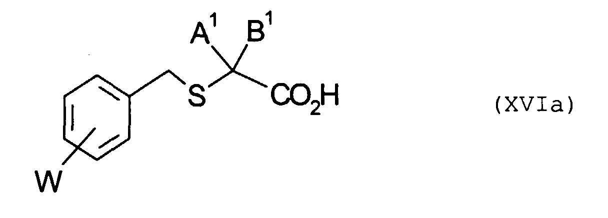 in which
A¹, B¹ and W have the meanings given in Claim 7,
are reacted with halogenating agents at temperatures of between -30°C and 150°C, preferably of between -20°C and 100°C.

9. Agent for controlling pests, characterized by a content of at least one compound of the formula (I) according to Claim 1.

10. Use of compounds of the formula (I) according to Claim 1 for controlling animal pests.

11. Process for controlling animal pests, characterized in that compounds of the formula (I) according to Claim 1 are applied to the pests and/or their habitat.

12. Process for preparing agents for controlling animal pests, characterized in that compounds of the formula (I) according to Claim 1 are mixed with extenders and/or surface-active substances.

## Revendications

1. Dérivés de 3-aryl-4-hydroxy-Δ³-dihydrothiophénones de formule (I) 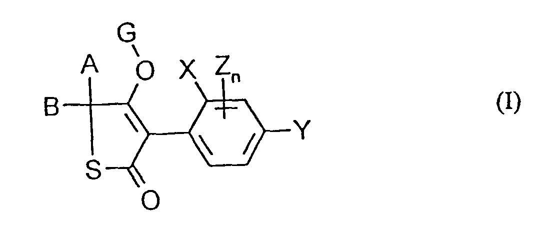 dans laquelle
X est un groupe alkyle en C₁ à C₆, un halogène, un groupe alkoxy en C₁ à C₆ ou halogénalkyle en C₁ à C₃,
Y est l'hydrogène, un groupe alkyle en C₁ à C₆, un halogène, un groupe alkoxy en C₁ à C₆ ou un groupe halogénalkyle en C₁ à C₃,
Z est un groupe alkyle à C₁ à C₆, un halogène ou groupe alkoxy en C₁ à C₆,
n représente un nombre de 0 à 3,
ou bien les restes X et Z forment conjointement avec le reste phényle auquel ils sont liés, le reste naphtalène de formule 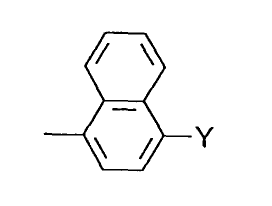 dans lequel
Y a la définition indiquée ci-dessus,
A et B forment conjointement avec l'atome de carbone auquel ils sont liés un noyau de 3 à 8 chaînons saturé ou non saturé, éventuellement interrompus par de l'oxygène et/ou du soufre et substitués le cas échéant une ou plusieurs fois identiques ou différentes par un halogène, un groupe alkyle en C₁ à C₆, alkoxy en C₁ à C₆, halogénalkyle en C₁ à C₄, halogénalkoxy en C₁ à C₄, alkylthio en C₁ à C₄, phényle éventuellement substitué une ou plusieurs fois identiques ou différentes par un halogène, un groupe alkyle en C₁ à C₆ ou alkoxy en C₁ à C₆, ou bien
A et B forment conjointement avec l'atome de carbone auquel ils sont liés un noyau de 3 à 8 chaînons dans lequel deux substituants forment, conjointement avec les atomes de carbone auxquels ils sont liés, un noyau pentagonal à heptagonal saturé ou mono-insaturé éventuellement substitué une ou plusieurs fois identiques ou différentes par un groupe alkyle en C₁ à C₆, alkoxy en C₁ à C₆ ou un halogène et pouvant être interrompu par de l'oxygène ou du soufre,
G représente de l'hydrogène (a) ou l'un des groupes 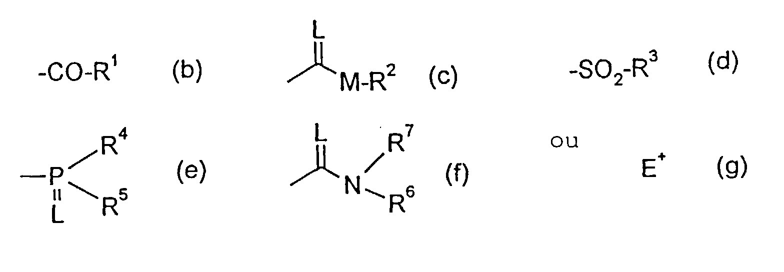 dans lesquels
E⁺ désigne un équivalent d'ion métallique ou un ion ammonium
L est l'oxygène ou le soufre et
M est l'oxygène ou le soufre,
R¹ est un groupe alkyle en C₁ à C₂₀, alcényle en C₂ à C₂₀, (alkoxy en C₁ à C₈)-(alkyle en C₁ à C₈), alkylthio en C₁ à C₈)-(alkyle en C₁ à C₈), poly(alkoxy en C₁ à C₈)-(alkyle en C₂ à C₈) dont chacun est éventuellement substitué une ou plusieurs fois identiques ou différentes par un halogène, ou un groupe cycloalkyle à noyau de 3 à 8 chaînons éventuellement substitué par un halogène ou par un groupe alkyle en C₁ à C₆ et pouvant être interrompu par au moins un atome d'oxygène et/ou un atome de soufre,
un groupe phényle éventuellement substitué une ou plusieurs fois identiques ou différents par un radical halogéno, nitro, alkyle en C₁ à C₆, alkoxy en C₁ à C₆, halogénalkyle en C₁ à C₆ ou halogénalkoxy en C₁ à C₆,
un groupe phényl-(alkyle en C₁ à C₆) éventuellement substitué une ou plusieurs fois identiques ou différentes par un radical halogéno, alkyle en C₁ à C₆, alkoxy en C₁ à C₆, halogénalkyle en C₁ à C₆ ou halogénalkoxy en C₁ à C₆,
un groupe hétaryle pentagonal ou hexagonal éventuellement substitué une ou plusieurs fois identiques ou différentes par un radical halogéno ou alkyle en C₁ à C₆,
un groupe phénoxy-(alkyle en C₁ à C₆) éventuellement substitué une ou plusieurs fois identiques ou différentes par un radical halogéno ou alkyle en C₁ à C₆, ou bien
un groupe (hétaryloxy pentagonal ou hexagonal)-(alkyle en C₁ à C₆) éventuellement substitué une ou plusieurs fois identiques ou différentes par un radical halogéno, amino ou alkyle en C₁ à C₆,
R² est un groupe alkyle en C₁ à C₂₀, alcényle en C₂ à C₂₀, (alkoxy en C₁ à C₈)-(alkyle en C₂ à C₈) ou poly(alkoxy en C₁ à C₈)-(alkyle en C₂ à C₈), chacun est éventuellement substitué une ou plusieurs fois identiques ou différentes par un halogène, ou bien un groupe cycloalkyle en C₃ à C₈ éventuellement substitué par un radical halogéno, alkyle en C₁ à C₄ ou alkoxy en C₁ à C₄, ou bien
un groupe phényle ou benzyle dont chacun est substitué le cas échéant une ou plusieurs fois identiques ou différentes par un radical halogéno, nitro, alkyle en C₁ à C₆, alkoxy en C₁ à C₆, ou halogénalkyle en C₁ à C₆,
R₃, R⁴ et R⁵ représentent indépendamment les uns des autres un groupe alkyle en C₁ à C₈, alkoxy en C₁ à C₈, alkylamino en C₁ à C₈, di(alkyle en C₁ à C₈)amino, alkylthio en C₁ à C₈, alcénylthio en C₂ à C₅, alcynylthio en C₂ à C₅, cycloalkylthio en C₃ à C₇ dont chacun est éventuellement substitué une ou plusieurs fois identiques ou différentes par un halogène, ou bien un groupe phényle, benzyle, phénoxy ou phénylthio dont chacun est substitué le cas échéant une ou plusieurs fois identiques ou différentes par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄, ou halogénalkyle en C₁ à C₄ et
R⁶ et R⁷ représentent indépendamment l'un de l'autre l'hydrogène, un groupe alkyle en C₁ à C₂₀, alkoxy en C₁ à C₂₀, alcényle en C₂ à C₈ ou (alkoxy en C₁ à C₂₀)-(alkyle en C₁ à C₂₀) dont chacun est substitué le cas échéant une ou plusieurs fois identiques ou différentes par un halogène, un groupe phényle ou benzyle dont chacun est substitué le cas échéant une ou plusieurs fois identiques ou différentes par un radical halogéno, halogénalkyle en C₁ à C₂₀, alkyle en C₁ à C₂₀ ou alkoxy en C₁ à C₂₀ ou forment conjointement un reste alkylène en C₂ à C₆ éventuellement interrompu par l'oxygène.

2. Composés de formule (I) suivant la revendication 1, dans laquelle
X est un groupe alkyle en C₁ à C₆, un halogène, un groupe alkoxy en C₁ à C₆ ou halogénalkyle en C₁ ou C₂,
Y est l'hydrogène, un groupe alkyle en C₁ à C₆, un halogène, un groupe alkoxy en C₁ à C₆ ou halogénalkyle en C₁ ou C₂,
Z est un groupe alkyle en C₁ à C₄, un halogène ou un groupe alkoxy en C₁ à C₄,
n représente un nombre de 0 à 2
ou bien les restes X et Z forment conjointement avec le reste phényle auquel ils sont liés le reste naphtalène de formule 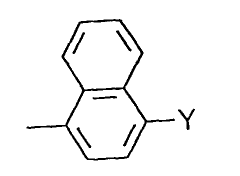 dans lequel
Y a la définition indiquée ci-dessus
A et B forment conjointement avec l'atome de carbone auquel ils sont liés un noyau de 3 à 8 chaînons saturé ou non saturé, éventuellement interrompu par de l'oxygène et/ou du soufre et substitué le cas échéant une ou plusieurs fois identiques ou différentes par un radical fluoro, chloro, alkyle en C₁ à C₅, alkoxy en C₁ à C₅, halogénalkyle en C₁ à C₃, halogénalkoxy en C₁ à C₃, alkylthio en C₁ à C₃ ou un radical phényle éventuellement substitué une ou plusieurs fois identiques ou différentes par un radical fluoro, chloro, bromo, alkyle en C₁ à C₄ au alkoxy en C₁ à C₄, ou bien
A et B forment conjointement avec l'atome de carbone auquel ils sont liés un noyau de 4 à 7 chaînons dans lequel deux substituants forment conjointement avec les atomes de carbone auxquels ils sont liés un noyau pentagonal ou hexagonal saturé ou non saturé, éventuellement substitué une ou plusieurs fois identiques ou différentes par un radical alkyle en C₁ à C₃, alkoxy en C₁ à C₃, fluoro ou chloro et pouvant être interrompu par de l'oxygène ou du soufre,
G représente de l'hydrogène (a) ou l'un des groupes 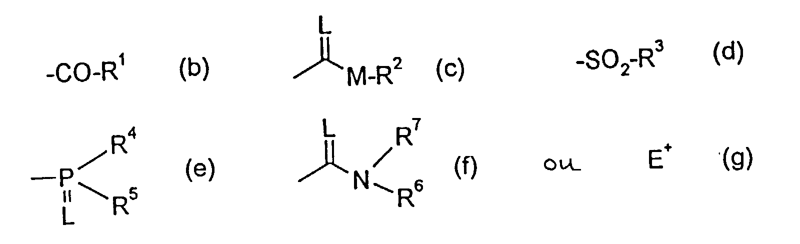 dans lesquels
E⁺ représente un équivalent non métallique ou un ion ammonium,
L est l'oxygène ou le soufre,
M est l'oxygène ou le soufre,
R¹ est un groupe alkyle en C₁ à C₁₆, alcényle en C₂ à C₁₆, (alkoxy en C₁ à C₆)-(alkyle en C₁ à C₆), (alkylthio en C₁ à C₆)-(alkyle en C₁ à C₆), poly(alkoxy en C₁ à C₆)-(alkyle en C₂ à C₆) dont chacun est substitué le cas échéant une ou plusieurs fois identiques ou différentes par un halogène, ou un groupe cycloalkyle à noyau de 3 à 7 atomes qui peut être interrompu par un ou deux atomes d'oxygène et/ou de soufre et qui est substitué par du fluor, du chlore ou un radical alkyle en C₁ à C₄,
un groupe phényle éventuellement substitué 1 à 5 fois identiques ou différentes par un radical halogéno, nitro, alkyle en C₁ en C₄, alkoxy en C₁ à C₄, halogénalkyle en C₁ à C₃ ou halogénalkoxy en C₁ à C₃,
un groupe phényl-(alkyle en C₁ à C₄) éventuellement substitué 1 à 5 fois identiques ou différentes par un radical halogéno, alkyle en C₁ à C₄, alkoxy en C₁ à C₄, halogénalkyle en C₁ à C₃ ou halogénalkoxy en C₁ à C₃,
un groupe pyridyle, thiényle, furannyle, pyrimidyle, thiazolyle ou pyrazolyle éventuellement substitué 1 à 4 fois identiques ou différentes par un halogène ou un radical alkyle en C₁ à C₆,
un groupe phénoxy-(alkyle en C₁ à C₅) éventuellement substitué 1 à 5 fois identiques ou différentes par un halogène ou un radical alkyle en C₁ à C₄,
un groupe pyridyloxy-, pyrimidyloxy-, thiazolyloxy-(alkyle en C₁ à C₅) éventuellement substitué 1 à 4 fois identiques ou différentes par un halogène, un radical amino ou alkyle en C₁ à C₄,
R² est un groupe alkyle en C₁ à C₁₆, alcényle en C₂ à C₁₆, (alkoxy en C₁ à C₆)-(alkyle en C₂ à C₆) ou poly(alkoxy en C₁ à C₆)-(alkyle en C₂ à C₆) éventuellement substitué une ou plusieurs fois identiques ou différentes par un halogène, ou un groupe cycloalkyle en C₃ à C₇ éventuellement substitué par un radical fluoro, chloro, alkyle en C₁ à C₃, alkoxy en C₁ à C₃ ou bien un groupe phényle ou benzyle dont chacun est éventuellement substitué 1 à 5 fois identiques ou différentes par un radical halogéno, nitro, alkyle en C₁ à C₄, alkoxy en C₁ à C₃ ou halogénalkyle en C₁ à C₃,
R³, R⁴, R⁵ représentent indépendamment les uns des autres un groupe alkyle en C₁ à C₆, alkoxy en C₁ à C₆, alkylamino en C₁ à C₆, di(alkyle en C₁ à C₆)amino, alkylthio en C₁ à C₆, alcénylthio en C₃ ou C₄, alcynylthio en C₂ à C₄, cycloalkylthio en C₃ à C₆ dont chacun est substitué le cas échéant une ou plusieurs fois identiques ou différentes par un halogène, ou un groupe phényle, benzyle, phénoxy ou phénylthio dont chacun est substitué le cas échéant 1 à 5 fois identiques ou différentes par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃ et
R⁶ et R⁷ représentent indépendamment l'un de l'autre l'hydrogène, un groupe alkyle en C₁ à C₂₀, alkoxy en C₁ à C₂₀, alcényle en C₂ à C₈ ou (alkoxy en C₁ à C₂₀)-(alkyle en C₁ à C₂₀) dont chacun est substitué le cas échéant une ou plusieurs fois identiques ou différentes par un halogène, un groupe phényle ou benzyle dont chacun est substitué le cas échéant 1 à 5 fois identiques ou différentes par un radical halogéno, halogénalkyle en C₁ à C₅, alkyle en C₁ à C₅, alkoxy en C₁ à C₅, ou forment conjointement un reste alkylène en C₄ à C₆ éventuellement interrompu par de l'oxygène ou du soufre.

3. Composés de formule (I) suivant la revendication 1, dans laquelle
X est un groupe méthyle, éthyle, propyle, isopropyle, fluoro, chloro, bromo, méthoxy, éthoxy ou trifluorométhyle,
Y est l'hydrogène, un groupe méthyle, éthyle, propyle, isopropyle, butyle, isobutyle, tertio-butyle, fluoro, chloro, bromo, méthoxy, éthoxy, ou trifluorométhyle,
Z est un groupe méthyle, éthyle, isopropyle, butyle, isobutyle, tertio-butyle, fluoro, chloro, bromo, méthoxy ou éthoxy,
n représente le nombre 0 ou 1,
ou bien les restes X et Z forment conjointement avec le reste phényle auquel ils sont liés le reste de formule 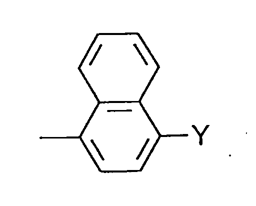 dans lequel Y a la définition indiquée ci-dessus,
A et B forment conjointement avec l'atome de carbone auquel ils sont liés un noyau de 3 à 8 chaînons saturé ou non saturé, éventuellement interrompu par de l'oxygène et/ou du soufre et substitué le cas échéant 1 à 5 fois identiques ou différentes par un radical fluoro, chloro, alkyle en C₁ à C₄, alkoxy en C₁ à C₄, trifluorométhyle, alkylthio en C₁ ou C₂ ou par un radical phényle éventuellement substitué 1 à 3 fois identiques ou différentes par un radical fluoro, chloro, méthyle ou méthoxy,
A et B forment conjointement avec l'atome de carbone auquel ils sont liés un noyau de 3 à 6 chaînons dans lequel deux substituants forment conjointement avec l'atome de carbone auquel ils sont liés un noyau pentagonal ou hexagonal saturé ou non saturé éventuellement substitué 1 à 5 fois identiques ou différentes par un radical méthyle, éthyle, méthoxy, éthoxy, fluoro ou chloro et pouvant être interrompu par de l'oxygène ou du soufre,
G représente l'hydrogène (a) ou l'un des groupes 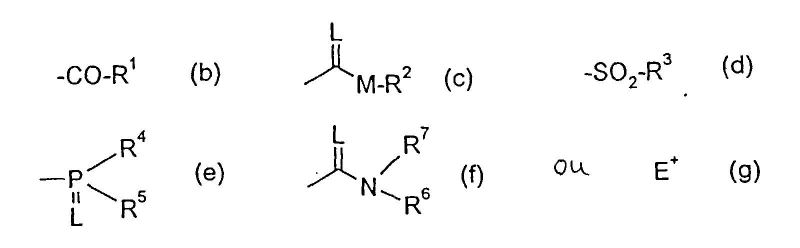 dans lesquels
E⁺ est un équivalent d'ion métallique ou un ion ammonium,
L est l'oxygène ou le soufre,
M est l'oxygène ou le soufre,
R₁ représente un groupe alkyle en C₁ à C₁₄, alcényle en C₂ à C₁₄, (alkoxy en C₁ à C₄)-(alkyle en C₁ à C₆), (alkylthio en C₁ à C₄)-(alkyle en C₁ à C₆), poly(alkoxy en C₁ à C₄)-(alkyle en C₂ à C₄) dont chacun est substitué le cas échéant 1 à 9 fois identiques ou différentes par du fluor ou du chlore, ou un groupe cycloalkyle à noyau de 3 à 6 atomes éventuellement substitué par un radical fluoro, chloro, méthyle ou éthyle et pouvant être interrompu par 1 ou 2 atomes d'oxygène et/ou de soufre,
un groupe phényle éventuellement substitué 1 à 3 fois identiques ou différentes par un radical fluoro, chloro, bromo, méthyle, éthyle, propyle, isopropyle, méthoxy, éthoxy, trifluorométhyle, trifluorométhoxy ou nitro,
un groupe phényl-(alkyle en C₁ à C₃) éventuellement substitué 1 à 3 fois identiques ou différentes par un radical fluoro, chloro, bromo, méthyle, éthyle, propyle, isopropyle, méthoxy, éthoxy, trifluorométhyle ou trifluorométhoxy,
un groupe pyridyle, thiényle ou furannyle dont chacun est substitué le cas échéant une à trois fois identiques ou différentes par un radical fluoro, chloro, bromo, méthyle ou éthyle,
un groupe phénoxy-(alkyle en C₁ à C₄) éventuellement substitué une à trois fois identiques ou différentes par un radical fluoro, chloro, méthyle ou éthyle, ou bien
un groupe pyridyloxy-(alkyle en C₁ à C₄), pyrimidyloxy-(alkyle en C₁ à C₄) et thiazolyloxy-(alkyle en C₁ à C₄) substitués chacun le cas échéant une à trois fois identiques ou différentes par un radical fluoro, chloro, amino, méthyle ou éthyle,
R² est un groupe alkyle en C₁ à C₁₄, alcényle en C₂ à C₁₄, (alkoxy en C₁ à C₄)-(alkyle en C₂ à C₆), ou poly(alkoxy en C₁ à C₄)-(alkyle en C₂ à C₆) dont chacun est substitué le cas échéant 1 à 9 fois identiques ou différentes par du fluor ou du chlore, un groupe cycloalkyle en C₃ à C₆ éventuellement substitué par un radical fluoro, chloro, méthyle, éthyle, méthoxy ou éthoxy,
ou bien un groupe phényle ou benzyle dont chacun est substitué le cas échéant une à trois frois identiques ou différentes par un radical fluoro, chloro, nitro, méthyle, éthyle, propyle, isopropyle, méthoxy, éthoxy ou trifluorométhyle,
R³, R⁴ et R⁵ représentent indépendamment les uns des autres un groupe alkyle en C₁ à C₄, alkoxy en C₁ à C₄, alkylamino en C₁ à C₄, di(alkyle en C₁ à C₄)amino, (alkylthio en C₁ à C₄) dont chacun est substitué le cas échéant 1 à 5 fois identiques ou différentes par du fluor ou du chlore, un groupe phényle, benzyle, phénoxy ou phénylthio dont chacun est substitué le cas échéant 1 à 3 fois identiques ou différentes par un radical fluoro, chloro, bromo, nitro, cyano, alkoxy en C₁ ou C₂, fluoralkoxy en C₁ à C₄, chloralkoxy en C₁ ou C₂, alkylthio en C₁ ou C₂, fluoralkylthio en C₁ ou C₂, chloralkylthio en C₁ ou C₂ ou alkyle en C₁ ou C₃ et
R⁶ et R⁷ représentent indépendamment l'un de l'autre l'hydrogène, un groupe alkyle en C₁ à C₁₀, alkoxy en C₁ à C₁₀ ou (alkoxy en C₁ à C₁₀)-(alkyle en C₁ à C₁₀) dont chacun est substitué le cas échéant 1 à 9 fois identiques ou différentes par du fluor, du chlore ou du brome, un groupe phényle éventuellement substitué 1 à 3 fois identiques ou différentes par un radical fluoro, chloro, bromo, halogénalkyle en C₁ ou C₂, alkyle en C₁ ou C₂, ou alkoxy en C₁ ou C₄, un groupe benzyle éventuellement substitué 1 à 3 fois identiques ou différentes par un radical fluoro, chloro, bromo, alkyle en C₁ à C₄, halogénalkyle en C₁ à C₄ ou alkoxy on C₁ à C₄, ou forment ensemble un reste alkylène en C₄ à C₆ éventuellement interrompu par de l'oxygène ou du soufre.

4. Procédé de production de composés de formule (I) suivant la revendication 1, caractérisé en ce que
A)
pour obtenir des dérivés de 3-aryl-4-hydroxy-Δ³-dihydrothiophénones de formule (Ia) 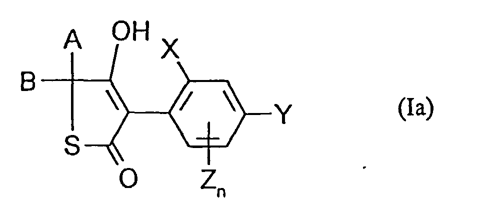 dans laquelle
A, B, X, Y, Z et n ont la définition indiquée dans la revendication 1,
on effectue la cyclisation intramoléculaire d'un ester d'acide β-cétocarboxylique de formule (II) 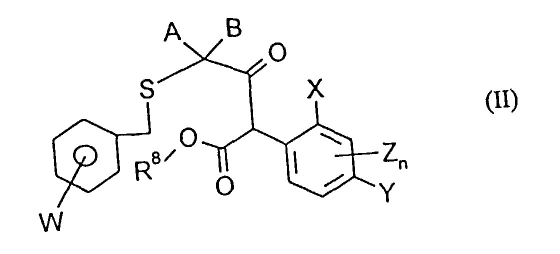 dans laquelle
A, B, X, Y, Z et m ont la définition indiquée ci-dessus,
W est l'hydrogène, un halogène, un groupe alkyle en C₁ à C₈ ou alkoxy en C₁ à C₈,
et
R₈ est un groupe alkyle en C₁ à C₈,
le cas échéant en présence d'un diluant et en présence d'un acide
(B)
pour obtenir des composés de formule (Ib) 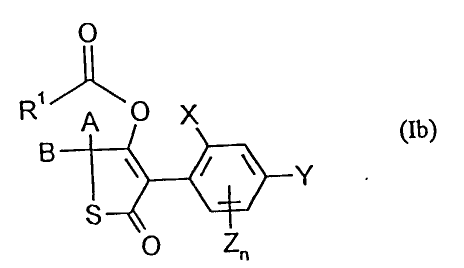 dans laquelle
A, B, X, Y, Z, R¹ et n ont la définition indiquée dans la revendication 1,
On fait réagir des composés de formule (Ia) 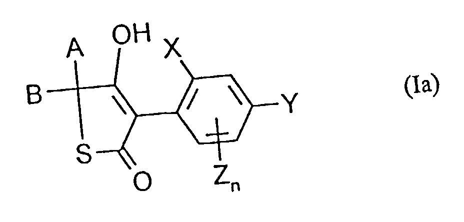 dans laquelle
A, B, X, Y, Z et n ont la définition indiquée ci-dessus,
α) avec des halogénures d'acides de formule (III) 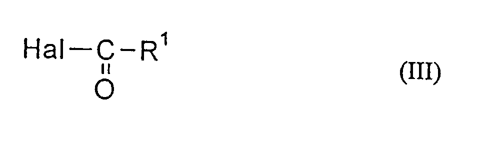 dans laquelle
R¹ a la définition indiquée ci-dessus
et
Hal est un halogène
le cas échéant en présence d'un diluant et en la présence éventuelle d'un accepteur d'acide
ou bien
β) avec des anhydrides d'acides carboxyliques de formule (IV)
R¹-CO-O-CO-R¹ (IV)
dans laquelle
R¹ a la définition indiquée ci-desssus,
le cas échant en présence d'un diluant et en la
présence éventuelle d'un accepteur d'acide,
ou bien
(C)
pour obtenir des composés de formule (Ic) 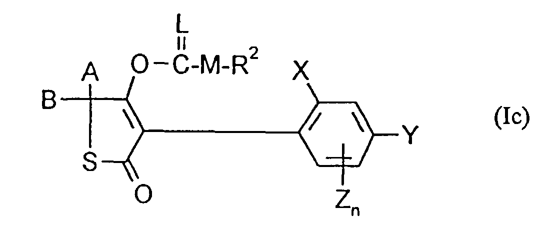 dans laquelle
A, B, X, Y, Z, R² et n ont la définition indiquée dans la revendication 1,
L est de l'oxygène et
M est de l'oxygène ou du soufre,
on fait réagir des composés de formule (Ia) 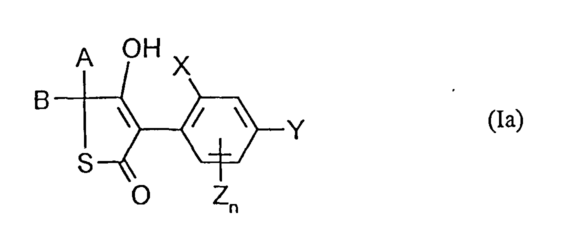 dans laquelle
A, B, X, Y, Z et n ont la définition indiquée ci-dessus,
avec un ester d'acide chloroformique ou un thioester d'acide chloroformique de formule (V)
R²-M-CO-Cl (V)
dans laquelle
R² et M ont la définition indiquée ci-dessus,
le cas échéant en présence d'un diluant et en la présence éventuelle d'un accepteur d'acide, ou bien
(D)
pour obtenir des composés de formule (Ic) 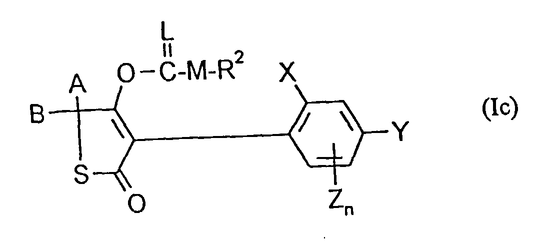 dans laquelle
A, B, R², X, Y, Z et n ont la définition indiquée ci-dessus,
L est le soufre et
M est l'oxygène ou le soufre,
on fait réagir des composés de formule (Ia) 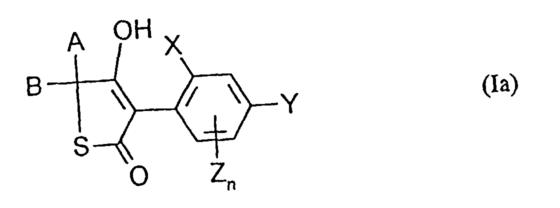 dans laquelle
A, B, X, Y, Z et n ont la définition indiquée ci-dessus,
α) avec des esters d'acide chloromonothioformique ou des esters d'acide chlorodithioformique de formule (VI) 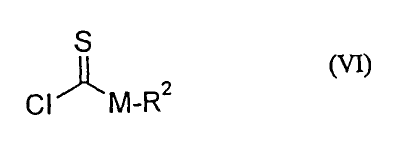 dans laquelle
M et R² ont la définition indiquée ci-dessus,
le cas échéant en présence d'un diluant et en la présence éventuelle d'un accepteur d'acide, ou bien
β) avec du sulfure de carbone, puis avec des halogénures d'alkyle de formule (VII)
R²-Hal (VII)
dans laquelle
R² a la définition indiquée ci-dessus,
et
Hal représente le chlore, le brome ou l'iode, ou bien
(E)
pour obtenir des composés de formule (Id) 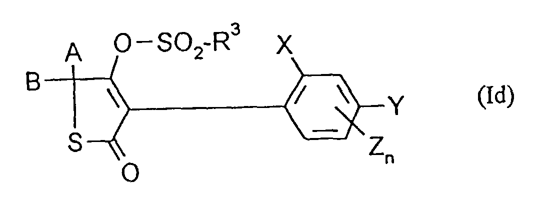 dans laquelle
A, B, X, Y, Z, R³ et n ont la définition indiquée dans la revendication 1,
on fait réagir des composés de formule (Ia) 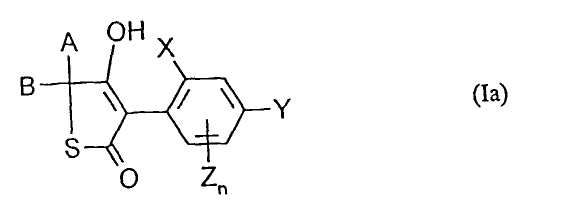 dans laquelle
A, B, X, Y, Z et n ont la définition indiquée ci-dessus,
avec des chlorures d'acides sulfoniques de formule (VIII)
R³-SO₂-Cl (VIII)
dans laquelle
R³ a la définition indiquée ci-dessus,
le cas échéant en présence d'un diluant et en présence éventuelle d'un accepteur d'acide, et
(F)
pour obtenir des composés de formule (Ie) 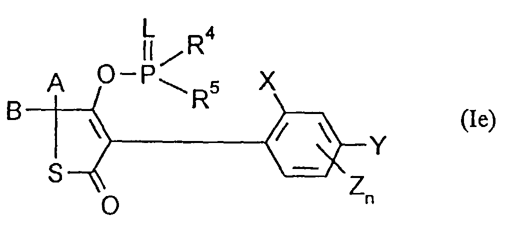 dans laquelle
A, B, L, X, Y, Z, R⁴, R⁵ et n ont la définition indiquée dans la revendication 1,
on fait réagir des composés de formule (Ia) 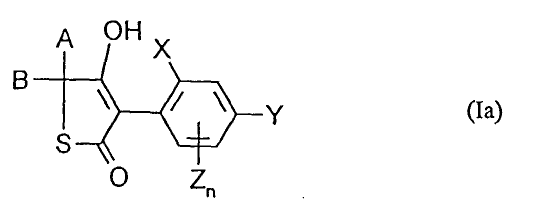 dans laquelle
A, B, X, Y, Z et n ont la définition indiquée ci-dessus avec des composés de phosphore de formule (IX) 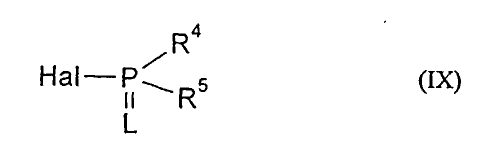
dans laquelle
L, R⁴ et R⁵ ont la définition indiquée ci-dessus et
Hal représente un halogène,
le cas échéant en présence d'un diluant et en la présence éventuelle d'un accepteur d'acide, ou bien
(G)
pour obtenir des composés de formule (If) 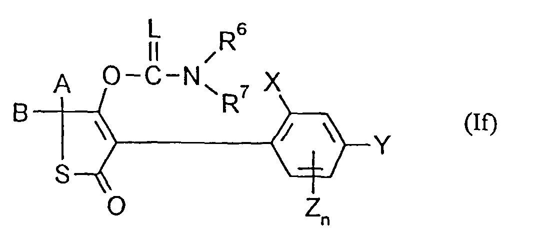 dans laquelle
A, B, L, X, Y, Z, R⁶, R⁷ et n ont la définition indiquée dans la revendication 1,
on fait réagir des composés de formule (Ia) 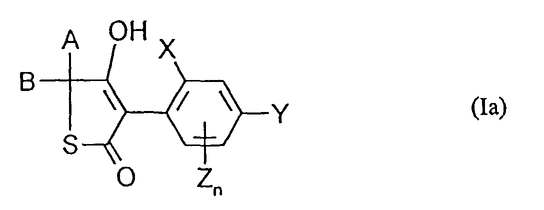 dans laquelle
A, B, X, Y, Z et n ont la définition indiquée ci-dessus
α) avec des isocyanates ou des isothiocyanates de formule (X)
R⁶-N=C=L (X)
dans laquelle
R⁶ et L ont la définition indiquée ci-dessus,
le cas échéant en présence d'un diluant et en la présence éventuelle d'un catalyseur,
ou bien
β) avec des chlorures d'acides carbamiques ou des chlorures d'acides thiocarbamiques de formule (XI) 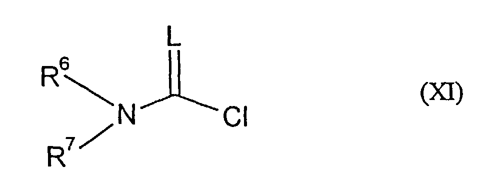 dans laquelle
L, R⁶ et R⁷ ont la définition indiquée ci-dessus,
le cas échéant en présence d'un diluant et en la présence éventuelle d'un accepteur d'acide,
ou bien
(H)
pour obtenir des composés de formule (Ig) 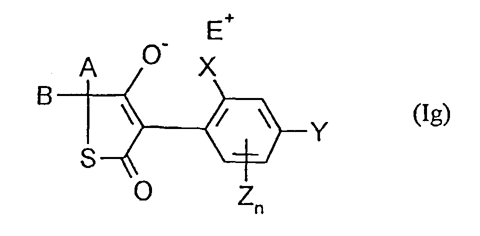 dans laquelle
X, Y, Z, A, B et n ont la définition indiquée ci-dessus et
E⁺ représente un équivalent d'ion métallique ou un ion ammonium,
on fait réagir des composés de formule (Ia) 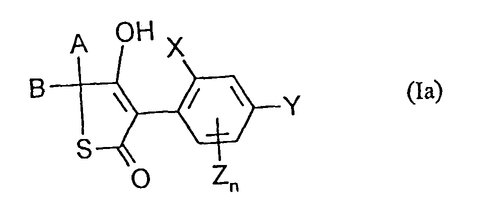 dans laquelle
X, Y, Z, A, B et n ont la définition indiquée ci-dessus,
avec des hydroxydes métalliques, des alcoolates métalliques ou des amines de formules (XII) et (XIII)
MeOR⁹ₜ (XII) 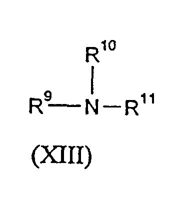
dans lesquelles
Me désigne des ions de métaux monovolents ou divalents et
t représente le nombre 1 ou 2, et
R⁹, R¹⁰ et R¹¹ représentent indépendamment les uns des autres l'hydrogène ou un groupe alkyle,
le cas échéant en présence d'un diluant.

5. Composés de formule (II) 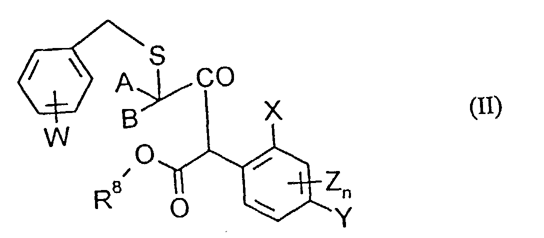 dans laquelle
A, B, X, Y, Z et n ont la définition indiquée dans la revendication 1, R⁸ est un groupe alkyle en C₁ à C₈ et W est l'hydrogène, un halogène, un groupe alkyle en C₁ à C₈ ou alkoxy en C₁ à C₈.

6. Procédé de production de composés de formule (II) suivant la revendication 5, dans lequel on acyle en présence de bases fortes un ester d'acide arylacétique de formule (XIV) 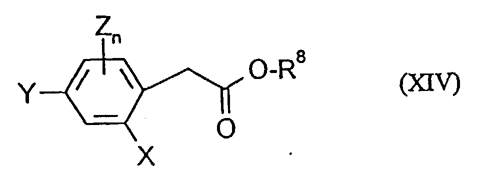 dans laquelle
X, Y, Z, R₈ et n ont la définition indiquée dans la revendication 5,
avec des halogénures d'acide 2-benzylthio-carboxylique de formule (XV) 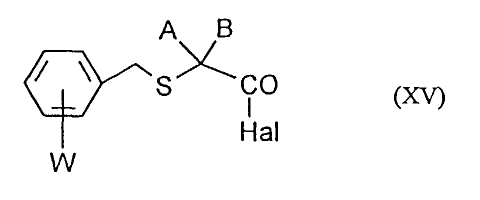 dans laquelle
A, B et W ont la définition indiquée dans la revendication 5 et
Hal représente un halogène.

7. Halogénures d'acides benzylthio-carboxyliques de formule (XVa) 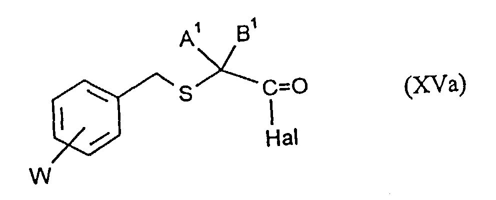 dans laquelle
W a la définition indiquée dans la revendication 5,
Hal est un halogène et
A¹ et B¹ forment conjointement avec l'atome de carbone auquel ils sont liés un cycle saturé ou non saturé éventuellement interrompu par au moins un hétéroatome et éventuellement substitué, ou bien
A¹ et B¹ forment conjointement avec l'atome de carbone auquel ils sont liés un cycle dans lequel des substituants forment conjointement avec les atomes de carbone auxquels ils sont liés un cycle saturé ou non saturé éventuellement substitué une ou plusieurs fois identiques ou différentes par un radical alkyle, alkoxy ou halogéno et pouvant être interrompu par de l'oxygène ou du soufre.

8. Procédé de production de composés de formule (XVa) suivant la revendication 7, caractérisé en ce qu'on fait réagir des acides benzylthio-carboxyliques de formule (XVIa) 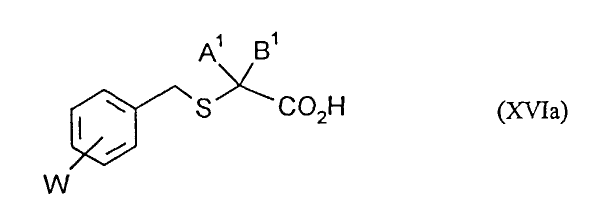 dans laquelle
A¹, B¹ et W ont les définitions indiqués dans la revendication 7,
avec des agents d'halogénation à des températures comprises entre -30°C et 150°C, de préférence entre -20°C et 100°C.

9. Composition pesticide, caractérisée par une teneur en au moins un composé de formule (I) suivant la revendication 1.

10. Utilisation de composés de formule (I) suivant la revendication 1 pour combattre des parasites animaux.

11. Procédé pour combattre des parasites animaux, caractérisé en ce qu'on épand des composés de formule (I) suivant la revendication 1 sur les parasites et/ou sur leur milieu.

12. Procédé de préparation de compositions destinées à combattre des parasites animaux, caractérisé en ce qu'on mélange des composés de formule (I) suivant la revendication 1 avec des diluants et/ou des agents tensioactifs.
